# EUROPEAN PATENT APPLICATION

(11) **EP 4 424 840 A1**
(43) Date of publication of application: **04.09.2024**
(21) Application number: 24166148.7
(22) Date of filing: 06.09.2017
(51) Int. Cl.: C12Q 1/68, A61P 11/00, C12Q 1/6883, G16B 40/20

(54) **METHODS AND SYSTEMS FOR DETECTING USUAL INTERSTITIAL PNEUMONIA**

(30) Priority: 07.09.2016 US 201662384609 P; 05.07.2017 US 201762528899 P
(62) Divisional of application: 17849490.2
(71) Applicant: Veracyte, Inc., South San Francisco, CA 94080 (US)
(72) Inventor: KENNEDY, Giulia C., South San Francisco, 94080 (US); HUANG, Jing, South San Francisco, 94080 (US); CHOI, Yoonha, South San Francisco, 94080 (US); PANKRATZ, Daniel, South San Francisco, 94080 (US); WALSH, Patric, Sean, South San Francisco, 94080 (US)
(74) Representative: Cabinet Becker et Associés

(57) **Abstract**

The present disclosure provides systems, methods, and classifiers for differentiating between samples as usual interstitial pneumonia (UIP) or non-UIP.

## Description

### CROSS-REFERENCE

This application claims priority to U.S. Provisional Patent Application Serial Number 62/384,609, filed September 7, 2016, and U.S. Provisional Patent Application Serial Number 62/528,899, filed July 5, 2017, each of which is entirely incorporated herein by reference.

### BACKGROUND

Interstitial lung diseases (ILD) are a heterogeneous group of acute and chronic bilateral parenchymal pulmonary disorders with similar clinical manifestations, but a wide spectrum of severity and outcome including varying disease progression, treatment response, and survival.¹ Among these, idiopathic pulmonary fibrosis (IPF) is one of the most common (incidence of 14-60 per 100,000 per year in North America) and severe ILD, characterized by progressive fibrosis, worsening lung function and death.³⁻⁶ In the appropriate clinical setting, IPF is defined by the presence of the usual interstitial pneumonia (UIP) pattern on HRCT and/or SLB.⁸ The lengthy time to diagnosis, coupled with the rapid course of disease, compels the need for new tools to minimize suffering of patients during the uncertain diagnostic process. Most patients diagnosed with IPF die within five years of their initial diagnosis.^{7,8} However, the recent availability of two new antifibrotic drugs pirfenidone and nintedanib, which have shown promise in stabilizing IPF disease progression, and other therapeutics in development may change this picture,⁹⁻¹¹ and accurate diagnosis is critical for appropriate therapeutic intervention. ^{5,12}

Distinguishing the diagnosis of IPF from other fibrotic IIPs has significant implications given these new possibilities for treatment with lung transplant and/or anti-fibrotic oral compounds.² Additionally, a number of the disorders that are often confused with IPF are treated with immunosuppressive agents. As treatment of IPF with combined immunosuppression has been shown to be harmful, choosing the correct treatment is critical.^{2,33}

IPF may be challenging to diagnose. Internationally recognized guidelines recommend the multidisciplinary evaluation of clinical, radiological, and pathological disease features in the diagnosis and management of ILD. The diagnostic approach to IPF requires exclusion of other interstitial pneumonias, as well as connective tissue disease and environmental and occupational exposures.³⁻⁶ Patients suspected of having IPF usually undergo high-resolution computed tomography (HRCT) of the chest, which confirms the disease with high specificity only if the pattern of usual interstitial pneumonia (UIP) is clearly evident. ^{5,13} Thus, a confident diagnosis of IPF is achievable without SLB for approximately one third of ILD patients.³⁴⁻³⁶ In those without a confident UIP pattern diagnosis on HRCT (e.g. Possible UIP, and the working category of Probable UIP), the positive predictive value (PPV) for the presence of histologic UIP has been estimated at approximately 60%,^{35,36} a level that is not considered sufficient to forgo confirmation by SLB.⁸ Accordingly, because HRCT results are frequently inconclusive, a large number of patients require an invasive diagnostic surgical lung biopsy (SLB) to clarify the histopathologic features of interstitial pneumonia and/or UIP pattern^{5,14} and the typical length of time to diagnose IPF from the onset of symptoms may be 1-2 years.¹⁵ With high procedural complication rates reported for cryobiopsy,³⁷ and in-hospital and 90-day mortality associated with SLB reaching 1.7% and 3.9% respectively,³⁸ a less invasive method of diagnosing IPF is greatly needed in the art.

Reliable identification of UIP pathology in transbronchial biopsies (TBBs) is challenged by the difficulty of sufficient sampling of alveolated lung parenchyma and heterogeneous disease distribution. Discordance between pathologists occurs, and a correct diagnosis may be dependent on individual experience.¹⁶ Despite histopathologic evaluation, a definitive diagnosis may remain elusive. In retrospective studies with high TBB sampling adequacy rates, UIP was confirmed in 30-43% of patients with clinical and radiographic features consistent with UIP,^{11,12} with a third study reporting a confirmation rate of <10%.¹³ This has led many to evaluate alternate bronchoscopic studies that may provide greater alveolar sampling.^{14,15} These are currently limited by availability and a lack of large multicenter studies.¹⁶ Diagnostic accuracy has been shown to increase when multidisciplinary teams (MDT) of pulmonologists, radiologists, and pathologists confer;¹⁷ unfortunately not all patients and their physicians have access to this level of expert review by an experienced MDT. Such reviews are time consuming and require patients to be seen at regional centers of recognized expertise.

Accordingly, more effective methods of diagnosing IPF, e.g., more robust methods of detecting UIP in bronchoscopic sampling that does not rely on sufficient sampling of alveoli, are required. In addition, methods of differentiating UIP from non-UIP are required.

While gene expression profiling studies in the scientific literature have reported differential expression between IPF and other ILD subtypes,^{18,19} none, except for our prior application, PCT/US2015/059309, incorporated herein by reference in its entirety, have attempted to classify UIP in datasets containing other subtypes frequently present as part of the clinician's differential diagnosis. Further, none have utilized actual or *in silico* sample pooling to achieve higher sensitivity and/or specificity of differential diagnosis. Additionally, none have reported classifiers that are agnostic to cellular heterogeneity.

The methods described herein are surprisingly able to obtain a higher sensitivity and/or sensitivity for differential diagnosis by utilizing physical or *in silico* pooling of patient samples. Further, the methods described herein are surprisingly agnostic to cellular heterogeneity despite prior indications that cellular homogeneity was required. Thus, the present disclosure provides significant improvements over the prior art for using differential gene expression to distinguish between IPF and other ILD subtypes.

### SUMMARY

The present disclosure provides methods of and systems used for differentiating between samples as usual interstitial pneumonia (UIP) or non-UIP using classifiers. The accuracy of the methods described herein have been confirmed using expert pathology diagnoses as truth labels. Thus, the methods described herein provide a pathology surrogate test that accurately distinguishes UIP from non-UIP patterns in samples such as, e.g., transbronchial biopsies (TBBs).

In some embodiments, the present disclosure provides a method and/or system for detecting whether a lung tissue sample is positive for usual interstitial pneumonia (UIP) or non-usual interstitial pneumonia (non-UIP). In some embodiments, a method is provided for determining whether a lung tissue sample is positive for usual interstitial pneumonia (UIP) comprising detecting mRNA expression levels in a biological sample of one or more gene listed in Table 1, Table 5, Table 15, or a combination thereof. In particular embodiments, the present disclosure provides a method and/or system for detecting whether a lung tissue sample is positive for usual interstitial pneumonia (UIP) or non-usual interstitial pneumonia (non-UIP) comprising detecting mRNA expression levels in a biological sample of one or more gene listed in Table 5. In some embodiments, the method comprises detecting all of the genes listed in Table 5. In some embodiments, the methods further comprise transforming the expression levels (e.g., expression levels of the one or more genes listed in Table 5) determined above into an UIP-score that is indicative of the likelihood that the subject has IPF (e.g., as opposed to another ILD). In some embodiments, a risk score is determined according to a model having a Negative Predictive Value (NPV) of greater than 70% for ruling out UIP. In some embodiments, a risk score is determined according to a model having a Positive Predictive Value (PPV) of greater than 80% for diagnosing UIP. In some embodiments a method is provided for: assaying the expression level of each of a first group of transcripts and a second group of transcripts in a test sample of a subject, wherein the first group of transcripts includes any one or more of the genes overexpressed in UIP and listed in any of Table 1 and/or Table 15 and the second group of transcripts includes any one or more of the genes under-expressed in UIP and listed in any of Table 1 and/or Table 15. In some embodiments a method is provided for: assaying the expression level of each of a first group of transcripts and a second group of transcripts in a test sample of a subject, wherein the first group of transcripts includes any one or more of the genes overexpressed in UIP and listed in Table 5 and the second group of transcripts includes any one or more of the genes under-expressed in UIP and listed in Table 5. In some embodiment, the method further provides for comparing the expression level of each of the first group of transcripts and the second group of transcripts with reference expression levels of the corresponding transcripts to (1) classify the lung tissue as usual interstitial pneumonia (UIP) if there is (a) an increase in an expression level corresponding to the first group or (b) a decrease in an expression level corresponding to the second group as compared to the reference expression levels, or (2) classify the lung tissue as non-usual interstitial pneumonia (non-UIP) if there is (c) an increase in the expression level corresponding to the second group or (d) a decrease in the expression level corresponding to the first group as compared to the reference expression levels. In some embodiments, the method further provides for determining and/or comparing sequence variants for any of the one or more genes listed in Table 1 and/or Table 15. In some embodiments, the method provides for determining and/or comparing sequence variants for any of the one or more genes listed in Table 5.

In some embodiments, the present disclosure provides a method of detecting whether a lung tissue sample is positive for usual interstitial pneumonia (UIP) or non-usual interstitial pneumonia (non-UIP), comprising: Assaying the expression level of each of a first group of transcripts and a second group of transcripts in a test sample of a subject, wherein the first group of transcripts includes one or more sequences corresponding to any one of the genes overexpressed in UIP and listed in Table 1 and/or Table 15 and the second group of transcripts includes one or more sequence corresponding to any one of the genes under-expressed in UIP and listed in Table 1 and/or Table 15; and comparing the expression level of each of the first group of transcripts and the second group of transcripts with reference expression levels of the corresponding transcripts to (1) classify the lung tissue as usual interstitial pneumonia (UIP) if there is (a) an increase in an expression level corresponding to the first group and/or (b) a decrease in an expression level corresponding to the second group as compared to the reference expression levels, or (2) classify the lung tissue as non-usual interstitial pneumonia (non-UIP) if there is (c) an increase in the expression level corresponding to the second group and/or (d) a decrease in the expression level corresponding to the first group as compared to the reference expression levels.

In some embodiments, the present disclosure provides a method of detecting whether a lung tissue sample is positive for usual interstitial pneumonia (UIP) or non-usual interstitial pneumonia (non-UIP), comprising: Assaying the expression level of each of a first group of transcripts and a second group of transcripts in a test sample of a subject, wherein the first group of transcripts includes one or more sequences corresponding to any one of the genes overexpressed in UIP and Table 5 and the second group of transcripts includes one or more sequences corresponding to any one of the genes under-expressed in UIP and listed in Table 5; and comparing the expression level of each of the first group of transcripts and the second group of transcripts with reference expression levels of the corresponding transcripts to (1) classify the lung tissue as usual interstitial pneumonia (UIP) if there is (a) an increase in an expression level corresponding to the first group and/or (b) a decrease in an expression level corresponding to the second group as compared to the reference expression levels, or (2) classify the lung tissue as non-usual interstitial pneumonia (non-UIP) if there is (c) an increase in the expression level corresponding to the second group and/or (d) a decrease in the expression level corresponding to the first group as compared to the reference expression levels. In some embodiments, the present disclosures provides a method of detecting whether a lung tissue sample is positive for usual interstitial pneumonia (UIP) or non-usual interstitial pneumonia (non-UIP), comprising: assaying the expression level of each of a first group of transcripts and a second group of transcripts in a test sample of a subject, wherein the first group of transcripts includes one or more sequences corresponding to any one of the genes overexpressed in UIP and listed in Table 1, Table 5, and/or Table 15 and the second group of transcripts includes one or more sequences corresponding to any one of the genes under-expressed in UIP and listed in Table 1, Table 5, and/or Table 15; and comparing the expression level of each of the first group of transcripts and the second group of transcripts with reference expression levels of the corresponding transcripts to (1) classify the lung tissue as usual interstitial pneumonia (UIP) if there is (a) an increase in an expression level corresponding to the first group and/or (b) a decrease in an expression level corresponding to the second group as compared to the reference expression levels, or (2) classify the lung tissue as non-usual interstitial pneumonia (non-UIP) if there is (c) either no change in, or an increase in, the expression level corresponding to the second group and/or (d) no change in, or a decrease in, the expression level corresponding to the first group as compared to the reference expression levels.

In some embodiments, the present disclosure provides a method of detecting whether a lung tissue sample is positive for usual interstitial pneumonia (UIP) or non-usual interstitial pneumonia (non-UIP), comprising: assaying by sequencing, array hybridization, or nucleic acid amplification the expression level of each of a first group of transcripts and a second group of transcripts in a test sample from a lung tissue of a subject, wherein the first group of transcripts includes one or more sequences corresponding to any one of the genes overexpressed in UIP and listed in Table 1 and/or Table 15 and the second group of transcripts includes one or more sequences corresponding to any one of the genes under-expressed in UIP and listed in any of Table 1 and/or Table 15; and comparing the expression level of each of the first group of transcripts and the second group of transcripts with reference expression levels of the corresponding transcripts to (1) classify the lung tissue as usual interstitial pneumonia (UIP) if there is (a) an increase in an expression level corresponding to the first group and/or (b) a decrease in an expression level corresponding to the second group as compared to the reference expression levels, or (2) classify the lung tissue as non-usual interstitial pneumonia (non-UIP) if there is (c) an increase in the expression level corresponding to the second group and/or (d) a decrease in the expression level corresponding to the first group as compared to the reference expression levels.

In some embodiments, the present disclosure provides a method of detecting whether a lung tissue sample is positive for usual interstitial pneumonia (UIP) or non-usual interstitial pneumonia (non-UIP), comprising: assaying by sequencing, array hybridization, or nucleic acid amplification the expression level of each of a first group of transcripts and a second group of transcripts in a test sample from a lung tissue of a subject, wherein the first group of transcripts includes one or more sequences corresponding to any one of the genes overexpressed in UIP and listed in Table 5 and the second group of transcripts includes one or more sequences corresponding to any one of the genes under-expressed in UIP and listed in any of Table 5; and comparing the expression level of each of the first group of transcripts and the second group of transcripts with reference expression levels of the corresponding transcripts to (1) classify the lung tissue as usual interstitial pneumonia (UIP) if there is (a) an increase in an expression level corresponding to the first group and/or (b) a decrease in an expression level corresponding to the second group as compared to the reference expression levels, or (2) classify the lung tissue as non-usual interstitial pneumonia (non-UIP) if there is (c) an increase in the expression level corresponding to the second group and/or (d) a decrease in the expression level corresponding to the first group as compared to the reference expression levels.

In some embodiments, the first group comprises 2 or more different transcripts, or 3 or more, 4 or more, 5 or more, 10 or more, 15 or more, 20 or more, or more than 20 different transcripts.

In some embodiments, the second group comprises 2 or more different transcripts, or 3 or more, 4 or more, 5 or more, 10 or more, 15 or more, 20 or more, or more than 20 different transcripts.

In some embodiments, the present disclosure provides a method of detecting whether a lung tissue sample is positive for UIP or non-UIP, comprising: assaying the expression level of two or more transcripts expressed in a test sample; and using a computer generated classifier to classify the sample as UIP and non-UIP; wherein the classifier was trained using a heterogeneous spectrum of non-UIP pathology subtypes comprising HP, NSIP, sarcoidosis, RB, bronchiolitis, and organizing pneumonia (OP); and wherein the two or more transcripts expressed in the test sample are selected from any two or more sequences listed in Table 1 and/or Table 15, or any two or more of SEQ ID NOS: 1-151.

In some embodiments, the present disclosure provides a method of detecting whether a lung tissue sample is positive for UIP or non-UIP, comprising: assaying the expression level of two or more transcripts expressed in a test sample; and using a computer generated classifier to classify the sample as UIP and non-UIP; wherein the classifier was trained using a heterogeneous spectrum of non-UIP pathology subtypes comprising HP, NSIP, sarcoidosis, RB, bronchiolitis, and organizing pneumonia (OP); and wherein the two or more transcripts expressed in the test sample are selected from any two or more sequences listed in Table 5.

In some embodiments, the test sample is a pool of a plurality of samples obtained from the subject. In some embodiments, the pool comprises 2, 3, 4, or 5 samples obtained from the subj ect.

In some embodiments, the method comprises pooling expression level data from a plurality of individual samples obtained from the subject. In some embodiments, expression level data from 2, 3, 4, or 5 samples obtained from the subject are pooled.

In some embodiments, the test sample is a biopsy sample or a bronchoalveolar lavage sample. In some embodiments, the biopsy sample is a transbronchial biopsy sample. In some embodiments, the test sample is fresh-frozen or fixed.

In some embodiments, assaying the expression level is accomplished using RT-PCR, DNA microarray hybridization, RNASeq, or a combination thereof. In some embodiments, the expression level is assayed by detecting a nucleotide expressed in the test sample or synthesized from a nucleotide expressed in the test sample. In some embodiments, the method comprises synthesizing cDNA from RNA expressed in the test sample prior to assaying the expression level. In some embodiments, the method comprises synthesizing double-stranded cDNA from the cDNA prior to assaying the expression level. In some embodiments, the method comprises synthesizing non-natural RNA from the double-stranded cDNA prior to assaying the expression level. In some embodiments, the non-natural RNA is cRNA. In some embodiments, the non-natural RNA is labeled. In some embodiments, the label comprises a sequencing adaptor or a biotin molecule. In some embodiments, the method comprises amplification of the nucleotide prior to assaying the expression level.

In some embodiments, the method comprises labeling one or more of the transcripts. In some embodiments, the methods further comprise measuring the expression level of at least one control nucleic acid in the test sample.

In some embodiments, the method comprises classifying the lung tissue as any one of interstitial lung diseases (ILD), a particular type of ILD, a non-ILD, or non-diagnostic. In some embodiments, the lung tissue is classified as either idiopathic pulmonary fibrosis (IPF) or nonspecific interstitial pneumonia (NSIP). In some embodiments, the method comprises using smoking status as a covariate to the classification step(s). In some embodiments, smoking status is determined by detecting an expression profile indicative of the subject's smoker status.

In some embodiments, the classification of the sample comprises detection of the expression levels of one or more transcripts that are susceptible to smoker status bias, wherein the transcripts that are susceptible to smoker status bias are weighted differently than transcripts that are not susceptible to smoker bias.

In some embodiments, the classification of the sample comprises detection of the expression levels of one or more transcripts that are susceptible to smoker status bias, and wherein the transcripts that are susceptible to smoker status bias are excluded from the classification step.

In some embodiments, the method comprises implementing a classifier trained using one or more features selected from gene expression, variants, mutations, fusions, loss of heterozygoxity (LOH), and biological pathway effect. In some embodiments, the classifier is trained using features including gene expression, sequence variants, mutations, fusions, loss of heterozygoxity (LOH), and biological pathway effect.

In some embodiments, the classification step further comprises detecting sequence variants in the test sample and comparing the sequence variants to the respective sequences in a reference sample to classify the sample as UIP or non-UIP.

In some embodiments, the methods disclosed herein for detecting whether a lung tissue sample is positive for UIP or non-UIP further comprise treating the subject with a compound capable of treating IPF if the sample is classified as UIP. In some embodiments, the compound is an anti-fibrotic. In some embodiments, the compound is selected from pirfenidone, nintedanib, pharmaceutically acceptable salts thereof, and combinations thereof.

In some embodiments, the classifying performed in the methods disclosed herein for detecting whether a lung tissue sample is positive for UIP or non-UIP, results in a specificity of at least about 90% and a sensitivity of at least about 70%.

In some embodiments, the methods disclosed herein for detecting whether a lung tissue sample is positive for UIP or non-UIP comprise assaying expression data for at least two transcripts selected from SEQ ID NOS: 1-320. In some embodiments, the methods disclosed herein for detecting whether a lung tissue sample is positive for UIP or non-UIP comprises assaying expression data for each of SEQ ID NOS: 1-320.

In some embodiments, the methods disclosed herein for detecting whether a lung tissue sample is positive for UIP or non-UIP comprise assaying expression data for at least two genes selected from the genes listed in Table 5. In some embodiments, the methods disclosed herein for detecting whether a lung tissue sample is positive for UIP or non-UIP comprises assaying expression data for each of the genes listed in Table 5.

In some embodiments, the methods disclosed herein further comprise (i) obtaining a sample from a subject, (ii) subjecting a first portion of the sample to cytological analysis that indicates that the first portion of the sample is ambiguous or indeterminate, and (iii) assaying a second portion of the sample as the test sample. In some embodiments, the first portion and second portion are different portions.

In some embodiments, the comparing the expression level of each of the first group of transcripts and the second group of transcripts with reference expression levels of the corresponding transcripts is performed using a trained algorithm that is trained with a plurality of samples, wherein the test sample is independent of the plurality of samples.

In some embodiments, the present disclosure presents a method of treating a patient with undiagnosed idiopathic pulmonary fibrosis (IPF), comprising, (A) measuring by array, sequencing, or qRT-PCR the level of expression of at least two genes in one or more samples obtained from a subject's airway, wherein the genes are selected from those listed in Table 1 and/or Table 15, and wherein the method comprises (i) pooling at least two samples prior to the measuring step; (ii) pooling at least two sets of expression data independently measured from two separate samples; or a combination of (i) and (ii); and (B) administering a compound effective for treating IPF if: (i) the expression level of each of the at least two genes is increased as compared to reference expression levels of the corresponding transcripts; and/or (ii) the expression level of each of the at least two genes is decreased as compared to reference expression levels of the corresponding transcripts; and/or (iii) the expression level of at least one of the at least two genes increased as compared to reference expression levels of the corresponding transcripts and at least one of the at least two genes is decreased as compared to reference expression levels of the corresponding transcripts.

In some embodiments, the administering step is performed only if the increase in (i) and/or the decrease in (ii) is significant.

In some embodiments, the present disclosure presents a method of treating a patient with undiagnosed idiopathic pulmonary fibrosis (IPF) comprising, (A) measuring by array, sequencing, or qRT-PCR the level of expression of at least two genes in one or more samples obtained from a subject's airway, wherein the genes are selected from those listed in Table 5, and wherein the method comprises (i) pooling at least two samples prior to the measuring step; (ii) pooling at least two sets of expression data independently measured from two separate samples; or a combination of (i) and (ii); and (B) administering a compound effective for treating IPF if: (i) the expression level of each of the at least two genes is increased as compared to reference expression levels of the corresponding transcripts; and/or (ii) the expression level of each of the at least two genes is decreased as compared to reference expression levels of the corresponding transcripts; and/or (iii) the expression level of at least one of the at least two genes increased as compared to reference expression levels of the corresponding transcripts and at least one of the at least two genes is decreased as compared to reference expression levels of the corresponding transcripts.

In some embodiments, the administering step is performed only if the increase in (i) and/or the decrease in (ii) is significant.

In some embodiments, the present disclosure provides a method of detecting whether a pooled lung tissue test sample is positive for UIP or non-UIP, comprising: (A) assaying the expression level of one or more transcripts expressed in a test sample; and (B) classifying the test sample as UIP or non-UIP using a computer generated trained classifier; wherein the computer generated trained classifier is trained using expression levels of one or more transcripts expressed in a plurality of individual training samples obtained from a plurality of subjects, each training sample having a confirmed diagnoses of UIP or non-UIP, wherein at least two of the training samples were obtained from a single subject; and wherein the test sample is pooled prior to the classifying.

In some embodiments, the pooling comprises physical pooling. In some embodiments, the pooling comprises *in silico* pooling.

In some embodiments, the classifier training uses expression levels of one or more transcripts listed in Table 1 and/or Table 15. In some embodiments, the classifier training uses expression levels of one or more genes listed in Table 5. In some embodiments, the classifier training uses expression levels of all of the transcripts listed in Table 1. In some embodiments, the classifier training uses expression levels of all of the transcripts listed in Table 15. In some embodiments, the classifier training uses expression levels of all of the transcripts listed in Table 5. In some embodiments, the classifier training uses expression levels of all of the transcripts listed in Table 5 and one or more additional gene listed in Table 1 or Table 15. In some embodiments, the classifier training uses expression levels of all of the transcripts listed in Table 1 and in Table 15. In some embodiments, the computer generated trained classifier classifies the test sample as UIP or non-UIP based upon the expression level of one or more transcripts listed in Table 1 and/or Table 15. In some embodiments, the classifier classifies the test sample as UIP or non-UIP based upon the expression level of all of the transcripts listed in Table 1. In some embodiments, the classifier classifies the test sample as UIP or non-UIP based upon the expression level of all of the transcripts listed in Table 15. In some embodiments, the classifier classifies the test sample as UIP or non-UIP based upon the expression level of all of the transcripts listed in Table 1 and in Table 15. In some embodiments, the classifier training uses expression levels of all of the genes listed in Table 5. In some embodiments, the computer generated trained classifier classifies the test sample as UIP or non-UIP based upon the expression level of one or more transcript listed in Table 5. In some embodiments, the classifier classifies the test sample as UIP or non-UIP based upon the expression level of all of the transcripts listed in Table 5.

In some embodiments, the present disclosure provides a method of detecting whether a pooled lung tissue test sample is positive for a disease or condition comprising: (A) assaying the expression level of one or more transcripts expressed in a test sample; and (B) classifying the test sample as either positive for, or negative for, the disease or condition using a computer generated trained classifier; wherein the computer generated trained classifier is trained using expression levels of one or more transcripts expressed in a plurality of individual training samples obtained from a plurality of subjects, each training sample having a confirmed diagnoses of positive or negative for the disease or condition, wherein at least two of the training samples were obtained from a single subject; and wherein the test sample is pooled prior to the classifying.

In some embodiments, the pooling comprises physical pooling. In some embodiments, the pooling comprises *in silico* pooling. In some embodiments, the classifier classifies the sample based on the expression level of one or more gene listed in Table 5. In particular embodiments the classifier classifies the sample based on the expression level of all the genes listed in Table 5.

In some embodiments, the disease or condition is selected from: a lung disorder, lung cancer, interstitial lung disease (ILD), idiopathic pulmonary fibrosis (IPF), usual interstitial pneumonia (UIP) or non-usual interstitial pneumonia (non-UIP), acute lung injury, bronchiolitis, desquamative interstitial pneumonia, diffuse alveolar damage, emphysema, eosinophilic pneumonia, nonspecific interstitial pneumonia (NSIP) (including subtypes of cellular, mixed, or Favor), granulomatous disease, hypersensitivity pneumonitis (HP), Favor subtype hypersensitivity pneumonitis (Favor HP), organizing pneumonia, pneumocystis pneumonia, pulmonary hypertension, respiratory bronchiolitis, pulmonary sarcoidosis, smoking-related interstitial fibrosis, chronic obstructive pulmonary disease (COPD), a history of exposure to smoke, long-term exposure to smoke, short-term exposure to smoke, and chronic interstitial fibrosis.

In some embodiments, the present disclosure provides a method of treating a subject in need thereof with a therapeutic effective for treating idiopathic pulmonary fibrosis (IPF) comprising administering an effective dose of a compound effective for treating IPF to the subject in need thereof, wherein the subject in need thereof has an expression level of one or more genes in Table 5 that indicates the subject is in need of treatment for IPF as determined by a computer-generated trained classifier.

In some embodiments, the computer-generated trained classifier is trained using expression levels of one or more transcripts expressed in a plurality of individual training samples obtained from a plurality of subjects, each training sample having a confirmed diagnoses of UIP or non-UIP, wherein at least two of the training samples were obtained from a single subject; and wherein the test sample is pooled prior to the classifying. In particular embodiments, the computer-generated trained classifier identifies a sample obtained from the subject as UIP. In particular embodiments, the computer-generated trained classifier identifies a sample obtained from the subject as IPF.

In some embodiments, the present disclosure provides a method for identifying whether a subject is positive for a lung disorder, comprising: (a) obtaining a tissue sample of the subject; (b) subjecting a first portion of the tissue sample to cytological testing that indicates that the first portion is ambiguous or suspicious; (c) upon identifying that the first portion is ambiguous or suspicious, assaying a second portion of the tissue sample for an expression level of one or more markers associated with the lung disorder; (d) processing the expression level with a trained algorithm to generate a classification of the tissue sample as positive for the lung disorder at an accuracy of at least about 90%, wherein the trained algorithm is trained with a training set comprising a plurality of training samples, and wherein the tissue sample is independent of the plurality of samples; and (e) electronically outputting the classification, thereby identifying whether the subject is positive for the lung disorder.

In some embodiments, the tissue sample is a lung tissue sample. In some embodiments, the tissue sample is a non-lung tissue sample. In some embodiments, the non-lung tissue sample is a respiratory epithelium sample. In some embodiments, the respiratory epithelium sample is from a nose or mouth of the subject.

In some embodiments, the expression level is of a plurality of markers associated with UIP.

In some embodiments, the accuracy is at least about 95%.

In some embodiments, the classification is generated at a specificity of at least about 90%. In some embodiments, the classification is generated at a sensitivity of at least about 70%.

In some embodiments, the trained algorithm is configured to classify a lung tissue sample at an accuracy of at least about 90% across at least 100 independent test samples.

In some embodiments, the classification is electronically outputted on a graphical user interface of an electronic display of a user.

In some embodiments, the lung disorder is usual interstitial pneumonia (UIP) or non-usual interstitial pneumonia (non-UIP).

In some embodiments, the first portion is different than the second portion.

In some embodiments, the present disclosure provides a method for identifying whether a subject is positive for usual interstitial pneumonia (UIP) or non-usual interstitial pneumonia (non-UIP), comprising: (a) obtaining a tissue sample of the subject; (b) subjecting a first portion of the tissue sample to cytological testing that indicates that the first portion is ambiguous or suspicious; (c) upon identifying that the first portion is ambiguous or suspicious, assaying a second portion of the tissue sample for an expression level of one or more markers associated with UIP; (d) processing the expression level with a trained algorithm to generate a classification of the tissue sample as positive for UIP or non-UIP at an accuracy of at least about 90%, wherein the trained algorithm is trained with a training set comprising a plurality of training samples, and wherein the tissue sample is independent of the plurality of samples; and (e) electronically outputting the classification, thereby identifying whether the subject is positive for UIP or non-UIP.

Another aspect of the present disclosure provides a non-transitory computer readable medium comprising machine-executable code that, upon execution by one or more computer processors, implements any of the methods above or elsewhere herein.

Another aspect of the present disclosure provides a computer system comprising one or more computer processors and a non-transitory computer readable medium coupled thereto. The non-transitory computer-readable medium comprises machine-executable code that, upon execution by the one or more computer processors, implements any of the methods above or elsewhere herein.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### INCORPORATION BY REFERENCE

All publications, patents, and patent applications mentioned in this specification are herein incorporated by reference to the same extent as if each individual publication, patent, or patent application was specifically and individually indicated to be incorporated by reference. To the extent publications and patents or patent applications incorporated by reference contradict the disclosure contained in the specification, the specification is intended to supersede and/or take precedence over any such contradictory material.

### BRIEF DESCRIPTION OF THE DRAWINGS

The novel features of the disclosure are set forth with particularity in the appended claims. A better understanding of the features and advantages of the present disclosure will be obtained by reference to the following detailed description that sets forth illustrative embodiments, in which the principles of the disclosure are utilized, and the accompanying drawings (also "Figure" and "FIG." herein), of which:

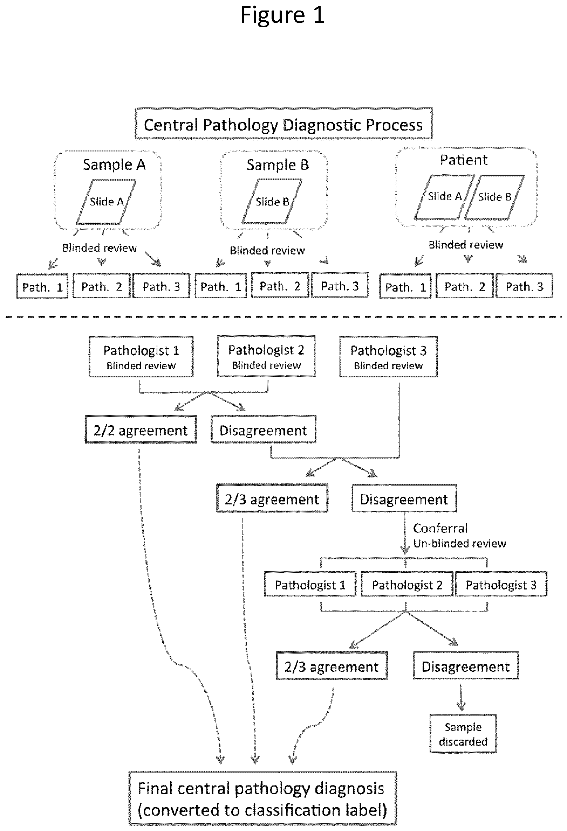

**Figure 1****.** Central pathology diagnostic process for a hypothetical patient with two samples (sample A and sample B). Three expert pathologists participate in the review process. For sample-level diagnosis, the glass slides for each sample are reviewed by each pathologist (Pathologist is abbreviated as Path.). For patient-level diagnosis, glass slides from all samples (two in this exercise) are gathered and reviewed together by each pathologist. Both sample-level and patient-level diagnoses go through the same review process. A majority vote is used as the final diagnosis, unless expert pathologists disagree even after the conferral, in which case, the sample is omitted due to lack of confidence in the diagnosis. Only a single such case was observed among all banked tissues (n=128).

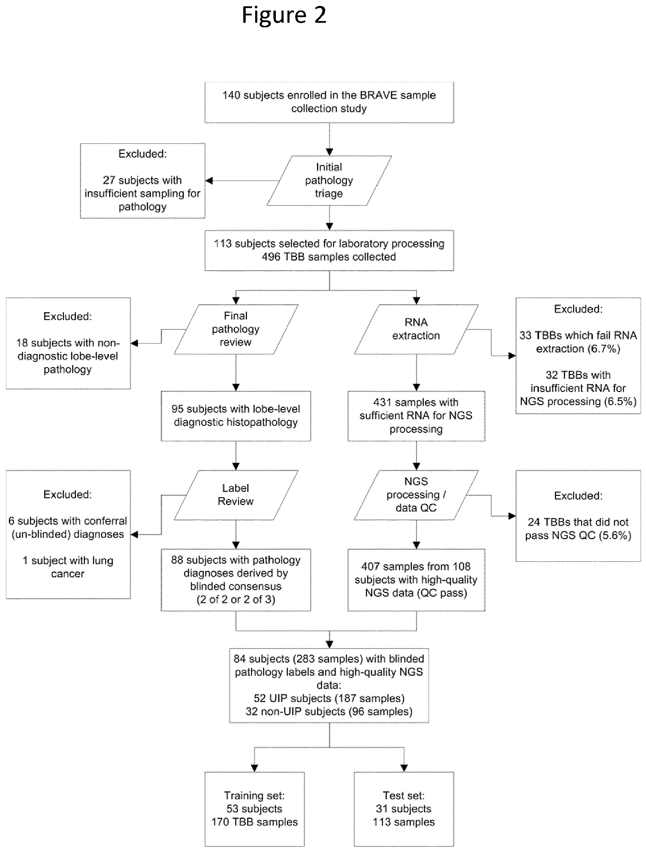

**Figure 2****.** Sample exclusion/inclusion procedure. Figure 2 shows a flow diagram of the 113 patients and associated TBB samples screened for use in this study. The figure illustrates the cohorts (central squares), processing steps (trapezoids), and exclusions (lateral squares), of patients and samples at each sequential step of processing.

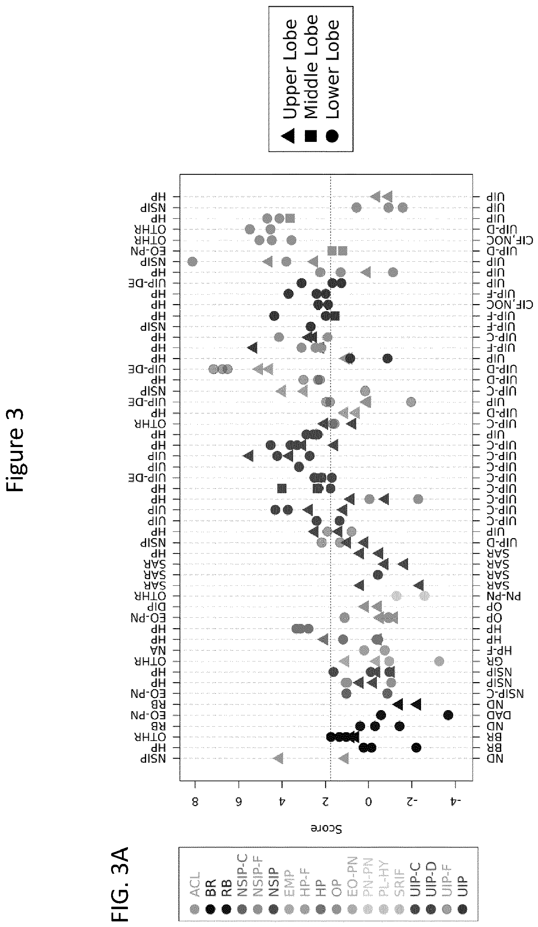

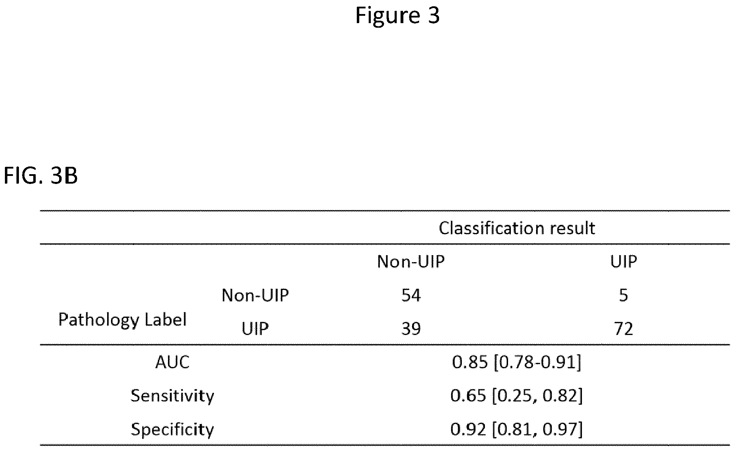

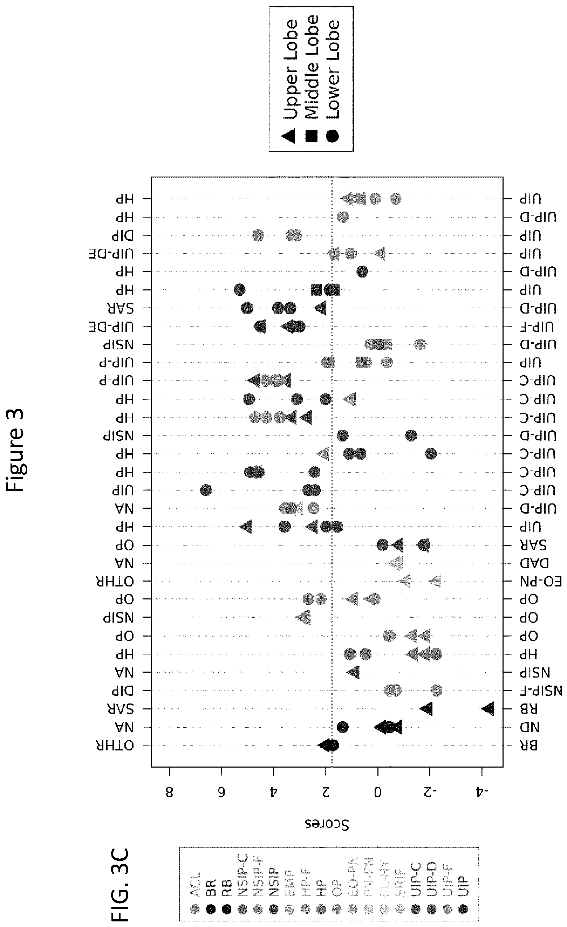

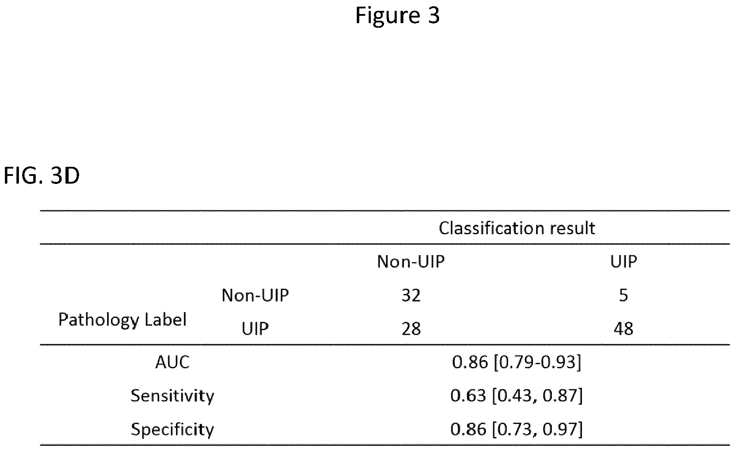

**Figure 3****.** Classifier performance. FIGS. 3A-3D show single-sample classification performance. A classifier trained on 53 patients was used to score, by cross validation, the individual TBB samples used in training (FIG. 3A, FIG. 3B) and to prospectively score TBB samples from an independent test cohort of 31 patients (FIG.3C, FIG. 3D). Classification scores (y-axis), organized vertically by patient, are plotted for each TBB sample in the training (FIG. 3A) and validation (FIG. 3C) sets. Individual samples are colored by lobe-level pathology diagnoses, with symbols denoting the lobe of origin (legends). Patient level pathology diagnoses are provided on the lower x-axis and radiology diagnoses are provided on the upper x-axis of each plot. The decision boundary, determined in cross validation on the training set and applied prospectively to the test set, is shown as a horizontal dashed line. Overall performance summaries when all samples are scored are provided in cross-validation on the training set (FIG. 3B) and prospectively on the validation set (FIG. 3D). The total numbers of true positive, true negative, false positive and false negative samples in each cohort are summarized in 2 x 2 tables. Receiver-operator characteristic areas under the curve (ROC-AUC), sensitivity and specificity, with associated 90% confidence intervals, are listed. Pathology and radiology acronyms used in Figure 3: ACL, acute lung injury; BR, bronchiolitis; CIF, NOC, chronic interstitial fibrosis, not otherwise classified; DIP, desquamative interstitial pneumonia; DAD, diffuse alveolar damage; EMP, emphysema; EO-PN, eosinophilic pneumonia; NA, not available/missing; ND, non-diagnostic; NSIP, nonspecific interstitial pneumonia; NSIP-C, cellular NSIP; NSIP-F, Favor NSIP; GR, granulomatous disease; HP, hypersensitivity pneumonitis; HP-F, Favor HP; OP, organizing pneumonia; OTHR, other; PN-PN, pneumocystis pneumonia; PL-HY, pulmonary hypertension; RB, respiratory bronchiolitis; SRC, sarcoidosis; SRIF, smoking-related interstitial fibrosis; UIP, usual interstitial pneumonia; UIP-C, classic UIP; UIP-D, difficult UIP; UIP-F, Favor UIP; UIP-DE, definite UIP; UIP-P, probable UIP.

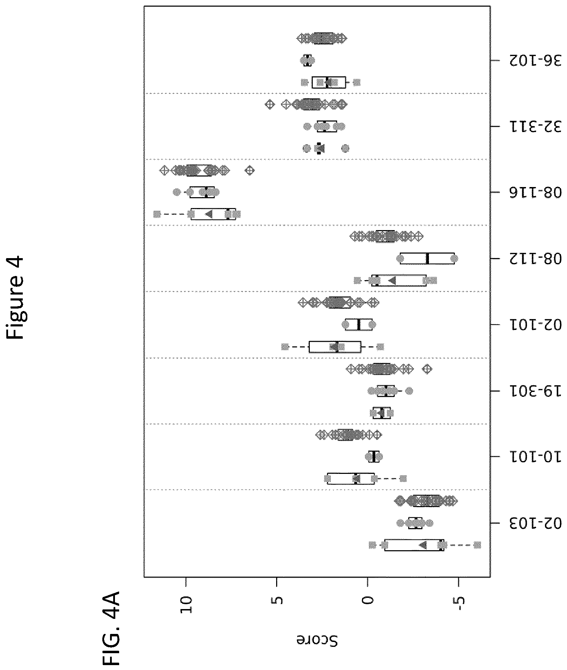

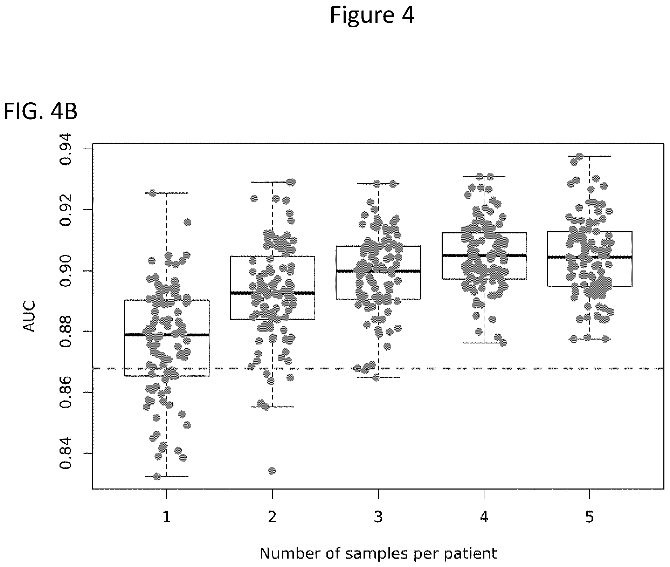

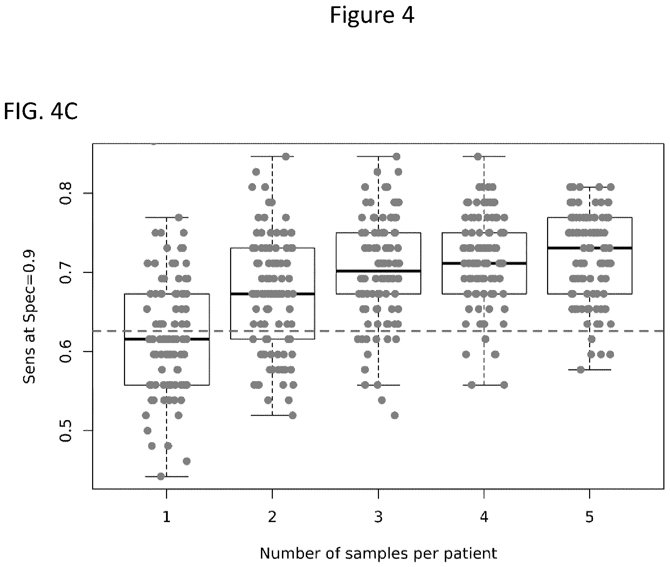

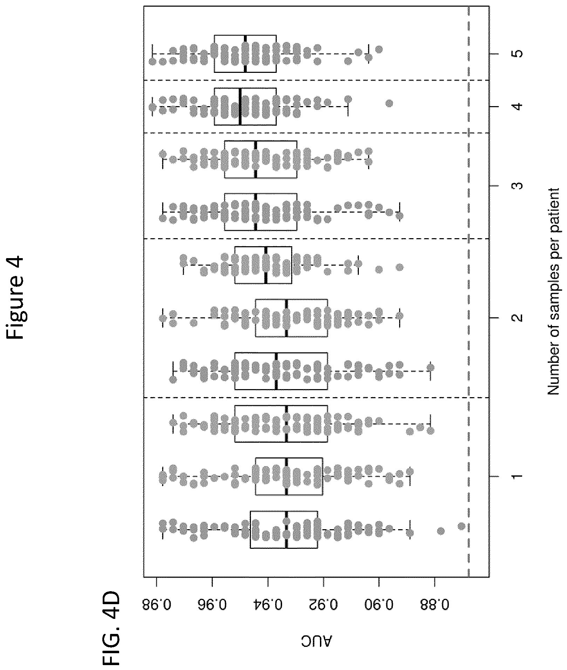

**Figure 4**: Classification of UIP in mixtures of TBBs from the same patient. FIG. 4A shows TBB samples from eight patients (x-axis), which were processed *in vitro* as individual samples and scored (y-axis) by the 84 patient classifier (blue squares). The average score for the individual TBB samples from each patient is shown for comparison (dark blue triangles). FIG. 4B shows *in silico* simulation of mixtures of multiple (2-5 per patient) TBB samples for the entire 84 patient cohort by random sampling of single-sample TBB data. Mixtures were scored by the 84 patient UIP classifier, and ROC-AUC point estimates for classification performance across the entire cohort were generated 100-fold, and plotted for each mixture condition. Box plots denote median ROC-AUCs at each sampling condition. FIG. 4C shows the performance shown in FIG. 4B, expressed as test sensitivity in mixtures at a targeted specificity of 90%. Test sensitivity improves to ~72% with reduced variability. The horizontal dashed red lines shows the ROC-AUC for the single sample classifier as a reference point. FIG. 4D shows mixture simulation in a set of 33 subjects with two upper lobe and three lower lobe TBBs available for every subject. There is no improvement in performance when sampling is restricted to the upper or lower lobes.

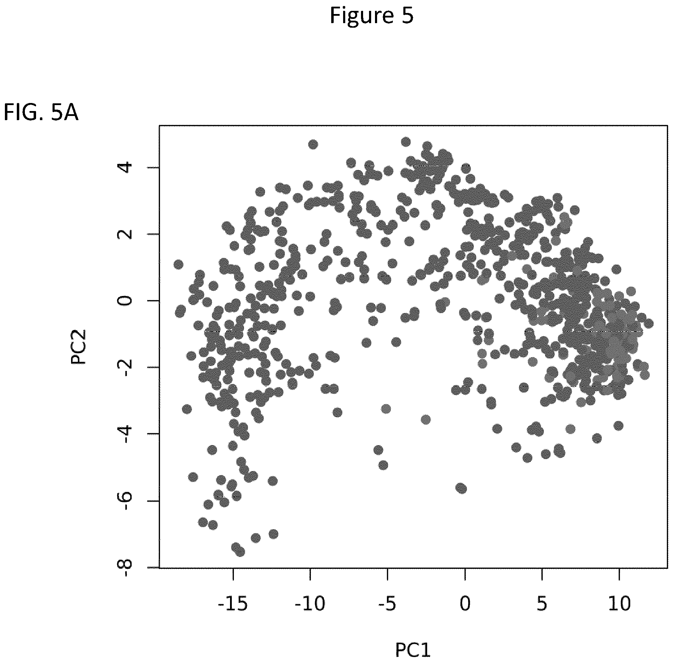

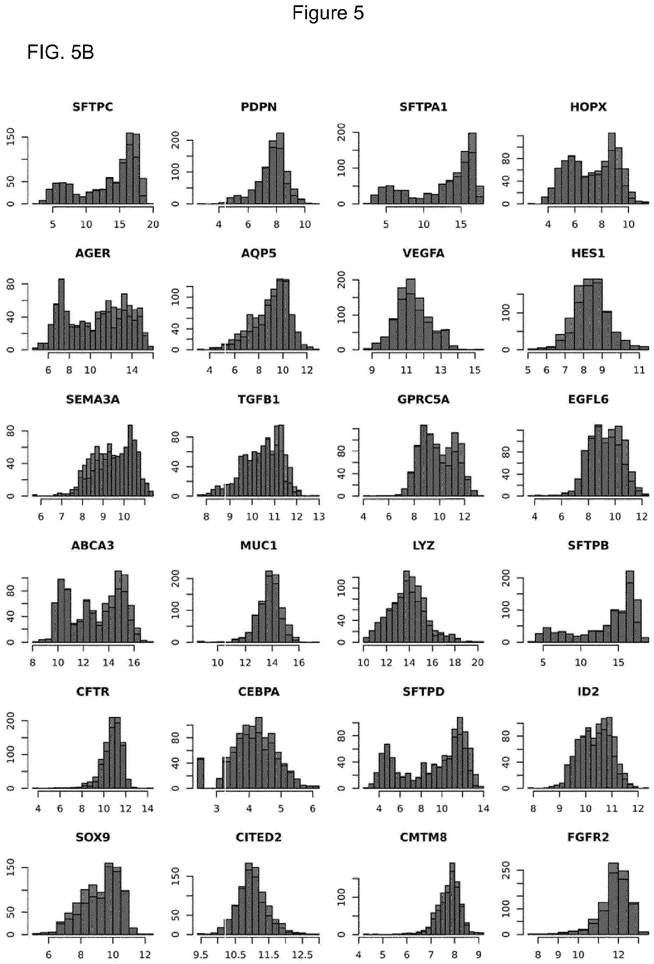

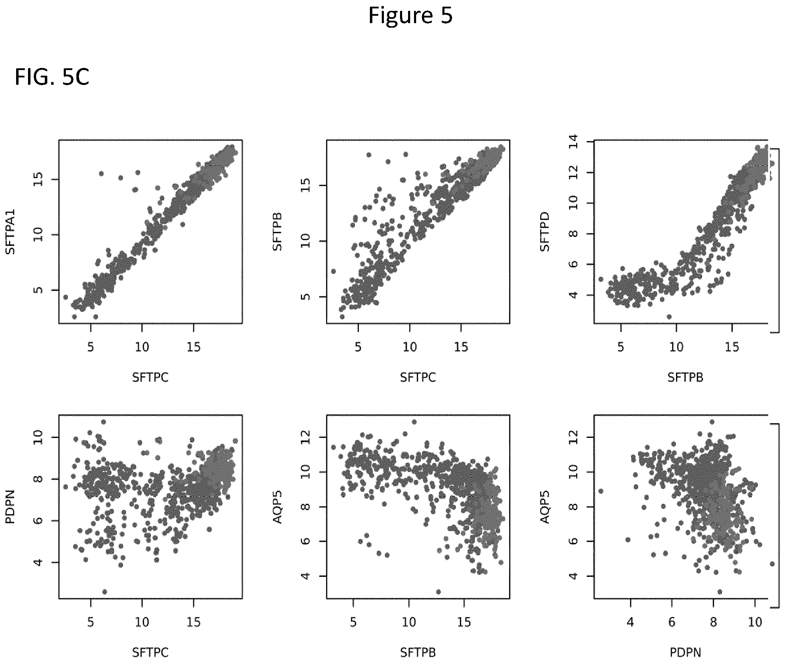

**Figure 5****.** FIG. 5A shows unsupervised clustering by principal components using 24 markers (a subset of the 44); TBB samples in blue, SLB samples in orange. FIG. 5B shows bimodal expression within the population of TBBs for 9 genes: SFTPB, SFTPC, SFTPD, ABCA3, CEBPA, AGER, GPRC5A, HOPX, and SFTPA1; TBB expression counts in blue, SLB expression counts in orange). FIG. 5C shows correlated, directionally consistent expression between SFTPA1, SFTPB, SFTPC, and SFTPD, but not between PDPN and AQP5, or between members of these two groups; TBB expression counts in blue, SLB expression counts in orange).

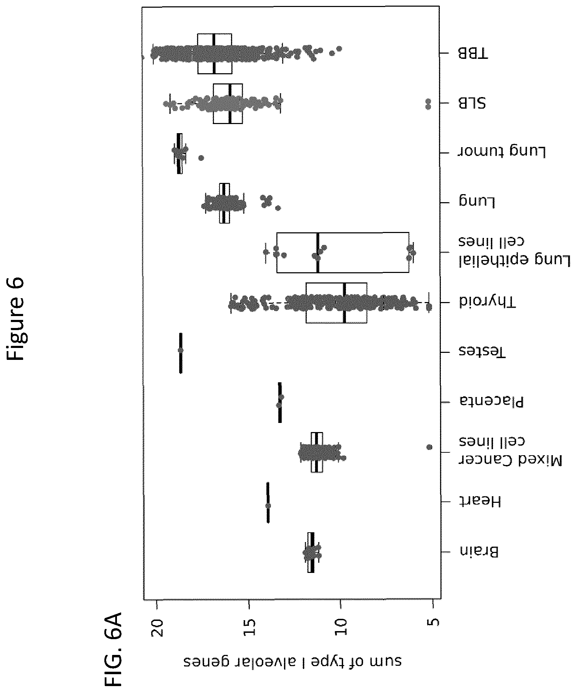

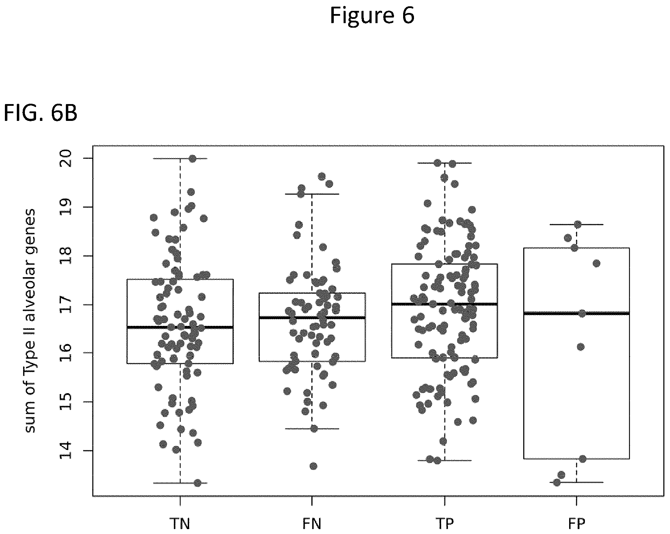

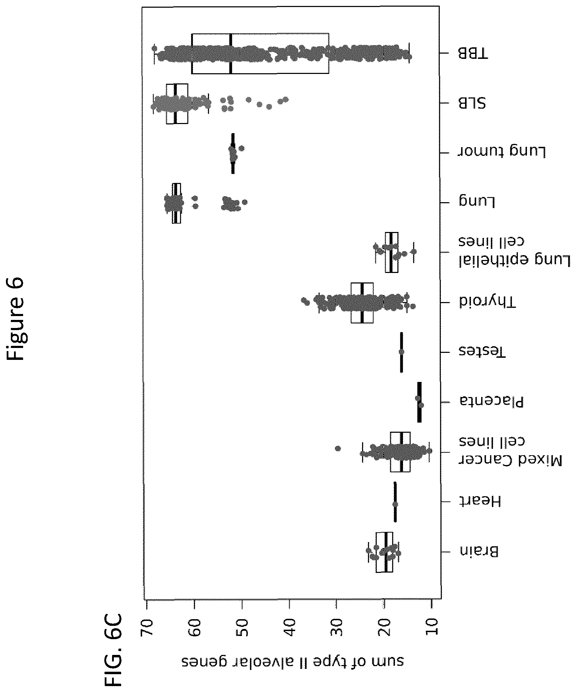

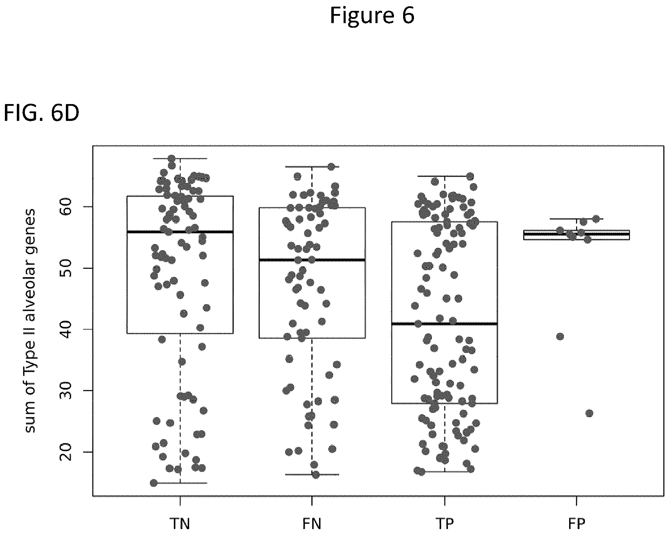

**Figure 6**: Distribution of alveolar gene expression in transbronchial biopsies. FIG. 6A shows the summed expression (type I alveolar statistic) of two markers of type I alveolar cells (y-axis) for multiple tissue, cell line and tumor types (x-axis). Expression in normal lung tissue, lung tumors, surgical lung biopsies (SLBs, n=22) and transbronchial biopsies in the current study is also shown for comparison (n=283). FIG. 6B shows type I alveolar statistics plotted for each TBB sample, grouped as a function of classification correctness relative to pathology truth labels (e.g., true negatives, false negatives, true positives, and false positives). FIG. 6C shows the summed expression (type II alveolar statistic) of four markers of alveolar cells (y-axis) for multiple tissue, cell line and tumor types (x-axis). Expression in normal lung tissue, lung tumors, surgical lung biopsies (SLBs, n=22) and transbronchial biopsies in the current study is also shown for comparison (n=283). FIG. 6D shows type II alveolar statistics plotted for each TBB sample, grouped as a function of classification correctness relative to pathology truth labels (e.g., true negatives, false negatives, true positives, and false positives). Pairwise correlation on explant samples obtained from three patients diagnosed with IPF (Patients P1, P2, and P3). Locations (upper or lower, central or peripheral) are indicated for each sample. The top 200 differentially expressed genes separating IPF samples from normal lung samples were used to compute pairwise Pearson correlation coefficients and plotted as a heatmap with higher correlation represented in magenta color, and lower correlation represented in green color. Correlation between and with normal lung samples are in the 0·7 range (not shown).

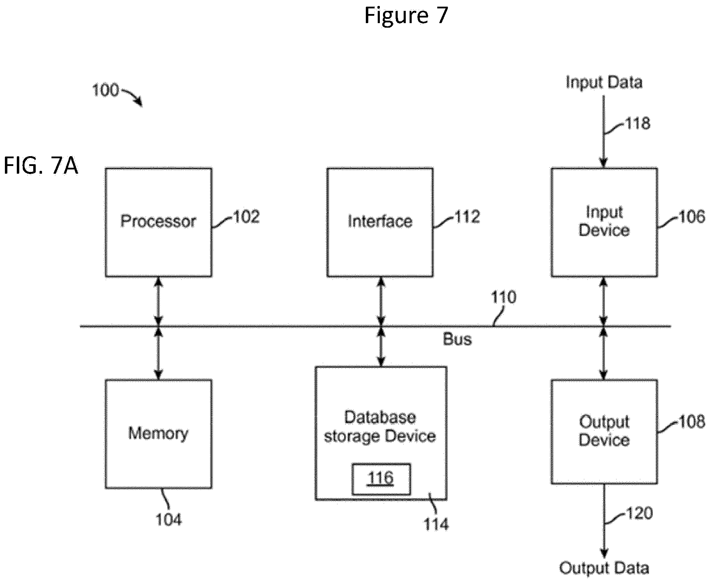

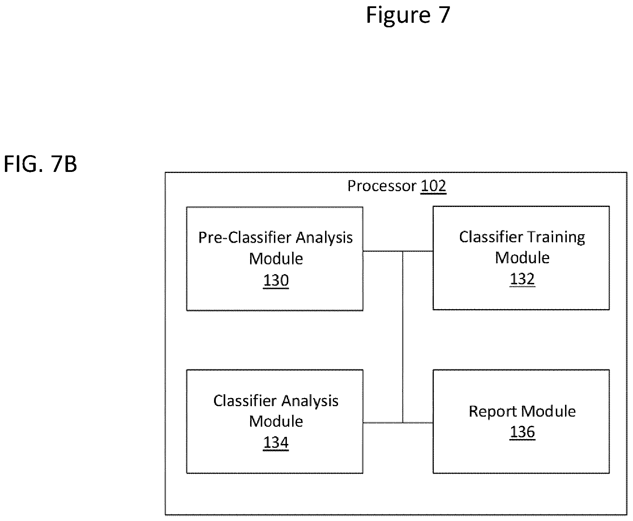

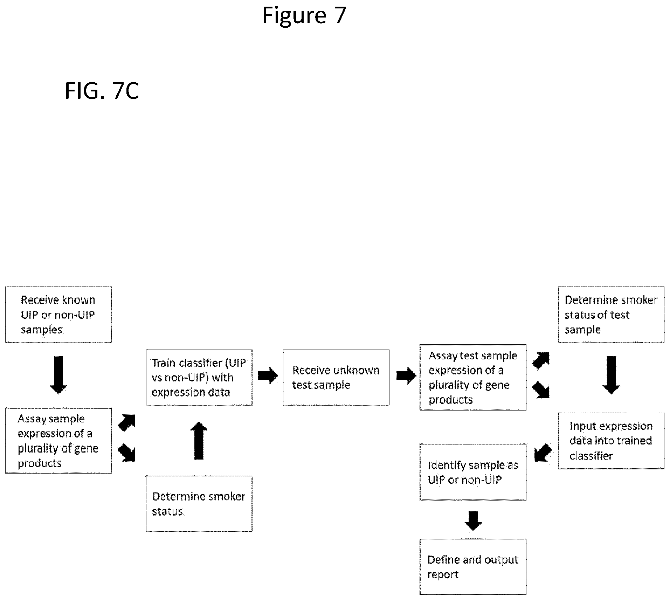

**Figure 7**. Computer systems; processors; and computer executable processes for training and utilizing the classifiers disclosed herein. FIG. 7A shows an illustration of a computer system usable for implementing aspects disclosed herein. FIG. 7B shows a detailed illustration of the processor of the computer system of FIG. 7A. FIG. 7C shows a detailed illustration of one non-limiting method of the present disclosure, wherein gene product expression data for known UIP and non-UIP samples are used to train a classifier (e.g., using a classifier training module) for differentiating UIP vs. non-UIP, wherein the classifier in some cases considers smoker status as a covariant, and wherein gene product expression data from unknown samples are input into the trained classifier to identify the unknown samples as either UIP or non-UIP, and wherein the results of the classification via the classifier are defined and output via a report.

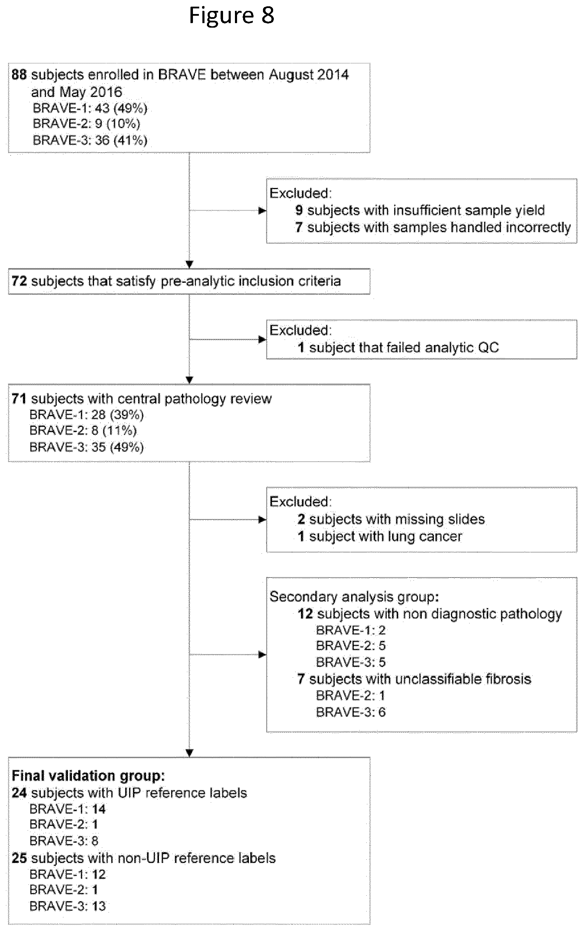

**Figure 8****.** Flow diagram illustrating the derivation of the Envisia final validation and secondary analysis groups from 88 BRAVE study subjects (Example 10).

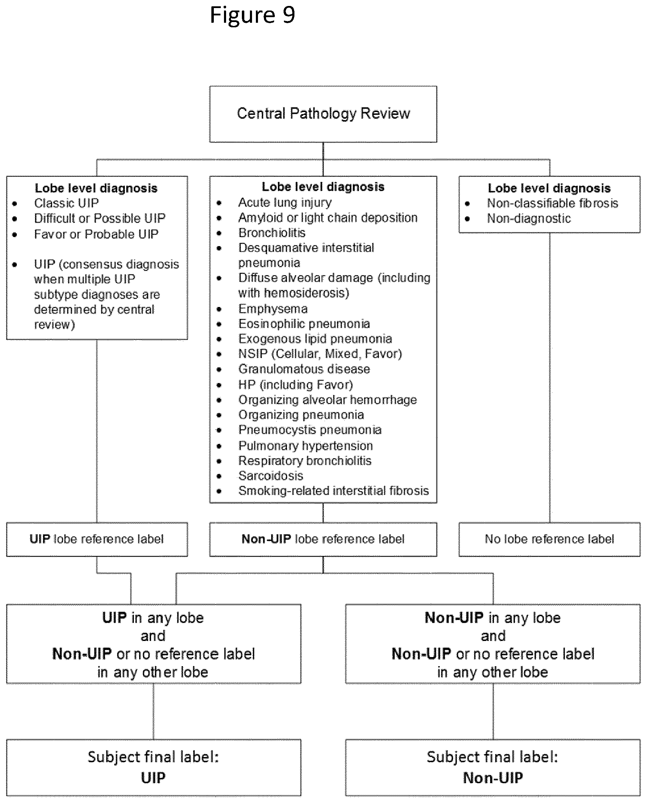

**Figure 9****.** Diagram of the central pathology review process used in the Example 10 study to determine reference labels for study subjects.

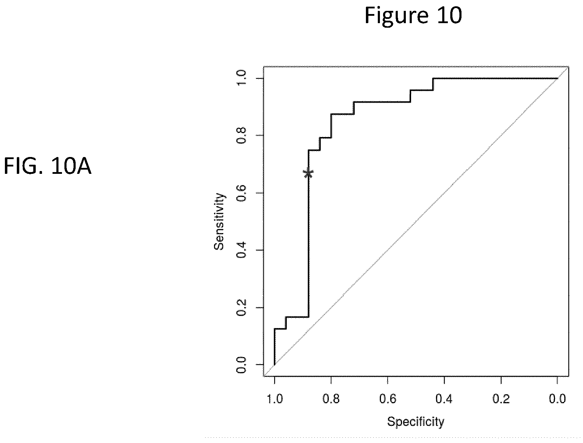

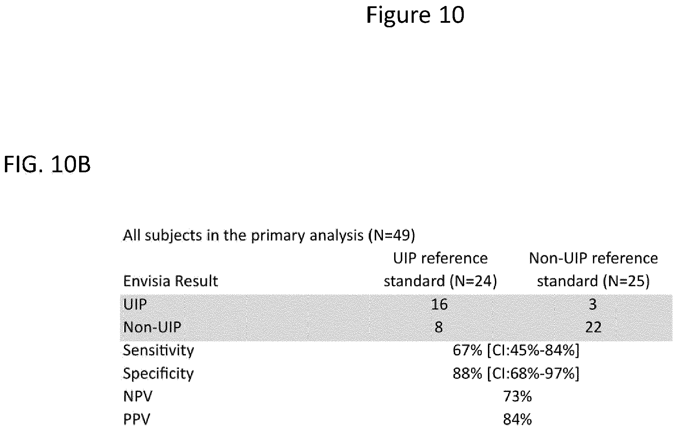

**Figure 10****.** Validation performance of the Envisia Genomic Classifier. FIG. 10A shows ROC-AUC curve for Envisia on the 49 subject final validation group, with the pre-specified decision boundary marked on the ROC curve with an asterisk. FIG. 10B shows a 2 x 2 table of Envisia classification results for the final validation group.

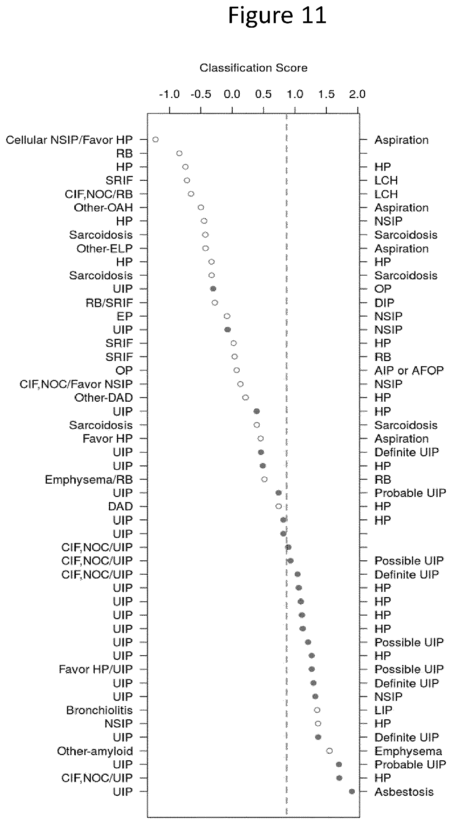

**Figure 11****.** Classification scores for 49 subjects in the Envisia validation group. Subjects were sorted left to right by increasing classification score (y-axis), with central pathology diagnoses on the lower x-axis and central radiology diagnoses (where available) on the upper x-axis. Solid circles represent subjects with UIP reference labels, hollow circles are subjects with non-UIP reference labels. The test decision boundary is shown with a dashed line.

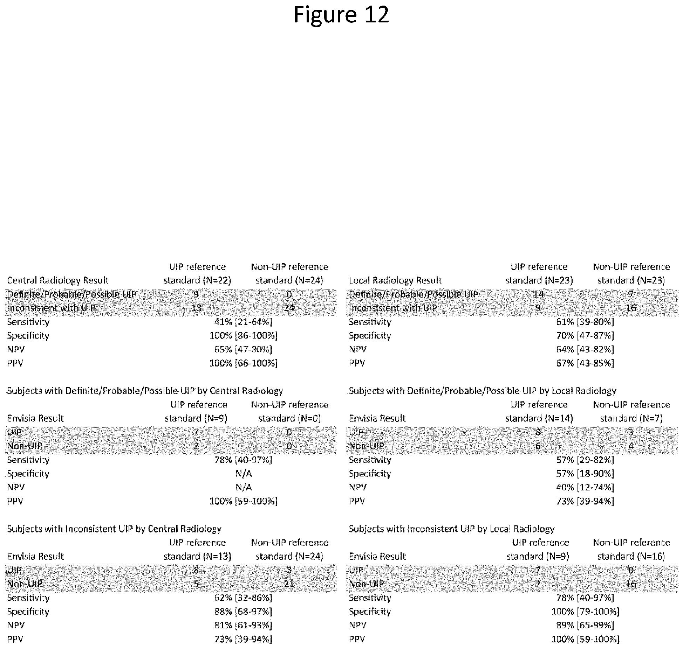

**Figure 12****.** Envisia performance in subject subgroups defined by radiology. 2 x 2 tables of central and local radiology diagnoses for 46 of the final validation subjects with available radiology are shown, against pathology as the reference standard. Envisia test performance against pathology is shown for the subsets of subjects with radiology consistent with UIP (Definite, Probable, and Possible UIP) and inconsistent with UIP. Envisia test results are evaluated separately against central and local radiology diagnoses.

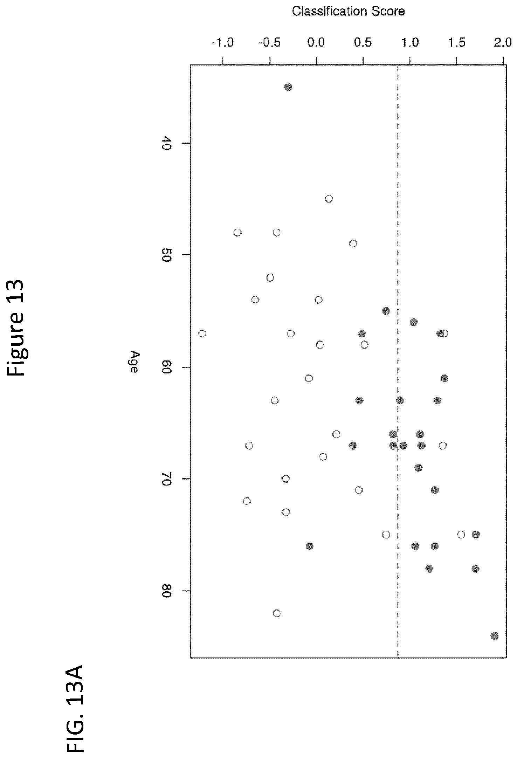

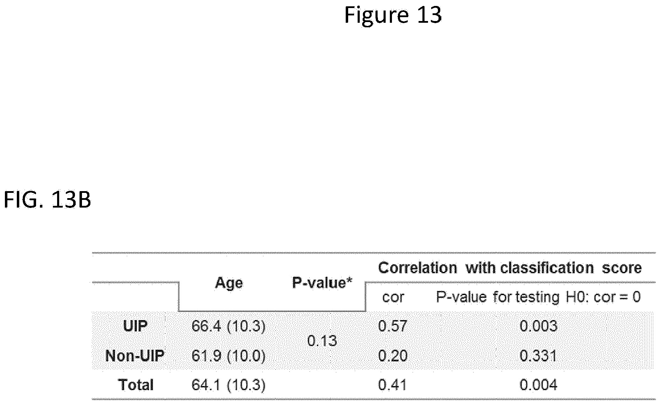

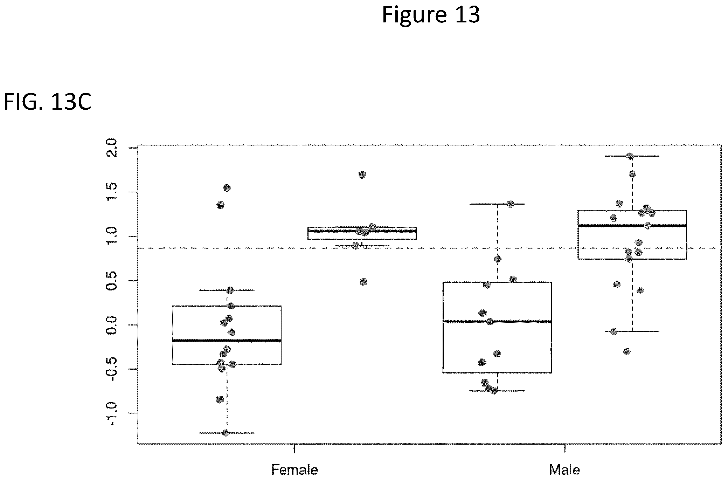

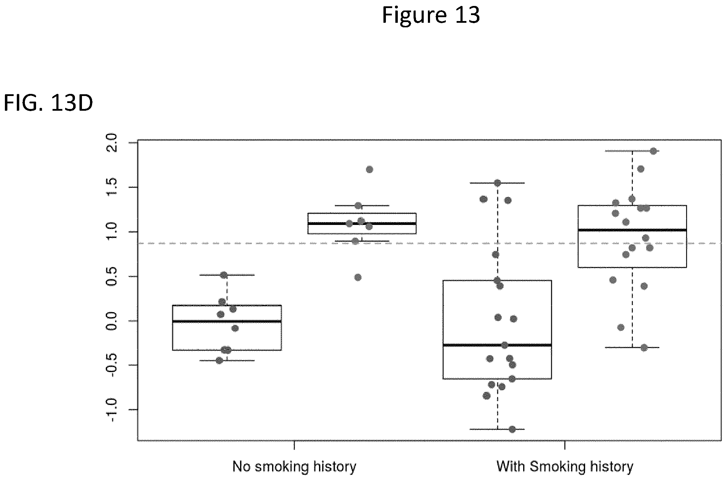

**Figure 13****.** Subgroup analysis of Envisia test performance against subject clinical factors. UIP subjects are marked in solid red circles, non-UIP subjects with hollow or blue circles. FIG. 13A: Envisia classification score as a function of validation cohort subject age. FIG. 13B: There is no significant correlation between subject age and classification score. FIG. 13C: Envisia score as a function of subject gender. Male patients with UIP have a greater tendency to be missed by the Envisia test (10 of 17 males with pathology UIP were called UIP by Envisia; 41% sensitivity, vs. 6 of 7 UIP females). FIG 13D: Envisia score as a function of subject smoking history. Male UIP patients with smoking history are misclassified by Envisia at a higher rate than nonsmokers.

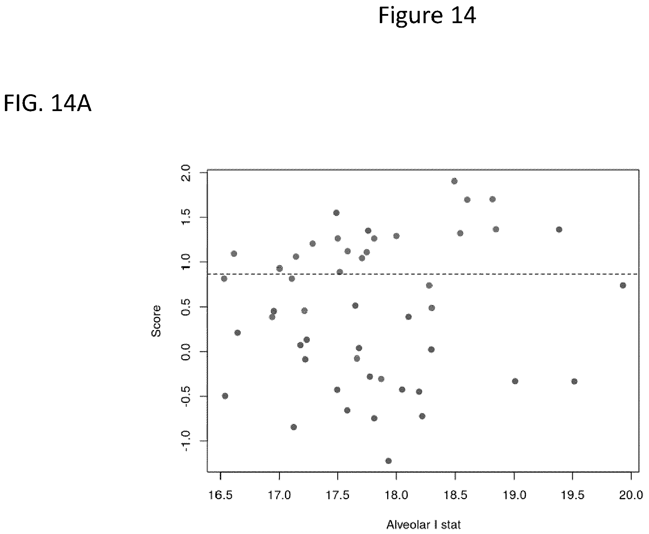

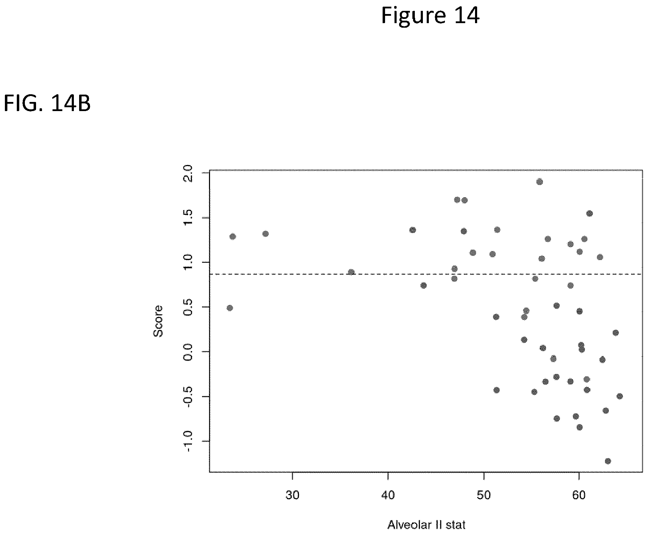

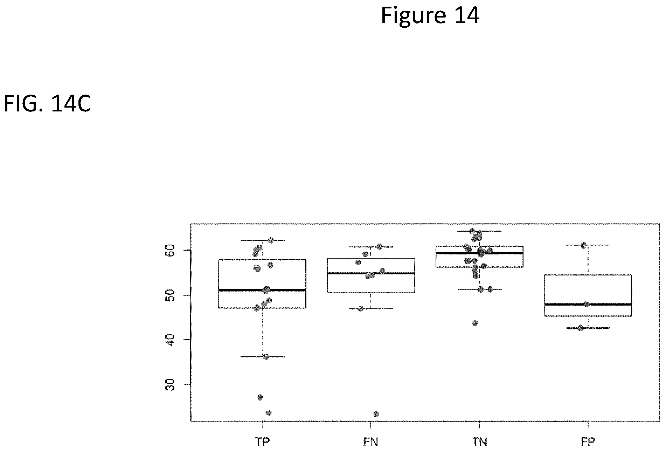

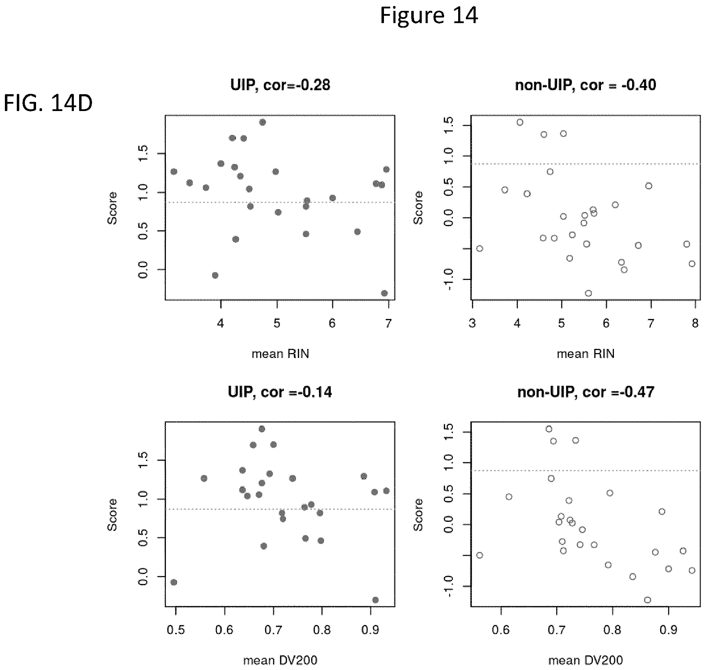

**Figure 14****.** Subgroup analysis of Envisia test performance against sample technical factors. UIP subjects are marked in solid red circles, non-UIP subjects with hollow or blue circles. FIGS. 14A and 14B: Summed gene expression statistics for estimating alveolar content. FIG. 14A shows alveolar type I cellular content (x-axis) and FIG. 14B shows alveolar type II content (x-axis)^{E10}, each are plotted against the Envisia score (y-axis). FIG. 14C: Envisia test true positives (TP), false negatives (FN), true negatives (TN), and false positives (FP) are plotted by alveolar type II content. There is no enrichment of low alveolar type II content among the subjects miscalled by the Envisia test. FIG. 14D: Correlation of Envisia classification score to sample quality (RIN or DV200), separated by UIP reference label. There is a correlation between stronger (more negative) classification scores and higher sample quality among non-UIP samples that is not evident in UIP samples.

### DETAILED DESCRIPTION

While various embodiments of the disclosure have been shown and described herein, it will be obvious to those skilled in the art that such embodiments are provided by way of example only. Numerous variations, changes, and substitutions may occur to those skilled in the art without departing from the disclosure. It should be understood that various alternatives to the embodiments of the disclosure described herein may be employed.

"Interstitial lung disease" or "ILD" (also known as diffuse parenchymal lung disease (DPLD)) as used herein refers to a group of lung diseases affecting the interstitium (the tissue and space around the air sacs of the lungs). ILD may be classified according to a suspected or known cause, or may be idiopathic. For example, ILD may be classified as caused by inhaled substances (inorganic or organic), drug induced (e.g., antibiotics, chemotherapeutic drugs, antiarrhythmic agents, statins), associated with connective tissue disease (e.g., systemic sclerosis, polymyositis, dermatomyositis, systemic lupus erythematous, rheumatoid arthritis), associated with pulmonary infection (e.g., atypical pneumonia, pneumocystis pneumonia (PCP), tuberculosis, chlamydia trachomatis, respiratory syncytial virus), associated with a malignancy (e.g., lymphangitic carcinomatosis), or may be idiopathic (e.g., sarcoidosis, idiopathic pulmonary fibrosis, Hamman-Rich syndrome, anti synthetase syndrome).

"ILD Inflammation" as used herein refers to an analytical grouping of inflammatory ILD subtypes characterized by underlying inflammation. These subtypes may be used collectively as a comparator against IPF and/or any other non-inflammation lung disease subtype. "ILD inflammation" can include HP, NSIP, sarcoidosis, and/or organizing pneumonia.

"Idiopathic interstitial pneumonia" or "IIP" (also referred to as noninfectious pneumonia" refers to a class of ILDs which includes, for example, desquamative interstitial pneumonia, nonspecific interstitial pneumonia, lymphoid interstitial pneumonia, cryptogenic organizing pneumonia, and idiopathic pulmonary fibrosis.

"Idiopathic pulmonary fibrosis" or "IPF" as used herein refers to a chronic, progressive form of lung disease characterized by fibrosis of the supporting framework (interstitium) of the lungs. By definition, the term is used when the cause of the pulmonary fibrosis is unknown ("idiopathic"). Microscopically, lung tissue from patients having IPF shows a characteristic set of histologic/pathologic features known as usual interstitial pneumonia (UIP), which is a pathologic counterpart of IPF.

"Nonspecific interstitial pneumonia" or "NSIP" is a form of idiopathic interstitial pneumonia generally characterized by a cellular pattern defined by chronic inflammatory cells with collagen deposition that is consistent or patchy, and a fibrosing pattern defined by a diffuse patchy fibrosis. In contrast to UIP, there is no honeycomb appearance nor fibroblast foci that characterize usual interstitial pneumonia.

"Hypersensitivity pneumonitis" or "HP" refers to also called extrinsic allergic alveolitis, (EAA) refers to an inflammation of the alveoli within the lung caused by an exaggerated immune response and hypersensitivity to as a result of an inhaled antigen (e.g., organic dust).

"Pulmonary sarcoidosis" or "PS" refers to a syndrome involving abnormal collections of chronic inflammatory cells (granulomas) that can form as nodules. The inflammatory process for HP generally involves the alveoli, small bronchi, and small blood vessels. In acute and subacute cases of HP, physical examination usually reveals dry rales.

The term "microarray" refers to an ordered arrangement of hybridizable array elements, preferably polynucleotide probes, on a substrate.

The term "polynucleotide," when used in singular or plural, generally refers to any polyribonucleotide or polydeoxribonucleotide, which may be unmodified RNA or DNA or modified RNA or DNA. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double-stranded or include single- and double- stranded regions. In addition, the term "polynucleotide" as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple -helical region often is an oligonucleotide. The term "polynucleotide" can also include DNAs (e.g., cDNAs) and RNAs that contain one or more modified bases (e.g., to provide a detectable signal, such as a fluorophore). Thus, DNAs or RNAs with backbones modified for stability or for other reasons are "polynucleotides" as that term is intended herein. Moreover, DNAs or RNAs comprising unusual bases, such as inosine, or modified bases, such as tritiated bases, are included within the term "polynucleotides" as defined herein. In general, the term "polynucleotide" embraces all chemically, enzymatically and/or metabolically modified forms of unmodified polynucleotides, as well as the chemical forms of DNA and RNA characteristic of viruses and cells, including simple and complex cells.

The term "oligonucleotide" refers to a relatively short polynucleotide (e.g., 100, 50, 20 or fewer nucleotides) including, without limitation, single-stranded deoxyribonucleotides, single- or double-stranded ribonucleotides, RNA:DNA hybrids and double-stranded DNAs. Oligonucleotides, such as single-stranded DNA probe oligonucleotides, are often synthesized by chemical methods, for example using automated oligonucleotide synthesizers that are commercially available. However, oligonucleotides may be made by a variety of other methods, including in vitro recombinant DNA-mediated techniques and by expression of DNAs in cells and organisms.

The terms "gene product" or "expression product" are used herein interchangeably to refer to the RNA transcription products (RNA transcript) of a gene, including mRNA, and the polypeptide translation product of such RNA transcripts. A gene product may be, for example, a polynucleotide gene expression product (e.g., an unspliced RNA, an mRNA, a splice variant mRNA, a microRNA, a fragmented RNA, and the like) or a protein expression product (e.g., a mature polypeptide, a post-translationally modified polypeptide, a splice variant polypeptide, and the like). In some embodiments the gene expression product may be a sequence variant including mutations, fusions, loss of heterozygoxity (LOH), and/or biological pathway effects.

The term "normalized expression level" as applied to a gene expression product refers to a level of the gene product normalized relative to one or more reference (or control) gene expression products.

A "reference expression level" as applied to a gene expression product refers to an expression level for one or more reference (or "control") gene expression products. A "reference normalized expression level" as applied to a gene expression product refers to a normalized expression level value for one or more reference (or control) gene expression products (i.e., a normalized reference expression level). In some embodiments, a reference expression level is an expression level for one or more gene product in normal sample, as described herein. In some embodiments, a reference expression level is determined experimentally. In some embodiments, a reference expression level is a historical expression level, e.g., a database value of a reference expression level in a normal sample, which sample indicates a single reference expression level, or a summary of a plurality of reference expression levels (such as, e.g., (i) an average of two or more, preferably three or more reference expression levels from replicate analysis of the reference expression level from a single sample; (ii) an average of two or more, preferably three or more reference expression levels from analysis of the reference expression level from a plurality of different samples (e.g., normal samples); (iii) and a combination of the above mentioned steps (i) and (ii) (i.e., average of reference expression levels analyzed from a plurality of samples, wherein at least one of the reference expression levels are analyzed in replicate). In some embodiments, the "reference expression level" is an expression level of sequence variants, for example, in a sample that has been definitively determined to be UIP or non-UIP by other approaches (i.e. confirmed pathological diagnosis).

A "reference expression level value" as applied to a gene expression product refers to an expression level value for one or more reference (or control) gene expression products. A "reference normalized expression level value" as applied to a gene expression product refers to a normalized expression level value for one or more reference (or control) gene expression products.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to re-anneal when complementary strands are present in an environment below their melting temperature. The higher the degree of desired homology between the probe and hybridizable sequence, the higher the relative temperature that may be used. As a result, it follows that higher relative temperatures may tend to make the reaction conditions more stringent, while lower temperatures less so. For additional details and explanation of stringency of hybridization reactions, see Ausubel et al., Current Protocols in Molecular Biology, (Wiley Interscience, 1995).

"Stringent conditions" or "high stringency conditions", as defined herein, typically: (1) employ low ionic strength solutions and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual (Cold Spring Harbor Press, 1989), and include the use of washing solution and hybridization conditions (e.g., temperature, ionic strength and %SDS) less stringent that those described above. An example of moderately stringent condition is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

"Sensitivity" as used herein refers to the proportion of true positives of the total number tested that actually have the target disorder (i.e., the proportion of patients with the target disorder who have a positive test result). "Specificity" as used herein refers to the proportion of true negatives of all the patients tested who actually do not have the target disorder (i.e., the proportion of patients without the target disorder who have a negative test result).

In the context of the present disclosure, reference to "at least one," "at least two," "at least five," etc. of the genes listed in any particular gene set means any one or any and all combinations of the genes listed.

The terms "splicing" and "RNA splicing" are used interchangeably and refer to RNA processing that removes introns and joins exons to produce mature mRNA with continuous coding sequence that moves into the cytoplasm of a eukaryotic cell.

"Therapeutically effective amount" or "Therapeutically effective dose" refers to an amount of a compound of the disclosure that, when administered to a subject, (e.g., preferably a mammal, more preferably a human), is sufficient to effect treatment, as defined below, of a disease or condition in the animal. The amount of a compound of the disclosure that constitutes a "therapeutically effective amount" will vary depending on the compound, the condition and its severity, the manner of administration, and the age of the subject to be treated, but can be determined routinely by one of ordinary skill in the art having regard to his own knowledge and to this disclosure. Accordingly when a compound administered at an "effective dose" this is intended to mean that the compound is capable of effecting treatment, as defined below, of a disease or condition (e.g., IPF) in a subject at such a dose.

"Treating" or "treatment" as used herein covers the treatment of the disease or condition of interest (e.g., IPF) in a subject, preferably a human, having the disease or condition of interest, and includes: (i) preventing or inhibiting the disease or condition from occurring in a subject, in particular, when such subject is predisposed to the condition but has not yet been diagnosed as having it; (ii) inhibiting the disease or condition, i.e., arresting its development; (iii) relieving the disease or condition, i.e., causing regression of the disease or condition; or (iv) relieving the symptoms resulting from the disease or condition. As used herein, the terms "disease," "disorder," and "condition" may be used interchangeably or may be different in that the particular malady, injury or condition may not have a known causative agent (so that etiology has not yet been worked out), and it is, therefore, not yet recognized as an injury or disease but only as an undesirable condition or syndrome, wherein a more or less specific set of symptoms have been identified by clinicians.

The term "exon" refers to any segment of an interrupted gene that is represented in a mature RNA product (B. Lewin, Genes 7V (Cell Press, 1990)). In theory the term "intron" refers to any segment of DNA that is transcribed but removed from within the transcript by splicing together the exons on either side of it. Operationally, exon sequences occur in the mRNA sequence of a gene as defined by Ref. SEQ ID numbers. Operationally, intron sequences are the intervening sequences within the genomic DNA of a gene, bracketed by exon sequences and usually having GT and AG splice consensus sequences at their 5' and 3' boundaries.

A "computer-based system" refers to a system of hardware, software, and data storage medium used to analyze information. Hardware of a patient computer-based system can include a central processing unit (CPU), and hardware for data input, data output (e.g., display), and data storage. The data storage medium can include any manufacture comprising a recording of the present information as described above, or a memory access device that can access such a manufacture.

As used herein the term "module" refers to any assembly and/or set of operatively-coupled electrical components that can include, for example, a memory, a processor, electrical traces, optical connectors, software (executing in hardware), and/or the like. For example, a module executed in the processor may be any combination of hardware-based module (e.g., a field-programmable gate array (FPGA), an application specific integrated circuit (ASIC), a digital signal processor (DSP)) and/or software-based module (e.g., a module of computer code stored in memory and/or executed at the processor) capable of performing one or more specific functions associated with that module.

To "record" data, programming or other information on a computer readable medium refers to a process for storing information, using various methods. Any convenient data storage structure may be chosen, based on the approaches used to access the stored information. A variety of data processor programs and formats may be used for storage, e.g. word processing text file, database format, etc.

A "processor" (or "computer processor") references any hardware and/or software combination that will perform the functions required of it. For example, a suitable processor may be a programmable digital microprocessor such as available in the form of an electronic controller, mainframe, server or personal computer (desktop or portable). Where the processor is programmable, suitable programming may be communicated from a remote location to the processor, or previously saved in a computer program product (such as a portable or fixed computer readable storage medium, whether magnetic, optical or solid state device based). For example, a magnetic medium or optical disk may carry the programming, and may be read by a suitable reader communicating with each processor at its corresponding station.

A "test sample" is a sample of one or more cells, preferable a tissue sample (e.g., a lung tissue sample such as a transbronchial biopsy (TBB) sample) obtained from a subject. In some embodiments, a test sample is a biopsy sample that may be obtained by various approaches (e.g., surgery). In particular embodiments, the test sample is a sample obtained by a video-assisted thoracoscopic surgery (VATS); a bronchoalveolar lavage (BAL); a transbronchial biopsy (TBB); or a cryo-transbronchial biopsy. A test sample may be obtained by an ancillary bronchoscopic procedure, such as brushing (such as by cytobrush, histobrush); bronchial biopsy; bronchial lavage; or needle-aspiration. The sample may be obtained by oral washings, touch preps, or sputum collection. The test sample may be obtained from a patient suspected of having a lung disease, e.g., an ILD, based on clinical signs and symptoms with which the patient presents (e.g., shortness of breath (generally aggravated by exertion), dry cough), and, in some cases the results of one or more of an imaging test (e.g., chest X-ray, computerized tomography (CT)), a pulmonary function test (e.g., spirometry, oximetry, exercise stress test), lung tissue analysis (e.g., histological and/or cytological analysis of samples obtained by bronchoscopy, bronchoalveolar lavage, surgical biopsy). In some embodiments, the test sample is obtained from a respiratory epithelium of the subject. The respiratory epithelium may be from the mouth, nose, pharynx, trachea, bronchi, bronchioles, or alveoli. However, other sources of respiratory epithelium also may be used. In some embodiments, the test sample is a pooled sample.

The term "pooling," is used herein to describe either (i) "physical pooling," i.e., actual mixing of samples together or (ii) "*in silico* pooling," *i.e.,* a method of pooling expression values of one or more genes detected in a sample. A non-limiting example of how such *in silico* pooling may be performed is outlined in Example 6. The terms "*in silico* mixing" and "*in silico* pooling" are used interchangeably herein. A sample, (e.g., a test sample) that comprises a plurality of samples that have undergone physical pooling may be refered to herein as a "pooled sample."

The term "subject," as used herein, generally refers to a mammal. Typically, the subject is a human. However, the term embraces other species, e.g., pigs, mice, rats, dogs, cats, or other primates. In certain embodiments, the subject is an experimental subject such as a mouse or rat. The subject may be a male or female. The subject may be an infant, a toddler, a child, a young adult, an adult or a geriatric. The subject may be a smoker, a former smoker or a nonsmoker. The subject may have a personal or family history of ILD. The subject may have an ILD-free personal or family history. The subject may exhibit one or more symptoms of ILD or another lung disorder (e.g., cancer, emphysema, COPD). For example, the subject may exhibit shortness of breath (generally aggravated by exertion) and/or dry cough), and, in some cases may have obtained results of one or more of an imaging test (e.g., chest X-ray, computerized tomography (CT)), a pulmonary function test (e.g., spirometry, oximetry, exercise stress test), lung tissue analysis (e.g., histological and/or cytological analysis of samples obtained by bronchoscopy, bronchoalveolar lavage, surgical biopsy) that is indicative of the potential presence of an ILD or another lung disorder. In some embodiments, a subject has or has been diagnosed with chronic obstructive pulmonary disease (COPD). In some embodiments, a subject does not have or has not been diagnosed with COPD. A subject under the care of a physician or other health care provider may be referred to as a "patient."

A "gene signature" is a gene expression pattern (*i.e.,* expression level of one or more gene, or fragments thereof), which is indicative of some characteristic or phenotype. In some embodiments, gene signature refers to the expression (and/or lack of expression) of a gene, a plurality of genes, a fragment of a gene or a plurality fragments of one or more genes, which expression and/or lack of expression is indicative of UIP, non-UIP, smoker-status, or non-smoker-status.

As used herein, "is a smoker" is meant to refer to a subject who currently smokes cigarettes or a person who has smoked cigarettes in the past or a person who has the gene signature of a person who currently smokes cigarettes or has smoked cigarettes in the past.

As used herein, "variant", when used to describe a feature used during training of a classifier of the present disclosure, refers to an alternative splice variant.

As used herein, "mutation", when used to describe a feature used during training of a classifier of the present disclosure, refers to a sequence deviation from a known normal reference sequence. In some embodiments, the deviation is a deviation from an accepted native gene sequence according to a publically accessible database such as the UniGene database (Pontius JU, Wagner L, Schuler GD. UniGene: a unified view of the transcriptome. In: The NCBI Handbook. Bethesda (MD): National Center for Biotechnology Information; 2003, incorporated herein), RefSeq (The NCBI handbook [Internet]. Bethesda (MD): National Library of Medicine (US), National Center for Biotechnology Information; 2002 Oct. Chapter 18, The Reference Sequence (RefSeq) Project, available at the World Wide Web address: ncbi.nlm.nih.gov/refseq/), Ensembl (EMBL, available at the World Wide Web address: ensembl.org/index.html), and the like. In some embodiments, the mutation includes an addition, deletion, or substitution of a sequence residue present in the reference sequence.

Abbreviations include: HRCT, high-resolution computed tomography; VATS, video-assisted thorascopic surgery; SLB, surgical lung biopsy; TBB, transbronchial biopsy; RB, respiratory bronchiolitis; OP, organizing pneumonia, DAD, diffuse alveolar damage, CIF/NOC, chronic interstitial fibrosis not otherwise classified; NMT, multidisciplinary team; CV, cross-validation; LOPO, leave-one-patient-out; ROC, receiver operator characteristic; AUC, area under the curve; RNASeq, RNA sequencing by next-generation sequencing technology; NGS, next-generation sequencing technology; H&E, hematoxylin and eosin; FDR, false discovery rate; IRB, Institutional Review Board; ATS, American Thoracic Society; COPD, chronic obstructive pulmonary disease; KEGG, Kyoto Encyclopedia of Genes and Genomes; CI, confidence interval.

Where a range of values is provided, it is understood that each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges, and are also encompassed within the disclosure, subject to any specifically excluded limit in the stated range. Where the stated range includes one or both of the limits, ranges excluding either or both of those included limits are also included in the disclosure. As used herein, "about" means plus or minus 10% of the indicated value.

### Methods for detecting usual interstitial pneumonia (UIP)

Disclosed herein are methods of and/or systems for using a molecular signature to differentiate UIP from non-UIP. The accurate diagnosis of UIP from samples (e.g., sample obtained from a patient) where expert pathology is not available stands to benefit ILD patients by accelerating diagnosis, thus facilitating treatment decisions and reducing surgical risk to patients and costs to the healthcare system.

Also disclosed herein are methods of and/or systems for using the smoker or non-smoker status of a subject to improve differentiation of UIP from other ILD subtypes using a molecular signature.

Thus, the methods and/or systems disclosed herein provide classifiers which can differentiate UIP from non-UIP patterns based on transcriptional data (e.g., high-dimensional transcriptional data) without prior knowledge of clinical or demographic information.

In some embodiments, the present disclosure provides methods for differentiating UIP from non-UIP using a classifier that comprises or consists of one or more sequences or fragments thereof presented in Table 1 and/or Table 15 or at least one sequence or fragment thereof from Table 1 and/or Table 15. In some embodiments, the present disclosure provides methods for differentiating UIP from non-UIP using a classifier that comprises or consists of one or more genes presented in Table 5 or at least one sequence or fragment thereof from Table 5. In some embodiments, the present disclosure provides methods for differentiating UIP from non-UIP using a classifier that comprises or consists of one or more sequences presented in Table 1 and/or Table 15 or at least one sequence from Table 1 and/or Table 15. In some embodiments, the present disclosure provides such methods that use a classifier comprising or consisting of at least 1, 2, 3, 4, 5, 6, 7, 8, 9, 10 or more of the sequences provided in Table 1 and/or Table 15. In some embodiments, the present disclosure provides such methods that use a classifier comprising or consisting of at least 1, 2, 3, 4, 5, 6, 7, 8, 9, 10 or more of the sequences provided in Table 5. For example, in some embodiments, the present disclosure provides such methods that use classifiers comprising or consisting of at least 11, 12, 13, 14, 15, 20, 30, 50, 100, 125, 150, or 151 sequences provided in Table 1, including all integers (e.g., 16, 17, 18, 19, 21, 22, 23, 24, 25 sequences, etc.) and ranges (e.g., from about 1-10 sequences from Table 1, from about 10-15 sequences, 10-20 sequences, 5-30 sequences, 5-50 sequences, 10-100 sequences, 50- 151 sequences, etc.) between. In some embodiments, the present disclosure provides such methods that use classifiers comprising or consisting of at least 11, 12, 13, 14, 15, 20, 30, 50, 100, 125, 150, or 169 sequences provided in Table 15, including all integers (e.g., 16, 17, 18, 19, 21, 22, 23, 24, 25 sequences, etc. from Table 15) and ranges (e.g., from about 1-10 sequences from Table 15, from about 10-15 sequences, 10-20 sequences, 5-30 sequences, 5-50 sequences, 10-100 sequences, 50-169 sequences, etc. from Table 15) between. In some embodiments, the present disclosure provides such methods that use classifiers comprising or consisting of at least 11, 12, 13, 14, 15, 20, 30, 50, 100, 125, 150, 160, 170, 180, 181, 182, 183, 184, 185, 186, 187, 188, 189, or 190 genes provided in Table 5, including all integers (e.g., 16, 17, 18, 19, 21, 22, 23, 24, 25 sequences, etc. from Table 5) and ranges (e.g., from about 1-10 sequences from Table 1, from about 10-15 sequences, 10-20 sequences, 5-30 sequences, 5-50 sequences, 10-100 sequences, 50-169 sequences, 60-190 sequence, etc. from Table 5) between. In some embodiments, the present disclosure provides such methods that use classifiers comprising or consisting of at least 11, 12, 13, 14, 15, 20, 30, 50, 100, 125, 150, 200, 250, 300, or 320 sequences provided in one or both of Table 1 and Table 15, including all integers (e.g., 16, 17, 18, 19, 21, 22, 23, 24, 25 sequences, etc. from Table 1 and/or Table 15) and ranges (e.g., from about 1-10 sequences from Table 1 and/or Table 15, from about 10-15 sequences, 10-20 sequences, 5-30 sequences, 5-50 sequences, 10-100 sequences, 50-200, 75-250, 100-300 sequences, etc. from Table 1 and/or Table 15) between. In some embodiments, the present disclosure provides such methods that use classifiers comprising or consisting of at least 11, 12, 13, 14, 15, 20, 30, 50, 100, 125, 150, 200, 250, 300, 320, 350, or more genes provided in one, two, or all of Tables 1, 5, and Table 15, including all integers (e.g., 16, 17, 18, 19, 21, 22, 23, 24, 25 sequences, etc. from Table 1, Table 5 and/or Table 15) and ranges (e.g., from about 1-10 sequences from Table 1, Table 5, and/or Table 15, from about 10-15 sequences, 10-20 sequences, 5-30 sequences, 5-50 sequences, 10-100 sequences, 50-200, 75-250, 100-300 sequences, etc. from Table 1, Table 5, and/or Table 15) between.

| **Table 1** | | | | | |
|---|---|---|---|---|---|
| **SEQ ID No** | **gene_id** | **Gene_Biotype** | **SEQ ID No** | **gene_id** | **Gene_Biotype** |
| 1. | ENSG00000162408 | prot_coding | 2. | ENSG00000163872 | prot_coding |
| 3. | ENSG00000116285 | prot_coding | 4. | ENSG00000197701 | prot_coding |
| 5. | ENSG00000219481 | prot_coding | 6. | ENSG00000168826 | prot_coding |
| 7. | ENSG00000204219 | prot_coding | 8. | ENSG00000178988 | prot_coding |
| 9. | ENSG00000117751 | prot_coding | 10. | ENSG00000178177 | prot_coding |
| 11. | ENSG00000159023 | prot_coding | 12. | ENSG00000109618 | prot_coding |
| 13. | ENSG00000116761 | prot_coding | 14. | ENSG00000250317 | prot_coding |
| 15. | ENSG00000117226 | prot_coding | 16. | ENSG00000081041 | prot_coding |
| 17. | ENSG00000163386 | prot_coding | 18. | ENSG00000145284 | prot_coding |
| 19. | ENSG00000186141 | prot_coding | 20. | ENSG00000163644 | prot_coding |
| 21. | ENSG00000122497 | prot_coding | 22. | ENSG00000163110 | prot_coding |
| 23. | ENSG00000203832 | prot_coding | 24. | ENSG00000138795 | prot_coding |
| 25. | ENSG00000143379 | prot_coding | 26. | ENSG00000205403 | prot_coding |
| 27. | ENSG00000143367 | prot_coding | 28. | ENSG00000153404 | prot_coding |
| 29. | ENSG00000163220 | prot_coding | 30. | ENSG00000206077 | prot_coding |
| 31. | ENSG00000007933 | prot_coding | 32. | ENSG00000145736 | prot_coding |
| 33. | ENSG00000143322 | prot_coding | 34. | ENSG00000145730 | prot_coding |
| 35. | ENSG00000174307 | prot_coding | 36. | ENSG00000168938 | prot_coding |
| 37. | ENSG00000143466 | prot_coding | 38. | ENSG00000113621 | prot_coding |
| 39. | ENSG00000135766 | prot_coding | 40. | ENSG00000120738 | prot_coding |
| 41. | ENSG00000163029 | prot_coding | 42. | ENSG00000253953 | prot_coding |
| 43. | ENSG00000115828 | prot_coding | 44. | ENSG00000261934 | prot_coding |
| 45. | ENSG00000135625 | prot_coding | 46. | ENSG00000155846 | prot_coding |
| 47. | ENSG00000115317 | prot_coding | 48. | ENSG00000186470 | prot_coding |
| 49. | ENSG00000228325 | prot_coding | 50. | ENSG00000026950 | prot_coding |
| 51. | ENSG00000074582 | prot_coding | 52. | ENSG00000137331 | prot_coding |
| 53. | ENSG00000123983 | prot_coding | 54. | ENSG00000244731 | prot_coding |
| 55. | ENSG00000144712 | prot_coding | 56. | ENSG00000240065 | prot_coding |
| 57. | ENSG00000168036 | prot_coding | 58. | ENSG00000204252 | prot_coding |
| 59. | ENSG00000187094 | prot_coding | 60. | ENSG00000137309 | prot_coding |
| 61. | ENSG00000179152 | prot_coding | 62. | ENSG00000137166 | prot_coding |
| 63. | ENSG00000173402 | prot_coding | 64. | ENSG00000124702 | prot_coding |
| 65. | ENSG00000163412 | prot_coding | 66. | ENSG00000112299 | prot_coding |
| 67. | ENSG00000227124 | prot_coding | 68. | ENSG00000111962 | prot_coding |
| 69. | ENSG00000184500 | prot_coding | 70. | ENSG00000112110 | prot_coding |
| 71. | ENSG00000181458 | prot_coding | 72. | ENSG00000048052 | prot_coding |
| 73. | ENSG00000034533 | prot_coding | 74. | ENSG00000006625 | prot_coding |
| 75. | ENSG00000198585 | prot_coding | 76. | ENSG00000075303 | prot_coding |
| 77. | ENSG00000172667 | prot_coding | 78. | ENSG00000158457 | prot_coding |
| 79. | ENSG00000078070 | prot_coding | 80. | ENSG00000050327 | prot_coding |
| 81. | ENSG00000033050 | prot_coding | 82. | ENSG00000072310 | prot_coding |
| 83. | ENSG00000105983 | prot_coding | 84. | ENSG00000108448 | prot_coding |
| 85. | ENSG00000164821 | prot_coding | 86. | ENSG00000141068 | prot_coding |
| 87. | ENSG00000012232 | prot_coding | 88. | ENSG00000196712 | prot_coding |
| 89. | ENSG00000130958 | prot_coding | 90. | ENSG00000242384 | prot_coding |
| 91. | ENSG00000041982 | prot_coding | 92. | ENSG00000073605 | prot_coding |
| 93. | ENSG00000136861 | prot_coding | 94. | ENSG00000167941 | prot_coding |
| 95. | ENSG00000136933 | prot_coding | 96. | ENSG00000154263 | prot_coding |
| 97. | ENSG00000160447 | prot_coding | 98. | ENSG00000161533 | prot_coding |
| 99. | ENSG00000148357 | prot_coding | 100. | ENSG00000181045 | prot_coding |
| 101. | ENSG00000170835 | prot_coding | 102. | ENSG00000211563 | miRNA |
| 103. | ENSG00000130653 | prot_coding | 104. | ENSG00000132199 | prot_coding |
| 105. | ENSG00000165997 | prot_coding | 106. | ENSG00000154655 | prot_coding |
| 107. | ENSG00000120539 | prot_coding | 108. | ENSG00000075643 | prot_coding |
| 109. | ENSG00000156113 | prot_coding | 110. | ENSG00000101000 | prot_coding |
| 111. | ENSG00000138166 | prot_coding | 112. | ENSG00000130005 | prot_coding |
| 113. | ENSG00000148925 | prot_coding | 114. | ENSG00000130513 | prot_coding |
| 115. | ENSG00000171714 | prot_coding | 116. | ENSG00000213965 | prot_coding |
| 117. | ENSG00000149090 | prot_coding | 118. | ENSG00000006659 | prot_coding |
| 119. | ENSG00000254761 | IincRNA | 120. | ENSG00000086544 | prot_coding |
| 121. | ENSG00000137474 | prot_coding | 122. | ENSG00000104812 | prot_coding |
| 123. | ENSG00000149289 | prot_coding | 124. | ENSG00000167757 | prot_coding |
| 125. | ENSG00000120647 | prot_coding | 126. | ENSG00000198464 | prot_coding |
| 127. | ENSG00000111679 | prot_coding | 128. | ENSG00000022556 | prot_coding |
| 129. | ENSG00000139197 | prot_coding | 130. | ENSG00000083814 | prot_coding |
| 131. | ENSG00000110900 | prot_coding | 132. | ENSG00000093072 | prot_coding |
| 133. | ENSG00000123358 | prot_coding | 134. | ENSG00000185133 | prot_coding |
| 135. | ENSG00000172789 | prot_coding | 136. | ENSG00000198792 | prot_coding |
| 137. | ENSG00000073910 | prot_coding | 138. | ENSG00000189306 | prot_coding |
| 139. | ENSG00000083544 | prot_coding | 140. | ENSG00000100376 | prot_coding |
| 141. | ENSG00000187630 | prot_coding | 142. | ENSG00000154642 | prot_coding |
| 143. | ENSG00000157379 | prot_coding | 144. | ENSG00000100557 | prot_coding |
| 145. | ENSG00000100592 | prot_coding | 146. | ENSG00000100650 | prot_coding |
| 147. | ENSG00000119711 | prot_coding | 148. | ENSG00000128891 | prot_coding |
| 149. | ENSG00000140718 | prot_coding | 150. | ENSG00000182810 | prot_coding |
| 151. | ENSG00000103044 | prot_coding | | | |

The ENSG identifiers listed herein (*i.e.,* the gene_ids) refer to gene identifiers for the Ensembl database available at the worldwide web address: ensembl.org, the content of which is incorporated herein by reference in its entirety.

In some particular embodiments, the present disclosure provides methods and/or systems for differentiating UIP from non-UIP using a classifier that comprises or consists of one or more of the sequences or fragments thereof listed in Table 1 and/or Table 15. In particular aspects, the classifier may contain 1, 2, 3, 4, 5, 6, 7, 8, or more additional genes. In other aspects, the classifier may omit 1, 2, 3, 4, 5, 6, 7, 8, or more, of these genes, while in some cases including other genes.

In some particular embodiments, the present disclosure provides methods and/or systems for differentiating UIP from non-UIP using a classifier that comprises or consists of one or more of the sequences or fragments thereof listed in Table 5. In particular aspects, the classifier may contain 1, 2, 3, 4, 5, 6, 7, 8, or more additional genes. In other aspects, the classifier may omit 1, 2, 3, 4, 5, 6, 7, 8, or more, of these genes, while in some cases including other genes. In certain embodiments, the present disclosure provides a method and/or system for differentiating UIP from non-UIP using the Envisia classifier, which may contain all of the genes listed in Table 5.

In some embodiments, the present disclosure provides a method and/or system for differentiating UIP from non-UIP using a classifier that comprises or consists of 2; 3; 4; 5; 6; 7; 8; 9; 10; 11; 12; 13; 14; 15; 16; 17; 18; 19; 20; 21; 22; 23; 24; 25; 26; 27; 28; 29; 30; 31; 32; 33; 34; 35; 36; 37; 38; 39; 40; 41; 42; 43; 44; 45; 46; 47; 48; 49; 50; 51; 52; 53; 54; 55; 56; 57; 58; 59; 60; 61; 62; 63; 64; 65; 66; 67; 68; 69; 70; 71; 72; 73; 74; 75; 76; 77; 78; 79; 80; 81; 82; 83; 84; 85; 86; 87; 88; 89; 90; 91; 92; 93; 94; 95; 96; 97; 98; 99; 100; 101; 102; 103; 104; 105; 106; 107; 108; 109; 110; 111; 112; 113; 114; 115; 116; 117; 118; 119; 120; 121; 122; 123; 124; 125; 126; 127; 128; 129; 130; 131; 132; 133; 134; 135; 136; 137; 138; 139; 140; 141; 142; 143; 144; 145; 146; 147; 148; 149; 150; or 151 of the following sequences: ENSG00000162408; ENSG00000116285; ENSG00000219481; ENSG00000204219; ENSG00000117751; ENSG00000159023; ENSG00000116761; ENSG00000117226; ENSG00000163386; ENSG00000186141; ENSG00000122497; ENSG00000203832; ENSG00000143379; ENSG00000143367; ENSG00000163220; ENSG00000007933; ENSG00000143322; ENSG00000174307; ENSG00000143466; ENSG00000135766; ENSG00000163029; ENSG00000115828; ENSG00000135625; ENSG00000115317; ENSG00000228325; ENSG00000074582; ENSG00000123983; ENSG00000144712; ENSG00000168036; ENSG00000187094; ENSG00000179152; ENSG00000173402; ENSG00000163412; ENSG00000227124; ENSG00000184500; ENSG00000181458; ENSG00000034533; ENSG00000198585; ENSG00000172667; ENSG00000078070; ENSG00000033050; ENSG00000105983; ENSG00000164821; ENSG00000012232; ENSG00000130958; ENSG00000041982; ENSG00000136861; ENSG00000136933; ENSG00000160447; ENSG00000148357; ENSG00000170835; ENSG00000130653; ENSG00000165997; ENSG00000120539; ENSG00000156113; ENSG00000138166; ENSG00000148925; ENSG00000171714; ENSG00000149090; ENSG00000254761; ENSG00000137474; ENSG00000149289; ENSG00000120647; ENSG00000111679; ENSG00000139197; ENSG00000110900; ENSG00000123358; ENSG00000172789; ENSG00000073910; ENSG00000083544; ENSG00000187630; ENSG00000157379; ENSG00000100557; ENSG00000100592; ENSG00000100650; ENSG00000119711; ENSG00000128891; ENSG00000140718; ENSG00000182810; ENSG00000103044; ENSG00000163872; ENSG00000197701; ENSG00000168826; ENSG00000178988; ENSG00000178177; ENSG00000109618; ENSG00000250317; ENSG00000081041; ENSG00000145284; ENSG00000163644; ENSG00000163110; ENSG00000138795; ENSG00000205403; ENSG00000153404; ENSG00000206077; ENSG00000145736; ENSG00000145730; ENSG00000168938; ENSG00000113621; ENSG00000120738; ENSG00000253953; ENSG00000261934; ENSG00000155846; ENSG00000186470; ENSG00000026950; ENSG00000137331; ENSG00000244731; ENSG00000240065; ENSG00000204252; ENSG00000137309; ENSG00000137166; ENSG00000124702; ENSG00000112299; ENSG00000111962; ENSG00000112110; ENSG00000048052; ENSG00000006625; ENSG00000075303; ENSG00000158457; ENSG00000050327; ENSG00000072310; ENSG00000108448; ENSG00000141068; ENSG00000196712; ENSG00000242384; ENSG00000073605; ENSG00000167941; ENSG00000154263; ENSG00000161533; ENSG00000181045; ENSG00000211563; ENSG00000132199; ENSG00000154655; ENSG00000075643; ENSG00000101000; ENSG00000130005; ENSG00000130513; ENSG00000213965; ENSG00000006659; ENSG00000086544; ENSG00000104812; ENSG00000167757; ENSG00000198464; ENSG00000022556; ENSG00000083814; ENSG00000093072; ENSG00000185133; ENSG00000198792; ENSG00000189306; ENSG00000100376; ENSG00000154642; alone or in any combination. In particular aspects, such a classifier contains additional genes, e.g., 1, 2, 3, 4, 5, 6, 7, 8, or more additional genes. In other aspects, the classifier omits certain of the above-mentioned genes, e.g., 1, 2, 3, 4, 5, 6, 7, 8, or more, of these genes, while in some cases including other genes.

In some embodiments, the present disclosure provides a method and/or system for differentiating UIP from non-UIP using a classifier that comprises or consists of all of the following sequences: ENSG00000162408; ENSG00000116285; ENSG00000219481; ENSG00000204219; ENSG00000117751; ENSG00000159023; ENSG00000116761; ENSG00000117226; ENSG00000163386; ENSG00000186141; ENSG00000122497; ENSG00000203832; ENSG00000143379; ENSG00000143367; ENSG00000163220; ENSG00000007933; ENSG00000143322; ENSG00000174307; ENSG00000143466; ENSG00000135766; ENSG00000163029; ENSG00000115828; ENSG00000135625; ENSG00000115317; ENSG00000228325; ENSG00000074582; ENSG00000123983; ENSG00000144712; ENSG00000168036; ENSG00000187094; ENSG00000179152; ENSG00000173402; ENSG00000163412; ENSG00000227124; ENSG00000184500; ENSG00000181458; ENSG00000034533; ENSG00000198585; ENSG00000172667; ENSG00000078070; ENSG00000033050; ENSG00000105983; ENSG00000164821; ENSG00000012232; ENSG00000130958; ENSG00000041982; ENSG00000136861; ENSG00000136933; ENSG00000160447; ENSG00000148357; ENSG00000170835; ENSG00000130653; ENSG00000165997; ENSG00000120539; ENSG00000156113; ENSG00000138166; ENSG00000148925; ENSG00000171714; ENSG00000149090; ENSG00000254761; ENSG00000137474; ENSG00000149289; ENSG00000120647; ENSG00000111679; ENSG00000139197; ENSG00000110900; ENSG00000123358; ENSG00000172789; ENSG00000073910; ENSG00000083544; ENSG00000187630; ENSG00000157379; ENSG00000100557; ENSG00000100592; ENSG00000100650; ENSG00000119711; ENSG00000128891; ENSG00000140718; ENSG00000182810; ENSG00000103044; ENSG00000163872; ENSG00000197701; ENSG00000168826; ENSG00000178988; ENSG00000178177; ENSG00000109618; ENSG00000250317; ENSG00000081041; ENSG00000145284; ENSG00000163644; ENSG00000163110; ENSG00000138795; ENSG00000205403; ENSG00000153404; ENSG00000206077; ENSG00000145736; ENSG00000145730; ENSG00000168938; ENSG00000113621; ENSG00000120738; ENSG00000253953; ENSG00000261934; ENSG00000155846; ENSG00000186470; ENSG00000026950; ENSG00000137331; ENSG00000244731; ENSG00000240065; ENSG00000204252; ENSG00000137309; ENSG00000137166; ENSG00000124702; ENSG00000112299; ENSG00000111962; ENSG00000112110; ENSG00000048052; ENSG00000006625; ENSG00000075303; ENSG00000158457; ENSG00000050327; ENSG00000072310; ENSG00000108448; ENSG00000141068; ENSG00000196712; ENSG00000242384; ENSG00000073605; ENSG00000167941; ENSG00000154263; ENSG00000161533; ENSG00000181045; ENSG00000211563; ENSG00000132199; ENSG00000154655; ENSG00000075643; ENSG00000101000; ENSG00000130005; ENSG00000130513; ENSG00000213965; ENSG00000006659; ENSG00000086544; ENSG00000104812; ENSG00000167757; ENSG00000198464; ENSG00000022556; ENSG00000083814; ENSG00000093072; ENSG00000185133; ENSG00000198792; ENSG00000189306; ENSG00000100376; ENSG00000154642. In particular aspects, the classifier contains 1, 2, 3, 4, 5, 6, 7, 8, or more additional genes.

In some embodiments, the present disclosure provides a method and/or system for differentiating UIP from non-UIP using a classifier that comprises or consists of 2; 3; 4; 5; 6; 7; 8; 9; 10; 11; 12; 13; 14; 15; 16; 17; 18; 19; 20; 21; 22; 23; 24; 25; 26; 27; 28; 29; 30; 31; 32; 33; 34; 35; 36; 37; 38; 39; 40; 41; 42; 43; 44; 45; 46; 47; 48; 49; 50; 51; 52; 53; 54; 55; 56; 57; 58; 59; 60; 61; 62; 63; 64; 65; 66; 67; 68; 69; 70; 71; 72; 73; 74; 75; 76; 77; 78; 79; 80; 81; 82; 83; 84; 85; 86; 87; 88; 89; 90; 91; 92; 93; 94; 95; 96; 97; 98; 99; 100; 101; 102; 103; 104; 105; 106; 107; 108; 109; 110; 111; 112; 113; 114; 115; 116; 117; 118; 119; 120; 121; 122; 123; 124; 125; 126; 127; 128; 129; 130; 131; 132; 133; 134; 135; 136; 137; 138; 139; 140; 141; 142; 143; 144; 145; 146; 147; 148; 149; 150; 151; 152; 153; 154; 155; 156; 157; 158; 159; 160; 161; 162; 163; 164; 165; 166; 167; 168; 169; 170; 171; 172; 173; 174; 175; 176; 177; 178; 179; 180; 181; 182; 183; 184; 185; 186; 187; 188; 189; or 190 of the following genes; alone or in combination: ENSG00000005381; ENSG00000005955; ENSG00000007908; ENSG00000007933; ENSG00000010379; ENSG00000012232; ENSG00000022556; ENSG00000026950; ENSG00000033050; ENSG00000038295; ENSG00000048052; ENSG00000054803; ENSG00000054938; ENSG00000060688; ENSG00000071909; ENSG00000072310; ENSG00000073605; ENSG00000078070; ENSG00000079385; ENSG00000081041; ENSG00000081985; ENSG00000082781; ENSG00000083814; ENSG00000086544; ENSG00000089902; ENSG00000092295; ENSG00000099251; ENSG00000099974; ENSG00000100376; ENSG00000100557; ENSG00000101544; ENSG00000102837; ENSG00000103044; ENSG00000103257; ENSG00000104812; ENSG00000105255; ENSG00000105559; ENSG00000105696; ENSG00000105784; ENSG00000105983; ENSG00000106018; ENSG00000106178; ENSG00000107929; ENSG00000108312; ENSG00000108551; ENSG00000109205; ENSG00000110092; ENSG00000110900; ENSG00000110975; ENSG00000111218; ENSG00000111321; ENSG00000111328; ENSG00000112164; ENSG00000112299; ENSG00000112852; ENSG00000114248; ENSG00000114923; ENSG00000115415; ENSG00000115607; ENSG00000116285; ENSG00000116761; ENSG00000119711; ENSG00000119725; ENSG00000120217; ENSG00000120738; ENSG00000120903; ENSG00000121380; ENSG00000121417; ENSG00000122497; ENSG00000124205; ENSG00000124702; ENSG00000124935; ENSG00000125255; ENSG00000128016; ENSG00000128266; ENSG00000128791; ENSG00000128891; ENSG00000130164; ENSG00000130487; ENSG00000130598; ENSG00000131095; ENSG00000131142; ENSG00000132199; ENSG00000132204; ENSG00000132915; ENSG00000132938; ENSG00000133636; ENSG00000133794; ENSG00000134028; ENSG00000134245; ENSG00000135148; ENSG00000135447; ENSG00000135625; ENSG00000136881; ENSG00000136883; ENSG00000136928; ENSG00000136933; ENSG00000137285; ENSG00000137463; ENSG00000137573; ENSG00000137709; ENSG00000137968; ENSG00000138166; ENSG00000138308; ENSG00000140274; ENSG00000140279; ENSG00000140323; ENSG00000140450; ENSG00000140465; ENSG00000140505; ENSG00000140718; ENSG00000141279; ENSG00000142178; ENSG00000142661; ENSG00000143185; ENSG00000143195; ENSG00000143320; ENSG00000143322; ENSG00000143367; ENSG00000143379; ENSG00000143603; ENSG00000144655; ENSG00000145248; ENSG00000145284; ENSG00000145358; ENSG00000145736; ENSG00000148541; ENSG00000148700; ENSG00000148702; ENSG00000149043; ENSG00000149289; ENSG00000151012; ENSG00000151572; ENSG00000152672; ENSG00000153404; ENSG00000154227; ENSG00000154451; ENSG00000156414; ENSG00000157103; ENSG00000157680; ENSG00000158457; ENSG00000159231; ENSG00000159674; ENSG00000161609; ENSG00000162594; ENSG00000163029; ENSG00000163110; ENSG00000163285; ENSG00000163412; ENSG00000163635; ENSG00000163644; ENSG00000163735; ENSG00000163817; ENSG00000163884; ENSG00000164604; ENSG00000164821; ENSG00000165948; ENSG00000165973; ENSG00000165983; ENSG00000166923; ENSG00000167748; ENSG00000168004; ENSG00000168036; ENSG00000168062; ENSG00000168394; ENSG00000168661; ENSG00000168938; ENSG00000169248; ENSG00000170113; ENSG00000170442; ENSG00000170509; ENSG00000170837; ENSG00000171016; ENSG00000171408; ENSG00000171649; ENSG00000171714; ENSG00000172137; ENSG00000172183; ENSG00000172215; ENSG00000172667; ENSG00000173809; ENSG00000173812; ENSG00000173926; ENSG00000175764; ENSG00000175806; ENSG00000176046; ENSG00000177182; ENSG00000177294; ENSG00000178187; and ENSG00000178229. In particular aspects, such a classifier contains additional genes, e.g., 1, 2, 3, 4, 5, 6, 7, 8, or more additional genes. In other aspects, the classifier omits certain of the above-mentioned genes, e.g., 1, 2, 3, 4, 5, 6, 7, 8, or more, of these genes, while in some cases including other genes.

In some embodiments, the present disclosure provides a method and/or system for differentiating UIP from non-UIP using a classifier that comprises or consists of 2; 3; 4; 5; 6; 7; 8; 9; 10; 11; 12; 13; 14; 15; 16; 17; 18; 19; 20; 21; 22; 23; 24; 25; 26; 27; 28; 29; 30; 31; 32; 33; 34; 35; 36; 37; 38; 39; 40; 41; 42; 43; 44; 45; 46; 47; 48; 49; 50; 51; 52; 53; 54; 55; 56; 57; 58; 59; 60; 61; 62; 63; 64; 65; 66; 67; 68; 69; 70; 71; 72; 73; 74; 75; 76; 77; 78; 79; 80; 81; 82; 83; 84; 85; 86; 87; 88; 89; 90; 91; 92; 93; 94; 95; 96; 97; 98; 99; 100; 101; 102; 103; 104; 105; 106; 107; 108; 109; 110; 111; 112; 113; 114; 115; 116; 117; 118; 119; 120; 121; 122; 123; 124; 125; 126; 127; 128; 129; 130; 131; 132; 133; 134; 135; 136; 137; 138; 139; 140; 141; 142; 143; 144; 145; 146; 147; 148; 149; 150; 151; 152; 153; 154; 155; 156; 157; 158; 159; 160; 161; 162; 163; 164; 165; 166; 167; 168; 169; 170; 171; 172; 173; 174; 175; 176; 177; 178; 179; 180; 181; 182; 183; 184; 185; 186; 187; 188; 189; or 190 of the following genes; alone or in combination: MPO; GGNBP2; SELE; FMO3; SLC6A13; EXTL3; NLRP2; BTN3A1; ABCF2; TLL1; HDAC9; CBLN4; CHRDL2; SNRNP40; MYO3B; SREBF1; GSDMB; MCCC1; CEACAM1; CXCL2; IL12RB2; ITGB5; ZNF671; ITPKC; RCOR1; TGM1; HSD17B7P2; DDTL; FAM118A; C14orf105; ADNP2; OLFM4; HAS3; SLC7A5; GYS1; FSD1; PLEKHA4; TMEM59L; RUNDC3B; LMBR1; VIPR2; CCL24; LARP4B; UBTF; RASD1; ODAM; CCND1; TSPAN11; SYT10; PRMT8; LTBR; CDK2AP1; GLP1R; VNN1; PCDHB2; LRRC31; SLC4A3; STAT1; IL18RAP; ERRFI1; CTH; ALDH6A1; ZNF410; CD274; EGR1; CHRNA2; BCL2L14; ZNF211; NBPF14; EDN3; KLHDC3; SCGB1D2; SLC10A2; ZFP36; GNAZ; TWSG1; C15orf57; LDLR; KLHDC7B; TNNI2; GFAP; CCL25; ENOSF1; LINC00470; PDE6A; MTUS2; NTS; ARNTL; ADAMDEC1; WNT2B; TRAFD1; PPP1R1A; EGR4; BAAT; KIF12; GABBR2; RABEPK; TUBB2B; MGARP; SULF1; POU2F3; SLC44A5; DUSP5; PLA2G12B; DUOXA2; DUOX2; DISP2; ARRDC4; CYP1A1; CYP1A2; FTO; NPEPPS; SIK1; MYOM3; XCL2; ILDR2; CRABP2; ABL2; TUFT1; SETDB1; KCNN3; CSRNP1; SLC10A4; SCD5; DDIT4L; GTF2H2; FAM13C; ADD3; HABP2; SYT8; ZC3H12C; SLC7A11; ANO4; CLEC4F; PLEKHG4B; CERS3; GBP5; TDRD9; SLC6A1; DGKI; TSPAN33; CBR3; SPON2; CCDC155; IL23R; SMC6; PDLIM5; GABRG1; EIF4E3; ATXN7; PPM1K; CXCL5; SLC6A20; KLF15; GPR85; DEFA4; IFI27L1; NELL1; PTER; GREM1; KLK1; HRASLS5; CTNNB1; BATF2; TAP1; ZNF30; PPIC; CXCL11; NIPA1; KRT86; HSD17B13; GPR27; PYGO1; PDE7B; ZIK1; ANO5; CALB2; ISG20; CXCR6; ZMAT3; TDRD12; EIF1; MARCH3; TTLL11; MSRA; NUPR1; CLVS1; FBXO39; ZNF454; andZNF543. In particular aspects, such a classifier contains additional genes, e.g., 1, 2, 3, 4, 5, 6, 7, 8, or more additional genes. In other aspects, the classifier omits certain of the above-mentioned genes, e.g., 1, 2, 3, 4, 5, 6, 7, 8, or more, of these genes, while in some cases including other genes.

In some embodiments, the present disclosure provides a method and/or system for differentiating UIP from non-UIP using a classifier described herein, wherein the method further comprises implementing a classifier that classifies the subject as a smoker or non-smoker. Such a smoker status classification can in some cases be implemented prior to implementing a UIP vs. non-UIP classifier, or a smoker status classification step may be built in as a covariate used during the training (e.g., using a classifier training module) of a UIP vs. non-UIP classifier of the present disclosure.

In particular embodiments, the present disclosure provides a method and/or system for differentiating UIP from non-UIP using the Envisia classifier, wherein the method further comprises implementing a classifier that classifies the subject as a smoker or non-smoker. Such a smoker status classification can in some cases be implemented prior to implementing the Envisia classifier, or a smoker status classification step may be built into the Envisia classifier as a covariate used during re training (e.g., using a classifier training module) of a UIP vs. non-UIP classifier comprising the genes listed in Table 5 according to the present disclosure.

In some embodiments, alternatively, or additionally, the method of and/or system for differentiating UIP from non-UIP using a classifier described herein (e.g., the Envisia classifier) further comprises a step of excluding or assigning differential weight to certain genes or variants thereof that are susceptible to smoker-status bias during the training (e.g., using a classifier training module) or implementation of the UIP vs. non-UIP classifier. As used herein, "smoker status bias" refers to genes or variants thereof, which in non-smoker patients are differentially expressed in UIP vs. non-UIP patients, but which are not detectably differentially expressed in UIP vs. non-UIP patients that are (or have been) smokers.

In some embodiments, the method of and/or system for the present disclosure comprises a tiered classifier comprising at least a first and a second classifier, wherein the first classifier is trained (e.g., using a classifier training module) to recognize gene signatures that distinguish smokers from non-smokers, and a second classifier is trained (e.g., using a classifier training module) to distinguish UIP vs. non-UIP in smokers or non-smokers, respectively. In some such embodiments, the second classifier is the Envisia classifier.

In some embodiments, alternatively, or additionally, the method of and/or system for differentiating UIP from non-UIP using a classifier described herein comprises a step of pooling a plurality of samples obtained from a subject, and then assaying the expression level of a group of transcripts present in the pooled sample. In some embodiments, the plurality of samples equals 2, 3, 4, or 5 samples. In some embodiments, the plurality of samples equals more than 5 samples. In some embodiments, the classifier comprises or consists of 1; 2; 3; 4; 5; 6; 7; 8; 9; 10; 11; 12; 13; 14; 15; 16; 17; 18; 19; 20; 21; 22; 23; 24; 25; 26; 27; 28; 29; 30; 31; 32; 33; 34; 35; 36; 37; 38; 39; 40; 41; 42; 43; 44; 45; 46; 47; 48; 49; 50; 51; 52; 53; 54; 55; 56; 57; 58; 59; 60; 61; 62; 63; 64; 65; 66; 67; 68; 69; 70; 71; 72; 73; 74; 75; 76; 77; 78; 79; 80; 81; 82; 83; 84; 85; 86; 87; 88; 89; 90; 91; 92; 93; 94; 95; 96; 97; 98; 99; 100; 101; 102; 103; 104; 105; 106; 107; 108; 109; 110; 111; 112; 113; 114; 115; 116; 117; 118; 119; 120; 121; 122; 123; 124; 125; 126; 127; 128; 129; 130; 131; 132; 133; 134; 135; 136; 137; 138; 139; 140; 141; 142; 143; 144; 145; 146; 147; 148; 149; 150; or 151 of SEQ ID NOs: 1-151, or any combination thereof. In particular aspects, such a classifier contains additional genes, e.g., 1, 2, 3, 4, 5, 6, 7, 8, or more additional genes. In other aspects, the classifier omits certain of the above-mentioned genes, e.g., 1, 2, 3, 4, 5, 6, 7, 8, or more, of these genes, while in some cases including other genes. In some embodiments, the classifier comprises or consists of 2; 3; 4; 5; 6; 7; 8; 9; 10; 11; 12; 13; 14; 15; 16; 17; 18; 19; 20; 21; 22; 23; 24; 25; 26; 27; 28; 29; 30; 31; 32; 33; 34; 35; 36; 37; 38; 39; 40; 41; 42; 43; 44; 45; 46; 47; 48; 49; 50; 51; 52; 53; 54; 55; 56; 57; 58; 59; 60; 61; 62; 63; 64; 65; 66; 67; 68; 69; 70; 71; 72; 73; 74; 75; 76; 77; 78; 79; 80; 81; 82; 83; 84; 85; 86; 87; 88; 89; 90; 91; 92; 93; 94; 95; 96; 97; 98; 99; 100; 101; 102; 103; 104; 105; 106; 107; 108; 109; 110; 111; 112; 113; 114; 115; 116; 117; 118; 119; 120; 121; 122; 123; 124; 125; 126; 127; 128; 129; 130; 131; 132; 133; 134; 135; 136; 137; 138; 139; 140; 141; 142; 143; 144; 145; 146; 147; 148; 149; 150; 151; 152; 153; 154; 155; 156; 157; 158; 159; 160; 161; 162; 163; 164; 165; 166; 167; 168; 169; 170; 171; 172; 173; 174; 175; 176; 177; 178; 179; 180; 181; 182; 183; 184; 185; 186; 187; 188; 189; or 190 of the genes listed in Table 5. In particular aspects, such a classifier contains additional genes, e.g., 1, 2, 3, 4, 5, 6, 7, 8, or more additional genes. In other aspects, the classifier omits certain of the above-mentioned genes, e.g., 1, 2, 3, 4, 5, 6, 7, 8, or more, of these genes, while in some cases including other genes.

In some embodiments, alternatively, or additionally, the method of and/or system for differentiating UIP from non-UIP using a classifier described herein comprises a step of *in silico* pooling of a plurality of samples obtained from a subject after assaying the expression level of a group of transcripts present in each of the plurality of samples. One example of such *in silico* pooling is described in Example 6. In some embodiments, a non-limiting example of *in silico* pooling comprises the steps of (i) assaying an expression level of a group of transcripts present in a first sample of a plurality of samples obtained from an individual subject; (ii) assaying an expression level of the same or an overlapping group of transcripts present in a second sample of the plurality of samples obtained from the individual subject; (iii) in some cases, assaying an expression level of the same or an overlapping group of transcripts (as compared to the first and second sample) in one or more additional samples in the plurality of samples obtained from the individual subject; (iv) scaling the expression levels; (v) averaging the scaled expression levels to produce an "*in silico-pooled*" expression level; (vi) performing variance stabilized transformation (VST) of the averaged scaled expression levels, (vii) score using VST of the in-silico pooled expressions; and (viii) compare the score to the decision boundary and assign UIP/non-UIP prediction label.

In some embodiments, the number of samples from the subject that are included in the plurality of samples pooled via *in silico* pooling equals 2, 3, 4, or 5 samples. In some embodiments, the number of samples in the plurality of samples equals more than 5 samples. In some embodiments, the classifier comprises or consists of 2; 3; 4; 5; 6; 7; 8; 9; 10; 11; 12; 13; 14; 15; 16; 17; 18; 19; 20; 21; 22; 23; 24; 25; 26; 27; 28; 29; 30; 31; 32; 33; 34; 35; 36; 37; 38; 39; 40; 41; 42; 43; 44; 45; 46; 47; 48; 49; 50; 51; 52; 53; 54; 55; 56; 57; 58; 59; 60; 61; 62; 63; 64; 65; 66; 67; 68; 69; 70; 71; 72; 73; 74; 75; 76; 77; 78; 79; 80; 81; 82; 83; 84; 85; 86; 87; 88; 89; 90; 91; 92; 93; 94; 95; 96; 97; 98; 99; 100; 101; 102; 103; 104; 105; 106; 107; 108; 109; 110; 111; 112; 113; 114; 115; 116; 117; 118; 119; 120; 121; 122; 123; 124; 125; 126; 127; 128; 129; 130; 131; 132; 133; 134; 135; 136; 137; 138; 139; 140; 141; 142; 143; 144; 145; 146; 147; 148; 149; 150; or 151 of SEQ ID NOs: 1-151, or any combination thereof. In particular aspects, such a classifier contains additional genes, e.g., 1, 2, 3, 4, 5, 6, 7, 8, or more additional genes. In other aspects, the classifier omits certain of the above-mentioned genes, e.g., 1, 2, 3, 4, 5, 6, 7, 8, or more, of these genes, while in some cases including other genes.

In some embodiments, the number of samples from the subject that are included in the plurality of samples pooled via *in silico* pooling equals 2, 3, 4, or 5 samples. In some embodiments, number of samples in the plurality of samples equals more than 5 samples. In some embodiments, the classifier comprises or consists of 2; 3; 4; 5; 6; 7; 8; 9; 10; 11; 12; 13; 14; 15; 16; 17; 18; 19; 20; 21; 22; 23; 24; 25; 26; 27; 28; 29; 30; 31; 32; 33; 34; 35; 36; 37; 38; 39; 40; 41; 42; 43; 44; 45; 46; 47; 48; 49; 50; 51; 52; 53; 54; 55; 56; 57; 58; 59; 60; 61; 62; 63; 64; 65; 66; 67; 68; 69; 70; 71; 72; 73; 74; 75; 76; 77; 78; 79; 80; 81; 82; 83; 84; 85; 86; 87; 88; 89; 90; 91; 92; 93; 94; 95; 96; 97; 98; 99; 100; 101; 102; 103; 104; 105; 106; 107; 108; 109; 110; 111; 112; 113; 114; 115; 116; 117; 118; 119; 120; 121; 122; 123; 124; 125; 126; 127; 128; 129; 130; 131; 132; 133; 134; 135; 136; 137; 138; 139; 140; 141; 142; 143; 144; 145; 146; 147; 148; 149; 150; 151; 152; 153; 154; 155; 156; 157; 158; 159; 160; 161; 162; 163; 164; 165; 166; 167; 168; 169; 170; 171; 172; 173; 174; 175; 176; 177; 178; 179; 180; 181; 182; 183; 184; 185; 186; 187; 188; 189; or 190 of the genes listed in Table 5. In particular aspects, such a classifier contains additional genes, e.g., 1, 2, 3, 4, 5, 6, 7, 8, or more additional genes. In other aspects, the classifier omits certain of the above-mentioned genes, e.g., 1, 2, 3, 4, 5, 6, 7, 8, or more, of these genes, while in some cases including other genes.

In some particular embodiments, a computer generated classifier for differentiating UIP from non-UIP is trained using expression levels of one or more transcripts expressed in a plurality of individual training samples obtained from a plurality of subjects, each training sample having a confirmed diagnoses (*i.e*., a "classification label" or "truth label" as disclosed herein (see, *e.g.,* FIG. 1)) of UIP or non-UIP, wherein at least two of the training samples were obtained from a single subject. In some embodiments, the present disclosure provides a method of detecting whether a pooled lung tissue test sample is positive for UIP or non-UIP using such a classifier (e.g., the Envisia classifier), wherein the method comprises (A) assaying the expression level of one or more transcripts expressed in a test sample; and (B) classifying the test sample as UIP or non-UIP using the computer-generated and trained classifier, wherein the test sample is pooled via physical pooling or via *in silico* pooling.

In some embodiments, by training on all samples separately, maximum representation and sampling diversity is achieved, and a priori sub-sampling bias of available samples is mitigated. Further, in some embodiments, by using pooled samples for the classification step, sampling effects are mitigated. Thus, in some embodiments, the use of a classifier trained on individual (non-pooled) samples with a test sample that has been pooled (either physically or via *in silico* pooling) provides improved accuracy for differentiating UIP from non-UIP.

Thus, in one embodiment, the present disclosure provides a method of detecting whether a pooled lung tissue test sample from a subject is positive for UIP or non-UIP using the Envisia classifier, the method comprising (A) assaying the expression level of one or more transcripts expressed in a test sample from the subject; and (B) classifying the test sample as UIP or non-UIP using the computer generated Envisia classifier, wherein the test sample comprises a plurality of samples from the subject that have been pooled via physical pooling or via *in silico* pooling. In some embodiments, the plurality of samples comprises 2, 3, 4, or 5 samples. In some embodiments, the plurality of samples comprises more than 5 samples.

In some embodiments, the method of and/or system for differentiating UIP from non-UIP using a classifier described herein (e.g., the Envisia classifier) comprises differentiating UIP from non-UIP in samples (e.g., a single sample or a pool of samples) that have variable cellular composition. In some embodiments, the samples (e.g., a single sample or a pool of samples) with variable cellular composition comprise type 1 alveolar cells; type 2 alveolar cells, bronchiolar cells, lung progenitor cells, or a combination thereof. In some embodiments, the accuracy of the classifier for differentiating UIP from non-UIP is not dependent on alveolar content of the sample or pooled samples that are classified. As used herein, the term "agnostic to cellular composition" is used in reference to such a classifier, for which the accuracy of the classifier for differentiating UIP from non-UIP is not dependent on alveolar content of the sample (e.g., a single sample or a pool of samples) being classified.

In some embodiments, the present disclosure presents a classifier that is agnostic to cellular composition, the classifier exhibiting a Pearson's correlation between the classifier accuracy and the alveolar content of a sample or pooled samples that is less than about 0.1; 0.09; 0.08; 0.07; 0.06; 0.05; 0.04; 0.03; 0.02; or less than about 0.01. In some embodiments, the present disclosure presents a classifier that is agnostic to cellular composition, the classifier exhibiting a Pearson's correlation between the classifier accuracy and the alveolar content of a sample or pooled samples that is greater than about -0.1; -0.09; -0.08; -0.07; -0.06; -0.05; -0.04; - 0.03; -0.02; or greater than about -0.01. In some embodiments, the classifier that is agnostic to cellular composition is the Envisia classifier.

Variable cellular composition in a sample may be detected via any suitable method. In some embodiments, variable cellular composition is determined using a semi-quantitative genomic measure of cellular content. In some embodiments, the semi-quantitative genomic measure of cellular content determines the relative abundance of alveolar cells in a sample.

In some embodiments, such a semi-quantitative genomic measure of alveolar content comprises a metric capable of determining the relative abundance of alveolar type 1 cells in a sample ("alveolar type 1 cell metric"). In some embodiments, the alveolar type 1 cell metric comprises one or more alveolar-specific gene. In some embodiments, the one or more alveolar-specific gene is a gene expressed primarily in alveolar type 1 cells. In certain embodiments, the one or more alveolar-specific gene expressed primarily in alveolar type 1 cells is selected from AQP5, PDPN, or a combination thereof. In particular embodiments, expression of AQP5, PDPN, or a combination thereof correlates with the abundance of alveolar type 1 cells in the sample. In particular embodiments, the method comprises detecting expression levels for AQP5 and PDPN, in some cases normalizing the expression levels, and summing the expression levels for these genes, wherein a high expression level indicates high alveolar type 1 cell content in the sample; a low expression level indicates low alveolar type 1 cell content in the sample; and a moderate expression level indicates moderate alveolar type 1 cell content in the sample.

In particular embodiments, the present disclosure provides a method of determining the relative abundance of type 1 alveolar cells present in two or more samples comprising (i) assaying an expression level in a first sample obtained from an individual subject of one or more transcripts of an alveolar-specific gene expressed primarily in alveolar type 1 cells; (ii) assaying an expression level in a second sample obtained from an individual subject of the same one or more transcript of an alveolar-specific gene expressed primarily in alveolar type 1 cells; (iii) and comparing the expression levels of the one or more transcripts between the two samples to determine the relative abundance of type 1 alveolar cells present in the samples. In some embodiments, the one or more alveolar-specific genes expressed primarily in alveolar type 1 cells is selected from AQP5, PDPN, or a combination thereof. In some embodiments, the one or more alveolar-specific genes expressed primarily in alveolar type 1 cells comprises AQP5, PDPN, or a combination thereof. In some embodiments, the one or more alveolar-specific genes expressed primarily in alveolar type 1 cells comprises both AQP5 and PDPN. In some embodiments, the first sample and the second sample are obtained from different subjects. In some embodiments, the first sample and the second sample are obtained from the same subject. In some embodiments, the method further comprises assaying an expression level in at least one additional sample obtained from an individual subject of the same one or more transcript of an alveolar-specific gene expressed primarily in alveolar type 1 cells and then comparing the expression level in the at least one additional sample to the expression level in the first and/or the second sample to determine the relative abundance of type 1 alveolar cells present in the samples. In some embodiments, at least two of the samples are obtained from the same subject. In some embodiments, at least 3, 4, 5, or more of the samples are obtained from the same subject. In some embodiments, all of the samples are obtained from different subjects.

In some embodiments, the present disclosure provides a semi-quantitative genomic measure of alveolar content comprising a metric capable of determining the relative abundance of alveolar type 2 cells in a sample ("alveolar type 2 cell metric"). In some embodiments, the metric comprises one or more alveolar-specific genes. In some embodiments, the one or more alveolar-specific genes are genes expressed primarily in alveolar type 2 cells. In certain embodiments, the one or more alveolar-specific genes expressed primarily in alveolar type 2 cells are selected from SFTPB, SFTPC, SFTPD, or a combination thereof. In certain embodiments, the one or more alveolar-specific genes expressed primarily in alveolar type 2 cells comprise SFTPB, SFTPC, SFTPD, or a combination thereof. In certain embodiments, the one or more alveolar-specific genes expressed primarily in alveolar type 2 cells comprises SFTPB, SFTPC, and SFTPD. In some embodiments, the alveolar type 2 cell metric further comprises one or more alveolar-specific genes that are expressed in both alveolar type 1 and alveolar type 2 cells. In certain embodiments, the gene expressed in both alveolar type 1 and alveolar type 2 cells is SFTPA1. In particular embodiments, the metric includes one or more alveolar-specific genes expressed primarily in alveolar type 2 cells and one or more genes expressed in both alveolar type 1 and alveolar type 2 cells. In particular embodiments, the metric comprises SFTPB, SFTPC, SFTPD, SFTPA1, or a combination thereof.

In particular embodiments, the present disclosure provides a method of determining the relative abundance of alveolar type 2 cells in a sample comprising detecting expression levels for FTPB, SFTPC, SFTPD, SFTPA1, or a combination thereof, in some cases normalizing the expression levels, and summing the expression levels for these genes, wherein a high expression level indicates high alveolar type 2 cell content in the sample; a low expression level indicates low alveolar type 2 cell content in the sample; and a moderate expression level indicates moderate alveolar type 2 cell content in the sample.

In particular embodiments, the present disclosure provides a method of determining the relative abundance of type 2 alveolar cells present in two or more samples; the method comprising (i) assaying an expression level in a first sample obtained from an individual subject of one or more transcripts of an alveolar-specific gene expressed primarily in alveolar type 2 cells; (ii) assaying an expression level in a second sample obtained from an individual subject of the same one or more transcripts of an alveolar-specific gene expressed primarily in alveolar type 2 cells; (iii) and comparing the expression levels of the one or more transcripts between the two samples to determine the relative abundance of type 2 alveolar cells present in the samples. In some such embodiments, the one or more alveolar-specific genes are expressed primarily in alveolar type 2 cells is selected from SFTPB, SFTPC, and SFTPD, and a combination thereof. In particular embodiments, the method comprises assaying the expression of each of SFTPB, SFTPC, and SFTPD in the first and second samples. Alternatively, or additionally, in various embodiments, the method further comprises assaying an expression level of one or more additional genes in the first sample and second samples. In some such embodiments, the one or more additional genes comprise genes expressed primarily in alveolar cells. In some embodiments, the additional genes are expressed in both alveolar type 1 and alveolar type 2 cells. In particular embodiments, the additional gene is SFTPA1. In some embodiments, the first sample and the second sample are obtained from different subjects. In some embodiments, the first sample and the second sample are obtained from the same subject. In some embodiments, the method further comprises assaying an expression level in at least one additional sample obtained from an individual subject of the same one or more transcripts of an alveolar-specific gene expressed primarily in alveolar type 1 cells and/or in both alveolar type 1 cells and alveolar type 2 cells and then comparing the expression level in the at least one additional sample to the expression level in the first and/or the second sample to determine the relative abundance of type 2 alveolar cells present in the samples. In some embodiments, at least two of the samples are obtained from the same subject. In some embodiments, at least 3, 4, 5, or more of the samples are obtained from the same subject. In some embodiments, all of the samples are obtained from different subjects.

Methods disclosed herein may involve comparing expression levels of informative-genes with one or more appropriate references. An "appropriate reference" is an expression level (or range of expression levels) of a particular informative-gene that is indicative of a known lung ILD status (*i.e*., UIP vs. non-UIP; IPF vs. non-IPF). An appropriate reference can be determined experimentally by a practitioner of the methods or can be a pre-existing value or range of values. An appropriate reference represents an expression level (or range of expression levels) indicative of UIP/non-UIP status. For example, an appropriate reference may be representative of the expression level of an informative gene in a reference (control) biological sample that is known to express UIP. When an appropriate reference is indicative of UIP, a lack of a detectable difference (e.g., lack of a statistically significant difference) between an expression level determined from a subject in need of characterization or diagnosis of UIP and the appropriate reference may be indicative of UIP in the subject. When an appropriate reference is indicative of UIP, a difference between an expression level determined from a subject in need of characterization or diagnosis of UIP and the appropriate reference may be indicative of the subject being free of UIP (*i.e.,* non-UIP).

Alternatively, an appropriate reference may be an expression level (or range of expression levels) of a gene that is indicative of a subject being free of UIP (*i.e.,* non-UIP). For example, an appropriate reference may be representative of the expression level of a particular informative gene in a reference (control) biological sample obtained from a subject who is known to be free of UIP. When an appropriate reference is indicative of a subject being free of UIP, a difference between an expression level determined from a subject in need of diagnosis of UIP and the appropriate reference may be indicative of UIP in the subject. Alternatively, when an appropriate reference is indicative of the subject being free of UIP, a lack of a detectable difference (e.g., lack of a statistically significant difference) between an expression level determined from a subject in need of diagnosis of UIP and the appropriate reference level may be indicative of the subject being free of UIP.

In some embodiments, the reference standard provides a threshold level of change, such that if the expression level of a gene in a sample is within a threshold level of change (increase or decrease depending on the particular marker) then the subject is identified as free of UIP, but if the levels are above the threshold then the subject is identified as being at risk of having UIP.

In some embodiments, the methods involve comparing the expression level of an informative gene to a reference standard that represents the expression level of the informative-gene in a control subject who is identified as not having UIP. This reference standard may be, for example, the average expression level of the informative gene in a population of control subjects who are identified as not having UIP.

The magnitude of difference between the expression level and an appropriate reference that is statistically significant may vary. For example, a significant difference that indicates UIP may be detected when the expression level of an informative gene in a biological sample is at least 1%, at least 5%, at least 10%, at least 25%, at least 50%, at least 100%, at least 250%, at least 500%, or at least 1000% higher, or lower, than an appropriate reference of that gene. Similarly, a significant difference may be detected when the expression level of an informative gene in a biological sample is at least 1.1-fold, 1.2-fold,1.5-fold, 2-fold, at least 3-fold, at least 4-fold, at least 5-fold, at least 6-fold, at least 7-fold, at least 8-fold, at least 9-fold, at least 10-fold, at least 20-fold, at least 30-fold, at least 40-fold, at least 50-fold, at least 100-fold, or more higher, or lower, than the appropriate reference of that gene. In some embodiments, at least a 20% to 50% difference in expression between an informative gene and an appropriate reference is significant. Significant differences may be identified by using an appropriate statistical test. Examples of tests for statistical significance are provided in Applied Statistics for Engineers and Scientists by Petruccelli, Chen and Nandram 1999 Reprint Ed, which is entirely incorporated herein by reference.

It is to be understood that a plurality of expression levels may be compared with a plurality of appropriate reference levels, e.g., on a gene-by-gene basis, in order to assess the UIP status of the subject. The comparison may be made as a vector difference. In such cases, multivariate tests, e.g., Hotelling's T2 test, may be used to evaluate the significance of observed differences. Examples of such multivariate tests are provided in Applied Multivariate Statistical Analysis by Richard Arnold Johnson and Dean W. Wichern Prentice Hall; 6th edition (April 2, 2007), which is entirely incorporated herein by reference.

### Classification Methods

The methods may also involve comparing a set of expression levels (referred to as an expression pattern or profile) of informative genes in a biological sample obtained from a subject with a plurality of sets of reference levels (referred to as reference patterns), each reference pattern being associated with a known UIP status, identifying the reference pattern that most closely resembles the expression pattern, and associating the known UIP status of the reference pattern with the expression pattern, thereby classifying (characterizing) the UIP status of the subj ect.

The methods may also involve building or constructing a prediction model, which may also be referred to as a classifier or predictor that can be used to classify the disease status of a subject. As used herein, a "UIP -classifier" is a prediction model that characterizes the UIP status of a subject based on expression levels determined in a biological sample obtained from the subject. Typically the model is built using samples for which the classification (UIP status) has already been ascertained. Once the model (classifier) is built, it may then be applied to expression levels obtained from a biological sample of a subject whose UIP status is unknown in order to predict the UIP status of the subject. In particular embodiments, the UIP-classifier is the Envisia classifier. Thus, the methods may involve applying a UIP-classifier (e.g., the Envisia classifier) to the expression levels, such that the UIP-classifier characterizes the UIP status of a subject based on the expression levels. The subject may be further treated or evaluated, e.g., by a health care provider, based on the predicted UIP status. In some embodiments, the subject may be treated with a compound selected from pirfenidone, nintedanib, or pharmaceutically acceptable salts thereof, based on the predicted UIP status (e.g., based on a classification of UIP determined by applying the classifier to gene expression data from a test sample obtained from the subject. The test sample may comprise a plurality (such as at least 1, 2, 3, 4, 5, or more samples) of physical or *in silico* pooled samples from the subject.

The classification methods may involve transforming the expression levels into a UIP risk-score that is indicative of the likelihood that the subject has UIP. In some embodiments, such as, for example, when an elastic net regression model such as GLMNET is used, the UIP risk-score may be obtained as the combination (e.g., sum, product, or other combination) of weighted expression levels, in which the expression levels are weighted by their relative contribution to predicting increased likelihood of having UIP.

A variety of prediction models may be used as a UIP-classifier. For example, a UIP-classifier may comprise an algorithm selected from logistic regression, partial least squares, linear discriminant analysis, quadratic discriminant analysis, neural network, naïve Bayes, C4.5 decision tree, k-nearest neighbor, random forest, support vector machine, or other appropriate method.

The UIP-classifier may be trained on a data set comprising expression levels of the plurality of informative genes in biological samples obtained from a plurality of subjects identified as having UIP. For example, the UIP-classifier may be trained on a data set comprising expression levels of a plurality of informative genes in biological samples obtained from a plurality of subjects identified as having UIP based histological findings. The training set will typically also comprise control subjects identified as not having UIP. As will be appreciated by the skilled artisan, the population of subjects of the training data set may have a variety of characteristics by design, e.g., the characteristics of the population may depend on the characteristics of the subjects for whom diagnostic methods that use the classifier may be useful. For example, the population may consist of all males, all females or may consist of both males and females. The population may consist of subjects with a history of cancer, subjects without a history of cancer, or subjects from both categories. The population may include subjects who are smokers, former smokers, and/or non-smokers.

A class prediction strength can also be measured to determine the degree of confidence with which the model classifies a biological sample. This degree of confidence may serve as an estimate of the likelihood that the subject is of a particular class predicted by the model.

Accordingly, the prediction strength conveys the degree of confidence of the classification of the sample and evaluates when a sample cannot be classified. There may be instances in which a sample is tested, but does not belong, or cannot be reliably assigned to, a particular class. This may be accomplished, for example, by utilizing a threshold, or range, wherein a sample which scores above or below the determined threshold, or within the particular range, is not a sample that can be classified (e.g., a "no call").

Once a model is built, the validity of the model can be tested using various methods. One way to test the validity of the model is by cross-validation of the dataset. To perform cross-validation, one, or a subset, of the samples is eliminated and the model is built, as described above, without the eliminated sample, forming a "cross-validation model." The eliminated sample is then classified according to the model, as described herein. This process is done with all the samples, or subsets, of the initial dataset and an error rate is determined. The accuracy of the model is then assessed. This model classifies samples to be tested with high accuracy for classes that are known, or classes have been previously ascertained. Another way to validate the model is to apply the model to an independent data set, such as a new biological sample having an unknown UIP status.

As will be appreciated by the skilled artisan, the strength of the model may be assessed by a variety of parameters including, but not limited to, the accuracy, sensitivity and specificity. Various methods for computing accuracy, sensitivity and specificity are described herein (See, e.g., the Examples). The UIP-classifier may have an accuracy of at least 60%, at least 65%, at least 70%, at least 75%, at least 80%, at least 85%, at least 90%, at least 95%, at least 99%, or more. The UIP classifier may have an accuracy in a range of about 60% to 70%, 70% to 80%, 80% to 90%, or 90% to 100%. The UIP-classifier may have a sensitivity of at least 60%, at least 65%, at least 70%, at least 75%, at least 80%, at least 85%, at least 90%, at least 95%, at least 99%, or more. The UIP-classifier may have a sensitivity in a range of about 60% to 70%, 70% to 80%, 80% to 90%, or 90% to 100%. The UIP-classifier may have a specificity of at least 60%, at least 65%, at least 70%, at least 75%, at least 80%, at least 85%, at least 90%, at least 95%, at least 99%, or more. The UIP -classifier may have a specificity in a range of about 60% to 70%, 70% to 80%, 80% to 90%, or 90% to 100%.

The Negative Predictive Value (NPV) may be greater than or equal to 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% for ruling out UIP in an intended use population (e.g., a subject, such as a patient). When UIP is ruled out, non-UIP may be ruled in.

The UIP classifier may have a positive predictive value (PPV) of greater than or equal to 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% for ruling in UIP. When UIP is ruled in, non-UIP may be ruled out.

The intended use population may have a prevalence of cancer at or about 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%.

In some embodiments, the method and/or systems of the present disclosure comprises: extracting nucleic acids (e.g., RNA, such as, e.g., total RNA) from a test sample (e.g. lung tissue); amplifying the nucleic acid to produce an expressed nucleic acid library (e.g., via polymerase chain reaction-mediated amplification of cDNAs (in some cases labeled cDNAs), which cDNAs may be produced from one or more RNA samples by reverse transcription (RT-PCR)); detecting expression of one or more nucleic acids present in the nucleic acid library (e.g., detecting RNA expression profiles by measuring cDNA species produced via RT-PCR) via an array (e.g., a microarray) or via direct sequencing (e.g., RNAseq); and determining whether the test sample is UIP or non-UIP using a trained classifier described herein (e.g., the Envisia classifier).

In some embodiments, the method and/or system of the present disclosure further comprises incorporating smoker status into the training exercise. In certain embodiments, smoker status is in some cases incorporated in one of the following ways:
(i) by using smoking status as a covariate in a UIP or non-UIP classifier during training (e.g., using a classifier training module).
(ii) by identifying a plurality of genes that are susceptible to smoker-status bias and excluding, or in some cases weighing such genes differently than genes that are not susceptible to such bias, during UIP or Non-UIP classifier training (e.g., using a classifier training module).
(iii) by constructing a tiered classification in which an initial classifier that is trained (e.g., using a classifier training module) to recognize gene signatures that distinguish smokers from non-smokers is used to pre-classify a test sample as "smoker" or "non-smoker" based upon the gene signature of the test sample; and then, subsequent to pre-classification, a distinct classifier that was trained (e.g., using a classifier training module) to distinguish UIP vs. non-UIP in either smokers or non-smokers is implemented. For example, if the pre-classifier determines that the test sample is from a smoker, a UIP vs. non-UIP classification is performed using a classifier trained (e.g., using a classifier training module) with UIP and non-UIP samples from smokers. Conversely, if the pre-classifier determines that the test sample is from a non-smoker, a UIP vs. non-UIP classification is performed using a classifier trained (e.g., using a classifier training module) with UIP and non-UIP samples from non-smokers. In some embodiments, such smoker- or non-smoker-specific classifiers provide improved diagnostic performance due, at least in part, to a reduction in background noise caused by the inclusion of genes susceptible to smoker-status bias in the classifier training.

Accordingly, the present disclosure also provides suitable classifiers for use in methods of differentiating UIP from non-UIP, as disclosed herein (e.g., the Envisia classifier). In various embodiments, the present disclosure provides a classifier suitable for differentiating UIP from non-UIP, wherein the classifier is trained (e.g., using a classifier training module such as, e.g., the Envisia classifer) using microarray, qRT-PCR, or sequencing data from a sample (e.g., an individual sample or a pooled sample) corresponding to one or more histopathology labels determined by an expert pathologist. In some embodiments, the sample is labelled UIP or non-UIP.

In some embodiments, the present disclosure presents a classifier comprising or consisting of one or more sequences or fragments thereof presented in Table 1 and/or Table 15, or at least one sequence or fragment thereof from Table 1 and/or Table 15. In some embodiments, the present disclosure provides a classifier comprising or consisting of at least 1, 2, 3, 4, 5, 6, 7, 8, 9, 10 or more of the sequences provided in any one or more or all of Table 1 and/or Table 15. For example, in some embodiments, the present disclosure provides a classifier comprising or consisting of at least 11, 12, 13, 14, 15, 20, 30, 50, 100, 150, 151 sequences provided in Table 1, including all integers (e.g., 16, 17, 18, 19, 21, 22, 23, 24, 25 sequences, etc.) and ranges (e.g., from about 1-10 sequences from any one or more or all of Tables 5, 7, 8, 9, 10, 11, or 12, from about 10-15 sequences, 10-20 sequences, 5-30 sequences, 5-50 sequences, 10-100 sequences, 50-151 sequences from any one or more or all of Table 1 and/or Table 15) between. In one embodiment, the present disclosure provides a classifier that comprises or consists of all sequences provided in Table 1 and/or Table 15.

In some embodiments, the present disclosure presents a classifier comprising or consisting of one or more sequences or fragments thereof presented in Table 5, or at least one sequence or fragment thereof from Table 5. In some embodiments, the present disclosure provides a classifier comprising or consisting of at least 1, 2, 3, 4, 5, 6, 7, 8, 9, 10 or more of the sequences provided in Table 5. For example, in some embodiments, the present disclosure provides a classifier comprising or consisting of at least 11, 12, 13, 14, 15, 20, 30, 50, 100, 150, 160, 170, 180, or 190 sequences provided in Table 5, including all integers (e.g., 16, 17, 18, 19, 21, 22, 23, 24, 25 sequences, etc.) and ranges (e.g., from about 1-10 sequences from any one or more or all of Tables 5, 7, 8, 9, 10, 11, or 12, from about 10-15 sequences, 10-20 sequences, 5-30 sequences, 5-50 sequences, 10-100 sequences, 50-150 sequences, 60-190 sequences from Table 5) between. In one embodiment, the present disclosure provides a classifier that comprises or consists of all sequences provided in Table 5.

In some particular embodiments, the present disclosure provides a classifier for differentiating UIP from non-UIP, wherein the classifier comprises or consists of one or more of SEQ ID NOs: 1-151, or fragments thereof, or any combination thereof. In one embodiment, the classifier comprises or consists of all 151 of the above mentioned sequences. In some embodiments, the present disclosure provides a classifier for differentiating UIP from non-UIP, wherein the classifier comprises or consists of 2; 3; 4; 5; 6; 7; 8; 9; 10; 11; 12; 13; 14; 15; 16; 17; 18; 19; 20; 21; 22; 23; 24; 25; 26; 27; 28; 29; 30; 31; 32; 33; 34; 35; 36; 37; 38; 39; 40; 41; 42; 43; 44; 45; 46; 47; 48; 49; 50; 51; 52; 53; 54; 55; 56; 57; 58; 59; 60; 61; 62; 63; 64; 65; 66; 67; 68; 69; 70; 71; 72; 73; 74; 75; 76; 77; 78; 79; 80; 81; 82; 83; 84; 85; 86; 87; 88; 89; 90; 91; 92; 93; 94; 95; 96; 97; 98; 99; 100; 101; 102; 103; 104; 105; 106; 107; 108; 109; 110; 111; 112; 113; 114; 115; 116; 117; 118; 119; 120; 121; 122; 123; 124; 125; 126; 127; 128; 129; 130; 131; 132; 133; 134; 135; 136; 137; 138; 139; 140; 141; 142; 143; 144; 145; 146; 147; 148; 149; 150; or 151 of the abovementioned 151 sequences. In particular aspects, the classifier contains 1, 2, 3, 4, 5, 6, 7, 8, or more additional genes or fragments thereof. In other aspects, the classifier omits 1, 2, 3, 4, 5, 6, 7, 8, or more, of the abovementioned 151 sequences, while in some cases including other genes. In other aspects, each of the 151 genes may be used in combination with any one or more, or up to 20 more, of the other genes.

In some particular embodiments, the present disclosure provides a classifier for differentiating UIP from non-UIP, wherein the classifier comprises or consists of one or more of the genes listed in Table 5, or fragments thereof, or any combination thereof. In one embodiment, the classifier comprises or consists of all 190 of the genes listed in Table 5. In some embodiments, the present disclosure provides a classifier for differentiating UIP from non-UIP, wherein the classifier comprises or consists of 2; 3; 4; 5; 6; 7; 8; 9; 10; 11; 12; 13; 14; 15; 16; 17; 18; 19; 20; 21; 22; 23; 24; 25; 26; 27; 28; 29; 30; 31; 32; 33; 34; 35; 36; 37; 38; 39; 40; 41; 42; 43; 44; 45; 46; 47; 48; 49; 50; 51; 52; 53; 54; 55; 56; 57; 58; 59; 60; 61; 62; 63; 64; 65; 66; 67; 68; 69; 70; 71; 72; 73; 74; 75; 76; 77; 78; 79; 80; 81; 82; 83; 84; 85; 86; 87; 88; 89; 90; 91; 92; 93; 94; 95; 96; 97; 98; 99; 100; 101; 102; 103; 104; 105; 106; 107; 108; 109; 110; 111; 112; 113; 114; 115; 116; 117; 118; 119; 120; 121; 122; 123; 124; 125; 126; 127; 128; 129; 130; 131; 132; 133; 134; 135; 136; 137; 138; 139; 140; 141; 142; 143; 144; 145; 146; 147; 148; 149; 150; 151; 152; 153; 154; 155; 156; 157; 158; 159; 160; 161; 162; 163; 164; 165; 166; 167; 168; 169; 170; 171; 172; 173; 174; 175; 176; 177; 178; 179; 180; 181; 182; 183; 184; 185; 186; 187; 188; 189; or 190 of the abovementioned 190 genes listed in Table 5. In particular aspects, the classifier contains the 190 genes listed in Table 5 and 1, 2, 3, 4, 5, 6, 7, 8, or more additional genes or fragments thereof. In other aspects, the classifier omits 1, 2, 3, 4, 5, 6, 7, 8, or more, of the abovementioned 190 genes listed in Table 5, while in some cases including other genes. In other aspects, each of the 190 genes may be used in combination with any one or more, or up to 20 more, of the other genes to classify as sample as UIP or non-UIP according to the methods disclosed herein.

In certain embodiments, the present disclosure provides a method of improving the detection of a disease or condition in a lung tissue sample, the method comprising (A) assaying the expression level of one or more transcripts expressed in a test sample; and (B) classifying the test sample as either positive for, or negative for, the disease or condition using a computer generated trained classifier (e.g., the Envisia classifier); wherein the computer generated trained classifier is trained using expression levels of one or more transcripts expressed in a plurality of individual training samples obtained from a plurality of subjects, each training sample having a confirmed diagnoses of positive or negative for the disease or condition, wherein at least two of the training samples were obtained from a single subject; and wherein the test sample is pooled prior to the classifying.

### Tissue samples

A lung tissue sample for use in a subject analytical or diagnostic method may be a biopsy sample (e.g., a biopsy sample obtained by video-assisted thoracoscopic surgery; VATS); a bronchoalveolar lavage (BAL) sample; a transbronchial biopsy; a cryo-transbronchial biopsy; and the like. Lung tissue samples for analysis may be provided in a suitable preservation solution. In some embodiments, a tissue sample is obtained by an ancillary bronchoscopic procedure such as brushing (such as by cytobrush, histobrush); bronchial biopsy; bronchial lavage; or needle-aspiration. In some embodiments, the tissue sample may be obtained by oral washings, touch preps, or sputum collection. In some embodiments, the tissue sample is obtained from a respiratory epithelium of the subject. The respiratory epithelium may be from the mouth, nose, pharynx, trachea, bronchi, bronchioles, or alveoli. However, other sources of respiratory epithelium also may be used.

Tissue samples may be obtained from a patient suspected of having a lung disease, e.g., an ILD, based on clinical signs and symptoms with which the patient presents (e.g., shortness of breath (generally aggravated by exertion), dry cough), and, in some cases the results of one or more imaging tests (e.g., chest X-ray, computerized tomography (CT)), a pulmonary function test (e.g., spirometry, oximetry, exercise stress test), and/or lung tissue analysis (e.g., histological and/or cytological analysis of samples obtained by bronchoscopy, bronchoalveolar lavage, surgical biopsy). In some cases the cytological or histological analysis of the tissue sample may be ambiguous or suspicious (or indeterminate) for a presence or absence of lung disease.

The lung tissue sample may be processed in any of a variety of ways. For example, the lung tissue sample may be subjected to cell lysis. The lung tissue sample may be preserved in RNAprotect solution (a solution that inhibits RNA degradation, e.g., that inhibits nuclease digestion of RNA) and subsequently subjected to cell lysis. Components such as nucleic acids and/or proteins may be enriched or isolated from the lung tissue sample, and the enriched or isolated component may be used in a subject method. Various methods of enriching for and isolating components such nucleic acids and may be used. Various methods of isolating RNA for expression analysis may be used.

### In vitro methods of determining expression product levels

Methods for determining gene expression product levels may include but are not limited to one or more of the following: additional cytological assays, assays for specific proteins or enzyme activities, assays for specific expression products including protein or RNA or specific RNA splice variants, in situ hybridization, whole or partial genome expression analysis, microarray hybridization assays, serial analysis of gene expression (SAGE), enzyme linked immunoabsorbance assays, mass-spectrometry, immunohistochemistry, blotting, sequencing, RNA sequencing (e.g., exome enriched RNA sequencing), DNA sequencing (e.g., sequencing of cDNA obtained from RNA); next-generation sequencing, nanopore sequencing, pyrosequencing, or Nanostring sequencing. For example, gene expression product levels may be determined according to the methods described in Kim, et.al. (Lancet Respir Med. 2015 Jun;3(6):473-82, incorporated herein in its entirety, including all supplements). As used herein, the terms "assaying" or "detecting" or "determining" are used interchangeably in reference to determining gene expression product levels. In embodiments, the above-mentioned methods of determining gene expression product levels are suitable for detecting or assaying gene expression product levels. Gene expression product levels may be normalized to an internal standard such as total mRNA or the expression level of a particular gene including but not limited to glyceraldehyde-3-phosphate dehydrogenase, or tubulin.

In various embodiments, a sample comprises cells harvested from a tissue sample (e.g., a lung tissue sample such as a TBB sample). Cells may be harvested from a sample using various techniques. For example, cells may be harvested by centrifuging a cell sample and resuspending the pelleted cells. The cells may be resuspended in a buffered solution such as phosphate-buffered saline (PBS). After centrifuging the cell suspension to obtain a cell pellet, the cells may be lysed to extract nucleic acid, *e.g*., messenger RNA (mRNA). All samples obtained from a subject, including those subjected to any sort of further processing, are considered to be obtained from the subject.

The sample, in one embodiment, is further processed before detection of the gene expression products is performed as described herein. For example, mRNA in a cell or tissue sample may be separated from other components of the sample. The sample may be concentrated and/or purified to isolate mRNA in its non-natural state, as the mRNA is not in its natural environment. For example, studies have indicated that the higher order structure of mRNA *in vivo* differs from the *in vitro* structure of the same sequence (*see, e.g.,* Rouskin et al. (2014). Nature 505, pp. 701-705, incorporated herein in its entirety for all purposes).

mRNA from the sample in one embodiment, is hybridized to a synthetic DNA probe, which in some embodiments, includes a detection moiety (e.g., detectable label, capture sequence, barcode reporting sequence). Accordingly, in these embodiments, a non-natural mRNA-cDNA complex is ultimately made and used for detection of the gene expression product. In another embodiment, mRNA from the sample is directly labeled with a detectable label, *e.g.*, a fluorophore. In a further embodiment, the non-natural labeled-mRNA molecule is hybridized to a cDNA probe and the complex is detected.

In one embodiment, once the mRNA is obtained from a sample, it is converted to complementary DNA (cDNA) in a hybridization reaction or is used in a hybridization reaction together with one or more cDNA probes. cDNA does not exist *in vivo* and therefore is a non-natural molecule. Furthermore, cDNA-mRNA hybrids are synthetic and do not exist *in vivo.* Besides cDNA not existing *in vivo,* cDNA is necessarily different than mRNA, as it includes deoxyribonucleic acid and not ribonucleic acid. The cDNA is then amplified, for example, by the polymerase chain reaction (PCR) or other amplification. For example, other amplification methods that may be employed include the ligase chain reaction (LCR) (Wu and Wallace, Genomics, 4:560 (1989), Landegren et al., Science, 241:1077 (1988), incorporated by reference in its entirety for all purposes, transcription amplification (Kwoh et al., Proc. Natl. Acad. Sci. USA, 86:1173 (1989), incorporated by reference in its entirety for all purposes), self-sustained sequence replication (Guatelli et al., Proc. Nat. Acad. Sci. USA, 87:1874 (1990), incorporated by reference in its entirety for all purposes), incorporated by reference in its entirety for all purposes, and nucleic acid based sequence amplification (NASBA). Examples of guidelines for selecting primers for PCR amplification are provided in McPherson et al., PCR Basics: From Background to Bench, Springer-Verlag, 2000, incorporated by reference in its entirety for all purposes. The product of this amplification reaction, *i.e*., amplified cDNA is also necessarily a non-natural product. First, as mentioned above, cDNA is a non-natural molecule. Second, in the case of PCR, the amplification process serves to create hundreds of millions of cDNA copies for every individual cDNA molecule of starting material. The number of copies generated are far removed from the number of copies of mRNA that are present *in vivo.*

In one embodiment, cDNA is amplified with primers that introduce an additional DNA sequence (*e.g*., adapter, reporter, capture sequence or moiety, barcode) onto the fragments (*e.g*., with the use of adapter-specific primers), or mRNA or cDNA gene expression product sequences are hybridized directly to a cDNA probe comprising the additional sequence (e.g., adapter, reporter, capture sequence or moiety, barcode). Amplification and/or hybridization of mRNA to a cDNA probe therefore serves to create non-natural double-stranded molecules from the non-natural single-stranded cDNA, or the mRNA, by introducing additional sequences and forming non-natural hybrids. Further, amplification procedures have error rates associated with them. Therefore, amplification introduces further modifications into the cDNA molecules. In one embodiment, during amplification with the adapter-specific primers, a detectable label, e.g., a fluorophore, is added to single-stranded cDNA molecules. Amplification therefore also serves to create DNA complexes that do not occur in nature, at least because (i) cDNA does not exist *in vivo*, (i) adapter sequences are added to the ends of cDNA molecules to make DNA sequences that do not exist *in vivo,* (ii) the error rate associated with amplification further creates DNA sequences that do not exist *in vivo,* (iii) the disparate structure of the cDNA molecules as compared to what exists in nature and (iv) the chemical addition of a detectable label to the cDNA molecules.

In some embodiments, the expression of a gene expression product of interest is detected at the nucleic acid level via detection of non-natural cDNA molecules.

The gene expression products described herein include RNA comprising the entire or partial sequence of any of the nucleic acid sequences of interest, or their non-natural cDNA product, obtained synthetically *in vitro* in a reverse transcription reaction. The term "fragment" is intended to refer to a portion of the polynucleotide that generally comprise at least 10, 15, 20, 50, 75, 100, 150, 200, 250, 300, 350, 400, 450, 500, 550, 600, 650, 700, 800, 900, 1,000, 1,200, or 1,500 contiguous nucleotides, or up to the number of nucleotides present in a full-length gene expression product polynucleotide disclosed herein. A fragment of a gene expression product polynucleotide will generally encode at least 15, 25, 30, 50, 100, 150, 200, or 250 contiguous amino acids, or up to the total number of amino acids present in a full-length gene expression product protein of the disclosure.

In certain embodiments, a gene expression profile may be obtained by whole transcriptome shotgun sequencing ("WTSS" or "RNAseq"; see, e.g., Ryan et al BioTechniques 45: 81- 94), which makes the use of high-throughput sequencing technologies to sequence cDNA in order to about information about a sample's RNA content. In general terms, cDNA is made from RNA, the cDNA is amplified, and the amplification products are sequenced.

After amplification, the cDNA or derivative thereof may be sequenced using any convenient method. For example, the fragments may be sequenced using Illumina's reversible terminator method, Roche's pyrosequencing method (454), Life Technologies' sequencing by ligation (the SOLiD platform) or Life Technologies' Ion Torrent platform. Examples of such methods are described in the following references: Margulies et al (Nature 2005 437: 376-80); Ronaghi et al (Analytical Biochemistry 1996 242: 84-9); Shendure (Science 2005 309: 1728); Imelfort et al (Brief Bioinform. 2009 10:609-18); Fox et al (Methods Mol Biol. 2009;553:79-108); Appleby et al (Methods Mol Biol. 2009;513: 19-39) and Morozova (Genomics. 2008 92:255-64), which are incorporated by reference for the general descriptions of the methods and the particular steps of the methods, including all starting products, reagents, and final products for each of the steps. As may be apparent, forward and reverse sequencing primer sites that compatible with a selected next-generation sequencing platform may be added to the ends of the fragments during the amplification step.

In other embodiments, the products may be sequenced using nanopore sequencing (e.g. as described in Soni et al Clin Chem 53: 1996-2001 2007, or as described by Oxford Nanopore Technologies). Nanopore sequencing is a single-molecule sequencing technology whereby a single molecule of DNA is sequenced directly as it passes through a nanopore. A nanopore is a small hole, of the order of 1 nanometer in diameter. Immersion of a nanopore in a conducting fluid and application of a potential (voltage) across it results in a slight electrical current due to conduction of ions through the nanopore. The amount of current which flows is sensitive to the size and shape of the nanopore. As a DNA molecule passes through a nanopore, each nucleotide on the DNA molecule obstructs the nanopore to a different degree, changing the magnitude of the current through the nanopore in different degrees. Thus, this change in the current as the DNA molecule passes through the nanopore represents a reading of the DNA sequence. Nanopore sequencing technology as disclosed in U.S. Pat. Nos. 5,795,782, 6,015,714, 6,627,067, 7,238,485 and 7,258,838 and U.S. patent application publications US2006003171 and US20090029477.

In some embodiments, the gene expression product of the subject methods is a protein, and the amount of protein in a particular biological sample is analyzed using a classifier derived from protein data obtained from cohorts of samples. The amount of protein may be determined by one or more of the following: enzyme-linked immunosorbent assay (ELISA), mass spectrometry, blotting, or immunohistochemistry.

In some embodiments, gene expression product markers and alternative splicing markers may be determined by microarray analysis using, for example, Affymetrix arrays, cDNA microarrays, oligonucleotide microarrays, spotted microarrays, or other microarray products from Biorad, Agilent, or Eppendorf. Microarrays provide particular advantages because they may contain a large number of genes or alternative splice variants that may be assayed in a single experiment. In some cases, the microarray device may contain the entire human genome or transcriptome or a substantial fraction thereof allowing a comprehensive evaluation of gene expression patterns, genomic sequence, or alternative splicing. Markers may be found using standard molecular biology and microarray analysis techniques as described in Sambrook Molecular Cloning a Laboratory Manual 2001 and Baldi, P., and Hatfield, W. G., DNA Microarrays and Gene Expression 2002.

Microarray analysis generally begins with extracting and purifying nucleic acid from a biological sample, (e.g. a biopsy or fine needle aspirate) using various approaches. For expression and alternative splicing analysis it may be advantageous to extract and/or purify RNA from DNA. It may further be advantageous to extract and/or purify niRNA from other forms of RNA, such as tRNA and rRNA.

Purified nucleic acid may further be labeled with a fluorescent label, radionuclide, or chemical label such as biotin, digoxigenin, or digoxin for example by reverse transcription, polymerase chain reaction (PCR), ligation, chemical reaction or other techniques. The labeling may be direct or indirect which may further require a coupling stage. The coupling stage can occur before hybridization, for example, using aminoallyl-UTP and NHS amino-reactive dyes (like cyanine dyes) or after, for example, using biotin and labelled streptavidin. In one example, modified nucleotides (e.g. at a 1 aaUTP: 4 TTP ratio) are added enzymatically at a lower rate compared to normal nucleotides, typically resulting in 1 every 60 bases (measured with a spectrophotometer). The aaDNA may then be purified with, for example, a column or a diafiltration device. The aminoallyl group is an amine group on a long linker attached to the nucleobase, which reacts with a reactive label (e.g. a fluorescent dye).

The labeled samples may then be mixed with a hybridization solution which may contain sodium dodecyl sulfate (SDS), SSC, dextran sulfate, a blocking agent (such as COT1 DNA, salmon sperm DNA, calf thymus DNA, PolyA or PolyT), Denhardt's solution, formamine, or a combination thereof.

A hybridization probe is a fragment of DNA or RNA of variable length, which is used to detect in DNA or RNA samples the presence of nucleotide sequences (the DNA target) that are complementary to the sequence in the probe. The probe thereby hybridizes to single-stranded nucleic acid (DNA or RNA) whose base sequence allows probe-target base pairing due to complementarity between the probe and target. The labeled probe is first denatured (by heating or under alkaline conditions) into single-stranded DNA and then hybridized to the target DNA.

To detect hybridization of the probe to its target sequence, the probe is tagged (or labeled) with a molecular marker; commonly used markers are 32P or Digoxigenin, which is nonradioactive antibody-based marker. DNA sequences or RNA transcripts that have moderate to high sequence complementarity (e.g. at least 70%, 80%, 90%, 95%, 96%, 97%, 98%, 99%, or more complementarity) to the probe are then detected by visualizing the hybridized probe via autoradiography or other imaging techniques. Detection of sequences with moderate or high complementarity depends on how stringent the hybridization conditions were applied; high stringency, such as high hybridization temperature and low salt in hybridization buffers, permits only hybridization between nucleic acid sequences that are highly similar, whereas low stringency, such as lower temperature and high salt, allows hybridization when the sequences are less similar. Hybridization probes used in DNA microarrays refer to DNA covalently attached to an inert surface, such as coated glass slides or gene chips, and to which a mobile cDNA target is hybridized.

A mix comprising target nucleic acids to be hybridized to probes on an array may be denatured by heat or chemical approaches and added to a port in a microarray. The holes may then be sealed and the microarray hybridized, for example, in a hybridization oven, where the microarray is mixed by rotation, or in a mixer. After an overnight hybridization, non-specific binding may be washed off (e.g., with SDS and SSC). The microarray may then be dried and scanned in a machine comprising a laser that excites the dye and a detector that measures emission by the dye. The image may be overlaid with a template grid and the intensities of the features (e.g., a feature comprising several pixels) may be quantified.

Various kits may be used for the amplification of nucleic acid and probe generation of the subject methods. Examples of kit that may be used in the present disclosure include but are not limited to Nugen WT-Ovation^{™} FFPE kit, cDNA amplification kit with Nugen Exon Module and Frag/Label module. The NuGEN WT-Ovation^{™} FFPE System V2 is a whole transcriptome amplification system that enables conducting global gene expression analysis on the vast archives of small and degraded RNA derived from FFPE samples. The system is comprised of reagents and a protocol required for amplification of as little as 50 ng of total FFPE RNA. The protocol may be used for qPCR, sample archiving, fragmentation, and labeling. The amplified cDNA may be fragmented and labeled in less than two hours for GeneChip^{™} 3' expression array analysis using NuGEN's FL-Ovation^{™} cDNA Biotin Module V2. For analysis using Affymetrix GeneChip^{™} Exon and Gene ST arrays, the amplified cDNA may be used with the WT-Ovation^{™} Exon Module, then fragmented and labeled using the FL-Ovation^{™} cDNA Biotin Module V2. For analysis on Agilent arrays, the amplified cDNA may be fragmented and labeled using NuGEN's FL-Ovation^{™} cDNA Fluorescent Module.

In some embodiments, Ambion^{™} WT-expression kit may be used. Ambion WT-expression kit allows amplification of total RNA directly without a separate ribosomal RNA (rRNA) depletion step. With the Ambion^{™} WT Expression Kit, samples as small as 50 ng of total RNA may be analyzed on Affymetrix^{™} GeneChip^{™} Human, Mouse, and Rat Exon and Gene 1.0 ST Arrays. In addition to the lower input RNA requirement and high concordance between the Affymetrix^{™} method and TaqMan^{™} real-time PCR data, the Ambion^{™} WT-expression kit provides a significant increase in sensitivity. For example, a greater number of probe sets detected above background may be obtained at the exon level with the Ambion^{™} WT-expression kit as a result of an increased signal-to-noise ratio. The Ambion^{™}-expression kit may be used in combination with additional Affymetrix^{™} labeling kits. In some embodiments, AmpTec^{™} Trinucleotide Nano mRNA Amplification kit (6299-A15) may be used in the subject methods. The ExpressArt^{™} TRinucleotide^{™} mRNA amplification Nano kit is suitable for a wide range, from 1 ng to 700 ng of input total RNA. According to the amount of input total RNA and the required yields of aRNA, it may be used for 1-round (input >300 ng total RNA) or 2-rounds (minimal input amount 1 ng total RNA), with aRNA yields in the range of >10 µg. AmpTec's proprietary TRinucleotide^{™} priming technology results in preferential amplification of mRNAs (independent of the universal eukaryotic 3'-poly(A)-sequence), combined with selection against rRNAs. This kit may be used in combination with cDNA conversion kit and Affymetrix^{™} labeling kit.

The raw data may then be normalized, for example, by subtracting the background intensity and then dividing the intensities making either the total intensity of the features on each channel equal or the intensities of a reference gene and then the t-value for all the intensities may be calculated. More sophisticated methods, include z-ratio, loess and lowess regression and RMA (robust multichip analysis), such as for Affymetrix chips.

In some embodiments, the above described methods may be used for determining transcript expression levels for training (e.g., using a classifier training module) a classifier to differentiate whether a subject has UIP or non-UIP. In some embodiments, the above described methods may be used for determining transcript expression levels for inputting into a classifier module that is able to differentiate whether a sample is UIP or non-UIP.

### DATA ANALYSIS

### (i) Comparison of Sample to Normal

In some embodiments, results of molecular profiling performed on a sample from a subject ("test sample") may be compared to a biological sample that is known or suspected to be normal ("normal sample"). In some embodiments, a normal sample is a sample that does not comprise or is expected to not comprise an ILD, or conditions under evaluation, or may test negative in the molecular profiling assay for the one or more ILDs under evaluation. In some embodiments, a normal sample is that which is or is expected to be free of any ILD, or a sample that may test negative for any ILD in the molecular profiling assay. The normal sample may be from a different subject from the subject being tested, or from the same subject. In some cases, the normal sample is a lung tissue sample obtained from a subject such as the subject being tested for example. The normal sample may be assayed at the same time, or at a different time from the test sample. In some embodiments, a normal sample is a sample that is known or suspected to be from a non-smoker. In particular embodiments, the normal sample is a sample that has been confirmed by at least two expert pathologists to be a non-UIP sample. In particular embodiments, the normal sample is a sample that has been confirmed by at least two expert pathologists to be a non-IPF sample.

The results of an assay on the test sample may be compared to the results of the same assay on a sample having a known disease state (e.g., normal, affected by a selected ILD (e.g., IPF, NSIP, etc.), smoker, non-smoker, non-UIP, UIP). In some cases the results of the assay on the normal sample are from a database, or a reference. In some cases, the results of the assay on the normal sample are a generally accepted value or range of values by those skilled in the art. In some cases the comparison is qualitative. In other cases the comparison is quantitative. In some cases, qualitative or quantitative comparisons may involve but are not limited to one or more of the following: comparing fluorescence values, spot intensities, absorbance values, chemiluminescent signals, histograms, critical threshold values, statistical significance values, gene product expression levels, gene product expression level changes, alternative exon usage, changes in alternative exon usage, protein levels, DNA polymorphisms, copy number variations, indications of the presence or absence of one or more DNA markers or regions, or nucleic acid sequences.

### (ii) Evaluation of Results

In some embodiments, the molecular profiling results are evaluated using various approaches for correlating gene product expression levels or alternative exon usage with specific phenotypes such as a particular ILD, or normalcy (e.g. disease or condition free). In some cases, a specified statistical confidence level may be determined in order to provide a diagnostic confidence level. For example, it may be determined that a confidence level of greater than 90% may be a useful predictor of the presence of an ILD or of a smoker or non-smoker status. In other embodiments, more or less stringent confidence levels may be chosen. For example, a confidence level of about or at least about 50%, 60%, 70%, 75%, 80%, 85%, 90%, 95%, 97.5%, 99%, 99.5%, or 99.9% may be chosen as a useful phenotypic predictor. The confidence level provided may in some cases be related to the quality of the sample, the quality of the data, the quality of the analysis, the specific methods used, and/or the number of gene expression products analyzed. The specified confidence level for providing a diagnosis may be chosen on the basis of the expected number of false positives or false negatives and/or cost. Methods for choosing parameters for achieving a specified confidence level or for identifying markers with diagnostic power include but are not limited to Receiver Operating Characteristic (ROC) curve analysis, binormal ROC, principal component analysis, partial least squares analysis, singular value decomposition, least absolute shrinkage and selection operator analysis, least angle regression, and the threshold gradient directed regularization method.

### (iii) Data analysis

Raw gene expression level and alternative splicing data may in some cases be improved through the application of methods and/or processes designed to normalize and or improve the reliability of the data. In some embodiments of the present disclosure the data analysis requires a computer or other device, machine or apparatus for application of the various methods and/or processes described herein due to the large number of individual data points that are processed. A "machine learning classifier" refers to a computational- based prediction data structure or method, employed for characterizing a gene expression profile. The signals corresponding to certain expression levels, which are obtained by, e.g., exome enriched RNA sequencing or microarray-based hybridization assays, are typically subjected to the classifier to classify the expression profile. Supervised learning generally involves "training" a classifier to recognize the distinctions among classes and then "testing" the accuracy of the classifier on an independent test set. For new, unknown samples the classifier may be used to predict the class in which the samples belong. In various embodiments, such training is be achieved, e.g., using a classifier training module.

In some cases, the robust multi-array average (RMA) method may be used to normalize raw data. The RMA method begins by computing background-corrected intensities for each matched cell on a number of microarrays. The background corrected values are restricted to positive values as described by Irizarry et al. Biostatistics 2003 April 4 (2): 249-64. After background correction, the base-2 logarithm of each background corrected matched-cell intensity is then obtained. The back-ground corrected, log-transformed, matched intensity on each microarray is then normalized using the quantile normalization method in which for each input array and each probe expression value, the array percentile probe value is replaced with the average of all array percentile points, this method is more completely described by Bolstad et al. Bioinformatics 2003. Following quantile normalization, the normalized data may then be fit to a linear model to obtain an expression measure for each probe on each microarray. Tukey's median polish algorithm (Tukey, J. W., Exploratory Data Analysis. 1977) may then be used to determine the log-scale expression level for the normalized probe set data.

Various other software and/or hardware modules or processes may be implemented. In certain methods, feature selection and model estimation may be performed by logistic regression with *lasso* penalty using *glmnet* (Friedman J, Hastie T, Tibshirani R. Regularization Paths for Generalized Linear Models via Coordinate Descent. Journal of statistical software 2010; 33(1): 1-22). Raw reads may be aligned using TopHat (Trapnell C, Pachter L, Salzberg SL. TopHat: discovering splice junctions with RNA-Seq. Bioinformatics 2009; 25(9): 1105-11.). Gene counts may be obtained using HTSeq (Anders S, Pyl PT, Huber W. HTSeq-a Python framework to work with high-throughput sequencing data. Bioinformatics 2014.) and normalized using DESeq (Love MI, Huber W, Anders S. Moderated estimation of fold change and dispersion for RNA-Seq data with DESeq2; 2014). In methods, top features (N ranging from 10 to 200) were used to train a linear support vector machine (SVM) (Suykens JAK, Vandewalle J. Least Squares Support Vector Machine Classifiers. Neural Processing Letters 1999; 9(3): 293-300) using the *e1071* library (Meyer D. Support vector machines: the interface to libsvm in package e1071. 2014.). Confidence intervals may be computed using the pROC package (Robin X, Turck N, Hainard A, et al. pROC: an open-source package for R and S+ to analyze and compare ROC curves. BMC bioinformatics 2011; 12: 77)

In addition, data may be filtered to remove data that may be considered suspect. In some embodiments, data deriving from microarray probes that have fewer than about 4, 5, 6, 7 or 8 guanosine and cytosine nucleotides may be considered to be unreliable due to their aberrant hybridization propensity or secondary structure issues. Similarly, data deriving from microarray probes that have more than about 12, 13, 14, 15, 16, 17, 18, 19, 20, 21, or 22 guanosine and cytosine nucleotides may be considered unreliable due to their aberrant hybridization propensity or secondary structure issues.

In some cases, unreliable probe sets may be selected for exclusion from data analysis by ranking probe-set reliability against a series of reference datasets. For example, RefSeq or Ensembl (EMBL) are considered very high quality reference datasets. Data from probe sets matching RefSeq or Ensembl sequences may in some cases be specifically included in microarray analysis experiments due to their expected high reliability. Similarly data from probe-sets matching less reliable reference datasets may be excluded from further analysis, or considered on a case by case basis for inclusion. In some cases, the Ensembl high throughput cDNA (HTC) and/or mRNA reference datasets may be used to determine the probe-set reliability separately or together. In other cases, probe-set reliability may be ranked. For example, probes and/or probe-sets that match perfectly to all reference datasets such as for example RefSeq, HTC, HTSeq, and mRNA, may be ranked as most reliable (1). Furthermore, probes and/or probe-sets that match two out of three reference datasets may be ranked as next most reliable (2), probes and/or probe-sets that match one out of three reference datasets may be ranked next (3) and probes and/or probe sets that match no reference datasets may be ranked last (4). Probes and or probe-sets may then be included or excluded from analysis based on their ranking. For example, one may choose to include data from category 1, 2, 3, and 4 probe-sets; category 1, 2, and 3 probe-sets; category 1 and 2 probe-sets; or category 1 probe-sets for further analysis. In another example, probe-sets may be ranked by the number of base pair mismatches to reference dataset entries. It is understood that there are many methods understood in the art for assessing the reliability of a given probe and/or probe-set for molecular profiling and the methods of the present disclosure encompass any of these methods and combinations thereof.

In some embodiments of the present disclosure, data from probe-sets may be excluded from analysis if they are not expressed or expressed at an undetectable level (not above background). A probe-set is judged to be expressed above background if for any group:
Integral from T0 to Infinity of the standard normal distribution<Significance (0.01) Where: T0=Sqr(GroupSize) (T-P)/Sqr(Pvar); GroupSize=Number of CEL files in the group, T=Average of probe scores in probe-set, P=Average of Background probes averages of GC content, and Pvar=Sum of Background probe variances/(Number of probes in probe-set) 2,

This allows probe-sets in which the average of probe-sets in a group is greater than the average expression of background probes of similar GC content as the probe-set probes as the center of background for the probe-set, and enables one to derive the probe-set dispersion from the background probe-set variance.

In some embodiments of the present disclosure, probe-sets that exhibit no, or low variance may be excluded from further analysis. Low-variance probe-sets are excluded from the analysis via a Chi-Square test. A probe-set is considered to be low-variance if its transformed variance is to the left of the 99 percent confidence interval of the Chi-Squared distribution with (N-l) degrees of freedom. (N-l)*Probe-set Variance/(Gene Probe-set Variance) of about Chi-Sq(N-l), where N is the number of input CEL files, (N-l) is the degrees of freedom for the Chi-Squared distribution, and the "probe-set variance for the gene" is the average of probe-set variances across the gene. In some embodiments of the present disclosure, probe-sets for a given gene or transcript cluster may be excluded from further analysis if they contain less than a minimum number of probes that pass through the previously described filter steps for GC content, reliability, variance and the like. For example, in some embodiments, probe-sets for a given gene or transcript cluster may be excluded from further analysis if they contain less than about 1, 2, 3, 4, 5, 6, 7, 8, 9, 10, 11, 12, 13, 14, 15, or less than about 20 probes.

Methods of data analysis of gene expression levels or of alternative splicing may further include the use of a feature selection method and/or process as provided herein. In some embodiments of the present disclosure, feature selection is provided by use of the LIMMA software package (Smyth, G. K. (2005). Limma: linear models for microarray data. In: Bioinformatics and Computational Biology Solutions using R and Bioconductor, R. Gentleman, V. Carey, S. Dudoit, R. Irizarry, W. Huber (eds.), Springer, New York, pages 397-420).

Methods of data analysis of gene expression levels and/or of alternative splicing may further include the use of a pre-classifier method and/or process (e.g., implemented by a pre-classifier analysis module). For example, a method and/or process may use a cell-specific molecular fingerprint to pre-classify the samples according to their composition and then apply a correction/normalization factor. This data/information may then be fed in to a final classification method and/or process which may incorporate that information to aid in the final diagnosis.

In certain embodiments, the methods of the present disclosure include the use of a pre-classifier method and/or process (e.g., implemented by a pre-classifier analysis module) that uses a molecular fingerprint to pre-classify the samples as smoker or non-smoker prior to application of a UIP/non-UIP classifier of the present disclosure.

Methods of data analysis of gene expression levels and/or of alternative splicing may further include the use of a classifier method and/or process (e.g., implemented by a classifier analysis module) as provided herein. In some embodiments of the present disclosure a diagonal linear discriminant analysis, k-nearest neighbor classifier, support vector machine (SVM) classifier, linear support vector machine, random forest classifier, or a probabilistic model-based method or a combination thereof is provided for classification of microarray data. In some embodiments, identification markers that distinguish samples (e.g. UIP from non-UIP, first ILD from second ILD, normal vs ILD), or distinguish subtypes (e.g. IPF vs. NSIP) are selected based on statistical significance of the difference in expression levels between classes of interest. In some cases, the statistical significance is adjusted by applying a Benjamini Hochberg procedure or another correction for false discovery rate (FDR).

In some cases, the classifier may be supplemented with a meta-analysis approach such as that described by Fishel and Kaufman et al. 2007 Bioinformatics 23(13): 1599-606. In some cases, the classifier may be supplemented with a meta-analysis approach such as a repeatability analysis. In some cases, the repeatability analysis selects markers that appear in at least one predictive expression product marker set.

Examples of methods for deriving and applying posterior probabilities to the analysis of microarray data are provided in Smyth, G. K. 2004 Stat. Appi. Genet. Mol. Biol. 3: Article 3, which is entirely incorporated herein by reference. In some cases, the posterior probabilities may be used to rank the markers provided by the classifier. In some cases, markers may be ranked according to their posterior probabilities and those that pass a chosen threshold may be chosen as markers whose differential expression is indicative of or diagnostic for samples that are for example UIP or non-UIP. Illustrative threshold values include prior probabilities of 0.7, 0.75, 0.8, 0.85, 0.9, 0.925, 0.95, 0.975, 0.98, 0.985, 0.99, 0.995 or higher.

A statistical evaluation of the results of the molecular profiling may provide, but is not limited to providing, a quantitative value or values indicative of one or more of the following: the likelihood of diagnostic accuracy; the likelihood a sample is UIP; the likelihood a sample is non-UIP; the likelihood of an ILD; the likelihood of a particular ILD; the likelihood of the success of a particular therapeutic intervention, the likelihood the subject is a smoker, and the likelihood the subject is a non-smoker. Thus a physician, who is not likely to be trained in genetics or molecular biology, need not understand the raw data. Rather, the data is presented directly to the physician in its most useful form to guide patient care. The results of the molecular profiling may be statistically evaluated using a number of methods, including, but not limited to: the students T test, the two-sided T test, pearson rank sum analysis, hidden Markov model analysis, analysis of q-q plots, principal component analysis, one-way ANOVA, two-way ANOVA, LIMMA and the like.

In some embodiments of the present disclosure, the use of molecular profiling alone or in combination with cytological analysis may provide a classification, identification, or diagnosis that is between about 85% accurate and about 99% or about 100% accurate. In some cases, the molecular profiling process and/or cytology provide a classification, identification, diagnosis of an ILD that is about, or at least about 85%, 86%, 87%, 88%, 90%, 91%, 92%, 93%, 94%, 95%, 96%, 97%, 97.5%, 98%, 98.5%, 99%, 99.5%, 99.75%, 99.8%, 99.85%, or 99.9% accurate. In some embodiments, the molecular profiling process and/or cytology provide a classification, identification, or diagnosis of the presence of a particular ILD type (e.g. IPF; NSIP; HP) that is about, or at least about 85%, 86%, 87%, 88%, 90%, 91%, 92%, 93%, 94%, 95%, 96%, 97%, 97.5%, 98%, 98.5%, 99%, 99.5%, 99.75%, 99.8%, 99.85%, or 99.9% accurate.

In some cases, accuracy may be determined by tracking the subject over time to determine the accuracy of the original diagnosis. In other cases, accuracy may be established in a deterministic manner or using statistical methods. For example, receiver operator characteristic (ROC) analysis may be used to determine the optimal assay parameters to achieve a specific level of accuracy, specificity, positive predictive value, negative predictive value, and/or false discovery rate.

In some embodiments of the present disclosure, gene expression products and compositions of nucleotides encoding for such products which are determined to exhibit the greatest difference in expression level or the greatest difference in alternative splicing between UIP and non-UIP, between UIP and normal, and/or between smoker and non-smoker may be chosen for use as molecular profiling reagents of the present disclosure. Such gene expression products may be particularly useful by providing a wider dynamic range, greater signal to noise, improved diagnostic power, lower likelihood of false positives or false negative, or a greater statistical confidence level than other methods.

In other embodiments of the present disclosure, the use of molecular profiling alone or in combination with cytological analysis may reduce the number of samples scored as non-diagnostic by about, or at least about 100%, 99%, 95%, 90%, 80%, 75%, 70%, 65%, or about 60% when compared to the use of standard cytological techniques used in the art. In some cases, the methods of the present disclosure may reduce the number of samples scored as indeterminate or suspicious by about, or at least about 100%, 99%, 98%, 97%, 95%, 90%, 85%, 80%, 75%, 70%, 65%, or about 60%, when compared to the standard cytological methods used in the art.

In some cases the results of the molecular profiling assays, are entered into a database for access by representatives or agents of a molecular profiling business, the individual, a medical provider, or insurance provider. In some cases assay results include sample classification, identification, or diagnosis by a representative, agent or consultant of the business, such as a medical professional. In other cases, a computer analysis of the data is provided automatically. In some cases the molecular profiling business may bill the individual, insurance provider, medical provider, researcher, or government entity for one or more of the following: molecular profiling assays performed, consulting services, data analysis, reporting of results, or database access.

In some embodiments of the present disclosure, the results of the molecular profiling are presented as a report on a computer screen or as a paper record. In some cases, the report may include, but is not limited to, such information as one or more of the following: the number of genes differentially expressed, the suitability of the original sample, the number of genes showing differential alternative splicing, a diagnosis, a statistical confidence for the diagnosis, the likelihood the subject is a smoker, the likelihood of an ILD, and indicated therapies.

### (iv) Categorization of Samples Based on Molecular Profiling Results

The results of the molecular profiling may be classified, e.g., into one of the following: smoker, non-smoker, ILD, a particular type of ILD, a non-ILD, or non-diagnostic (providing inadequate information concerning the presence or absence of an ILD). In some cases, the results of the molecular profiling may be classified into IPF versus NSIP categories. In particular cases, the results are classified as UIP or non-UIP.

In some embodiments of the present disclosure, results are classified using a trained classifier. The trained classifier may be a trained algorithm. Trained classifiers of the present disclosure implement methods and/or processes that have been developed using a reference set of known UIP and non-UIP samples. In some embodiments, training (e.g., using a classifier training module) comprises the comparison of gene expression product levels in a first set of biomarkers from a UIP sample to gene expression product levels in a second set of biomarkers from a non-UIP sample, where the first set of biomarkers includes at least one biomarker that is not in the second set. In some embodiments, training (e.g., using a classifier training module) comprises comparison of gene expression product levels in a first set of biomarkers from a first ILD that is non-UIP to gene expression product levels in a second set of biomarkers from a second ILD that is UIP, where the first set of biomarkers includes at least one biomarker that is not in the second set. In some embodiments, training (e.g., using a classifier training module) further comprises comparison of gene expression product levels in a first set biomarkers from a first subject that is a smoker to gene expression product levels in a second set of biomarkers from a second subject that is a non-smoker, where the first set of biomarkers includes at least one biomarker that is not in the second set. In some embodiments, either the entire classifier or portions of the classifier may be trained (e.g., using a classifier training module) using comparisons of expression levels of biomarker panels within a classification panel against all other biomarker panels (or all other biomarker signatures) used in the classifier. In some embodiments, either the entire classifier or portions of the classifier may be trained (e.g., using a classifier training module) using comparisons of expression levels measured in pooled samples comprising at least 2, 3, 4, 5, or more individual samples obtained from a single subject. In some embodiments, either the entire classifier or portions of the classifier may be trained (e.g., using a classifier training module) using comparisons of *in silico* pooled expression levels, as described herein, wherein the *in silico* pooled expression levels comprise pooled expression levels from at least 2, 3, 4, 5, or more individual samples obtained from a single subject. In some embodiments, classifiers trained, as described in this paragraph, compare 1; 2; 3; 4; 5; 6; 7; 8; 9; 10; 11; 12; 13; 14; 15; 16; 17; 18; 19; 20; 21; 22; 23; 24; 25; 26; 27; 28; 29; 30; 31; 32; 33; 34; 35; 36; 37; 38; 39; 40; 41; 42; 43; 44; 45; 46; 47; 48; 49; 50; 51; 52; 53; 54; 55; 56; 57; 58; 59; 60; 61; 62; 63; 64; 65; 66; 67; 68; 69; 70; 71; 72; 73; 74; 75; 76; 77; 78; 79; 80; 81; 82; 83; 84; 85; 86; 87; 88; 89; 90; 91; 92; 93; 94; 95; 96; 97; 98; 99; 100; 101; 102; 103; 104; 105; 106; 107; 108; 109; 110; 111; 112; 113; 114; 115; 116; 117; 118; 119; 120; 121; 122; 123; 124; 125; 126; 127; 128; 129; 130; 131; 132; 133; 134; 135; 136; 137; 138; 139; 140; 141; 142; 143; 144; 145; 146; 147; 148; 149; 150; or 151 of SEQ ID NOs: 1-151, or any combination thereof between a test sample and a reference sample or a group of reference samples to determine whether the test sample is UIP or non-UIP. In particular aspects, such a classifier compares additional genes, e.g., 1, 2, 3, 4, 5, 6, 7, 8, or more additional genes. In other aspects, the classifier omits certain of the above-mentioned genes, e.g., 1, 2, 3, 4, 5, 6, 7, 8, or more, of these genes, while in some cases including other genes.

In some embodiments, classifiers trained, as described herein, compare gene expression levels of 1; 2; 3; 4; 5; 6; 7; 8; 9; 10; 11; 12; 13; 14; 15; 16; 17; 18; 19; 20; 21; 22; 23; 24; 25; 26; 27; 28; 29; 30; 31; 32; 33; 34; 35; 36; 37; 38; 39; 40; 41; 42; 43; 44; 45; 46; 47; 48; 49; 50; 51; 52; 53; 54; 55; 56; 57; 58; 59; 60; 61; 62; 63; 64; 65; 66; 67; 68; 69; 70; 71; 72; 73; 74; 75; 76; 77; 78; 79; 80; 81; 82; 83; 84; 85; 86; 87; 88; 89; 90; 91; 92; 93; 94; 95; 96; 97; 98; 99; 100; 101; 102; 103; 104; 105; 106; 107; 108; 109; 110; 111; 112; 113; 114; 115; 116; 117; 118; 119; 120; 121; 122; 123; 124; 125; 126; 127; 128; 129; 130; 131; 132; 133; 134; 135; 136; 137; 138; 139; 140; 141; 142; 143; 144; 145; 146; 147; 148; 149; 150; 151; 152; 153; 154; 155; 156; 157; 158; 159; 160; 161; 162; 163; 164; 165; 166; 167; 168; 169; 170; 171; 172; 173; 174; 175; 176; 177; 178; 179; 180; 181; 182; 183; 184; 185; 186; 187; 188; 189; or 190 of the genes listed in Table 5 between a test sample and a reference sample or a group of reference samples to determine whether the test sample is UIP or non-UIP. In particular aspects, such a classifier compares additional genes, e.g., 1, 2, 3, 4, 5, 6, 7, 8, or more additional genes. In other aspects, the classifier omits certain of the above-mentioned genes, e.g., 1, 2, 3, 4, 5, 6, 7, 8, or more, of these genes, while in some cases including other genes.

Classifiers suitable for categorization of samples include but are not limited to k-nearest neighbor classifiers, support vector machines, linear discriminant analysis, diagonal linear discriminant analysis, updown, naive Bayesian classifiers, neural network classifiers, hidden Markov model classifiers, genetic classifiers, or any combination thereof.

In some cases, trained classifiers of the present disclosure may incorporate data other than gene expression or alternative splicing data, such as, but not limited to, DNA polymorphism data, sequencing data, scoring or diagnosis by cytologists or pathologists of the present disclosure, information provided by the pre-classifier method and/or process of the present disclosure, or information about the medical history of the subject of the present disclosure.

When classifying a biological sample for diagnosis of ILD (e.g., with UIP), there are typically two possible outcomes from a binary classifier. Similarly, when classifying a biological sample for diagnosis of smoker, there are typically two possible outcomes from a binary classifier. When a binary classifier is compared with actual true values (e.g., values from a biological sample), there are typically four possible outcomes. If the outcome from a prediction is p (where "p" is a positive classifier output, such as a particular ILD) and the actual value is also p, then it is called a true positive (TP); however if the actual value is n then it is said to be a false positive (FP). Conversely, a true negative (TN) has occurred when both the prediction outcome and the actual value are n (where "n" is a negative classifier output, such as no ILD, or absence of a particular disease tissue as described herein), and false negative (FN) is when the prediction outcome is n while the actual value is p. In one embodiment, consider a diagnostic test that seeks to determine whether a person has a certain disease. A false positive (FP) in this case occurs when the person tests positive, but actually does not have the disease. A FN, on the other hand, occurs when the person tests negative, suggesting they are healthy, when they actually do have the disease. In some embodiments, a receiver operator characteristic (ROC) curve assuming real-world prevalence of subtypes may be generated by re-sampling errors achieved on available samples in relevant proportions.

The positive predictive value (PPV), or precision rate, or post-test probability of disease, is the proportion of patients with positive test results who are correctly diagnosed. It is the most important measure of a diagnostic method as it reflects the probability that a positive test reflects the underlying condition being tested for. Its value does however depend on the prevalence of the disease, which may vary. False positive rate (α)=FP/(FP+TN)-specificity; False negative rate (β)=FN/(TP+FN)-sensitivity; Power=sensitivity=1- β; Likelihood-ratio positive=sensitivity/(l-specificity); Likelihood-ratio negative=(1-sensitivity )/specificity.

The negative predictive value is the proportion of patients with negative test results who are correctly diagnosed. PPV and NPV measurements may be derived using appropriate disease subtype prevalence estimates. An estimate of the pooled disease prevalence may be calculated from the pool of indeterminates which roughly classify into B vs M by surgery. For subtype specific estimates, in some embodiments, disease prevalence may sometimes be incalculable because there are not any available samples. In these cases, the subtype disease prevalence may be substituted by the pooled disease prevalence estimate.

In some embodiments, the level of expression products or alternative exon usage is indicative of one or the following: IPF, NSIP, HP, UIP, non-UIP.

In some embodiments, the level of expression products or alternative exon usage is indicative that the subject is a smoker or a non-smoker.

In some embodiments, the results of the expression analysis of the subject methods provide a statistical confidence level that a given diagnosis is correct. In some embodiments, such statistical confidence level is at least about, or more than about 85%, 90%, 91%, 92%, 93%, 94%, 95%, 96%, 97%, 98%, 99% 99.5%, or more.

### Reports

A subject method and/or system may include generating a report that provides an indication that a sample (a lung tissue sample) is a UIP sample (e.g., using a report module). A subject method and/or system may include generating a report that provides an indication that a sample (a lung tissue sample) is a non-UIP sample (e.g., using a report module). A subject method and/or system may include generating a report that provides an indication that a sample (a lung tissue sample) is an ILD sample (e.g., using a report module). A subject diagnostic method can include generating a report that provides an indication as to whether an individual being tested has an ILD. A subject diagnostic method can include generating a report that provides an indication as to whether an individual being tested is, or is not a smoker. A subject method (or report module) can include generating a report that provides an indication as to whether an individual being tested has IPF (and not, e.g., an ILD other than IPF; e.g., the report can indicate that the individual has IPF and not NSIP).

In some embodiments, a subject method of diagnosing UIP vs. non-UIP involves generating a report (e.g., using a report module). Such a report can include information such as a likelihood that the patient has UIP; a likelihood that the patient has non-UIP; a likelihood that the patient has IPF; a likelihood that the patient is a smoker; a recommendation regarding further evaluation; a recommendation regarding therapeutic drug and/or device intervention; and the like.

For example, the methods disclosed herein can further include a step of generating or outputting a report providing the results of a subject diagnostic method, the report may be provided in the form of an electronic medium (e.g., an electronic display on a computer monitor), or in the form of a tangible medium (e.g., a report printed on paper or other tangible medium). An assessment as to the results of a subject diagnostic method (e.g., a likelihood that the patient has UIP; a likelihood that the patient has non-UIP; a likelihood that the patient has IPF; a likelihood that an individual has an ILD; a likelihood that an individual has IPF; a likelihood that an individual is a smoker) may be referred to as a "report" or, simply, a "score." A person or entity that prepares a report ("report generator") may also perform steps such as sample gathering, sample processing, and the like. Alternatively, an entity other than the report generator can perform steps such as sample gathering, sample processing, and the like. A diagnostic assessment report may be provided to a user. A "user" may be a health professional (e.g., a clinician, a laboratory technician, a physician (e.g., a cardiologist), etc.).

A subject report can further include one or more of: 1) service provider information; 2) patient data; 3) data regarding the expression level of a given gene product or set of gene products, a score or classifier decision; 4) follow-up evaluation recommendations; 5) therapeutic intervention or recommendations; and 6) other features.

### Further Evaluation

Based on the expression level of a given gene product or set of gene products, and/or based on a report (as described above), a physician or other qualified medical personnel can determine whether further evaluation of the test subject (the patient) is required. Further evaluation can include, e.g., spirometry.

### Therapeutic intervention

Based on the expression level of a given gene product or set of gene products, and/or based on a report (as described above), a physician or other qualified medical personnel can determine whether appropriate therapeutic intervention is advised. Therapeutic intervention includes drug-based therapeutic intervention, device-based therapeutic intervention, and surgical intervention. Where a report indicates a likelihood that an individual has UIP and/or IPF, drug-based therapeutic intervention includes, e.g., administering to the individual an effective amount of pirfenidone, prednisone, azathioprine, and/or N-acetylcysteine. Surgical intervention includes, e.g., arterial bypass surgery.

### Computer-Implemented Methods, Systems and Devices

The methods of the present disclosure may be computer-implemented, such that method steps (e.g., assaying, comparing, calculating, and the like) are automated in whole or in part.

Accordingly, the present disclosure provides methods, computer systems, devices and the like in connection with computer-implemented methods of facilitating a diagnosis of an interstitial lung disease (e.g., a diagnosis of UIP, non-UIP, IPF, NSIP, HP, etc.), including differential diagnosis.

The present disclosure further provides methods, computer systems, devices and the like in connection with computer-implemented methods of facilitating determination of smoker status (e.g., smoker vs. non-smoker).

The present disclosure further provides methods, computer systems, devices and the like in connection with computer-implemented methods of facilitating a diagnosis of an interstitial lung disease (e.g., a diagnosis of UIP, non-UIP, IPF, NSIP, HP, etc.), including differential diagnosis, wherein the methods further comprise determining a subjects smoker status (smoker vs. non-smoker) and incorporating smoker status into the determination of the subjects interstitial lung disease diagnosis. In some embodiments, (i) smoker status is incorporated into the interstitial lung disease diagnosis as a covariate in the model used during training (e.g., using a classifier training module). This approach boosts signal-to-noise ratio, particularly in data derived from smokers (where noise is higher), and allows data derived from smokers and non-smokers to be combined and used simultaneously. In some embodiments, (ii) smoker status is incorporated into the interstitial lung disease diagnosis by identifying one or more genes that are susceptible to smoker status bias and excluding such genes or weighing such genes differently than other genes that are not susceptible to smoker-status during interstitial lung disease diagnosis classifier training. In some embodiments, (iii) smoker status is incorporated into the interstitial lung disease diagnosis by constructing a tiered classification in which an initial classifier is trained to recognize the gene signatures that distinguish smokers from non-smokers (e.g., using a classifier training module). Once patient samples are pre-classified as "smoker" or "non-smoker" (e.g., using a pre-classifier analysis module), distinct classifiers that were each trained to distinguish UIP vs. non-UIP in smokers or non-smokers, respectively may be implemented to diagnose interstitial lung disease. In still further embodiments, such methods comprising the step of incorporating smoker status into the determination of the subjects interstitial lung disease diagnosis include a combination of one or more of the above mentioned methods of such incorporation (i.e., a combination of two or more of embodiments (i) to (iii) in the instant paragraph.

For example, the method steps, including obtaining values for biomarker levels, comparing normalized biomarker (gene) expression levels to a control level, calculating the likelihood of UIP or non-UIP (and in some cases the likelihood a subject is a smoker), generating a report, and the like, may be completely or partially performed by a computer program product. Values obtained may be stored electronically, e.g., in a database, and may be subjected to a classifier executed by a programmed computer (e.g., using a classifier analysis module).

For example, the methods and/or systems of the present disclosure can involve inputting a biomarker level (e.g., a normalized expression level of a gene product) into a classifier analysis module to execute a method and/or process to perform the comparing and calculating step(s) described herein, and generate a report (e.g., using a report module) as described herein, e.g., by displaying or printing a report to an output device at a location local or remote to the computer. The output to the report may be a score (e.g., numerical score (representative of a numerical value) or a non-numerical score (e.g., non-numerical output (e.g., "IPF", "No evidence of IPF") representative of a numerical value or range of numerical values. In other aspects, the output may indicate "UIP" vs. "non-UIP." In other aspects, the output may indicate "Smoker" vs. "Non-smoker"

The present disclosure thus provides a computer program product including a computer readable storage medium having software and/or hardware modules stored on it. The software and/or hardware modules can, when executed by a processor, execute relevant calculations based on values obtained from analysis of one or more biological sample (e.g., lung tissue sample) from an individual. The computer program product has stored therein a computer program for performing the calculation(s).

The present disclosure provides systems for executing the program described above, which system generally includes: a) a central computing environment or processor executing software and/or hardware modules; b) an input device, operatively connected to the computing environment, to receive patient data, wherein the patient data can include, for example, biomarker level or other value obtained from an assay using a biological sample from the patient, as described above; c) an output device, connected to the computing environment, to provide information to a user (e.g., medical personnel); and d) a method and/or process executed by the central computing environment (e.g., a processor), where the method and/or process is executed based on the data received by the input device, and wherein the method and/or process calculates a value, wherein the value is indicative of the likelihood the subject has UIP, non-UIP, an ILD, or IPF, as described herein.

The present disclosure also provides systems for executing the program described above, which system generally includes: a) a central computing environment or processor executing software and/or hardware modules; b) an input device, operatively connected to the computing environment, to receive patient data, wherein the patient data can include, for example, biomarker level or other value obtained from an assay using a biological sample from the patient, as described above; c) an output device, connected to the computing environment, to provide information to a user (e.g., medical personnel); and d) a method and/or process executed by the central computing environment (e.g., a processor), where the method and/or process is executed based on the data received by the input device, wherein the method and/or process calculates a value, which value is indicative of the likelihood the subject has UIP, non-UIP, an ILD, or IPF as described herein, and wherein the method and/or process uses smoking status (smoker vs. non-smoker) as a covariate in the model used during training. In some embodiments, the method and/or process excludes or weighs one or more genes that are susceptible to smoker status bias differently during classifier training to enrich the feature space used for training with genes that are not confounded or affected by smoking status.

In still further embodiments, the present disclosure provides systems for executing the program described above, which system generally includes: a) a central computing environment or processor executing software and/or hardware modules; b) an input device, operatively connected to the computing environment, to receive patient data, wherein the patient data can include, for example, biomarker level or other value obtained from an assay using a biological sample from the patient, as described above; c) an output device, connected to the computing environment, to provide information to a user (e.g., medical personnel); and d) a first method and/or process executed by the central computing environment (e.g., a processor), where the first method and/or process is executed based on the data received by the input device, wherein the first method and/or process calculates a value, which value is indicative of the likelihood a subject is a smoker or a non-smoker, as described herein, wherein the subject's status as a smoker or non-smoker causes the first method and/or process to apply a second method and/or process specifically trained (e.g., using a classifier training module) to distinguish UIP vs. non-UIP in smokers or non-smokers, respectively and e) wherein the second method and/or process is executed by the central computing environment (e.g., a processor), where the second method and/or process is executed based on the data received by the input device, and wherein the second method and/or process calculates a value, which value is indicative of the likelihood the subject has an ILD, as described herein.

### Computer Systems

Figure 7A illustrates a processing system 100 including at least one processor 102, or processing unit or plurality of processors, memory 104, at least one input device 106 and at least one output device 108, coupled together via a bus or group of buses 110. Processing system may be implemented on any suitable device, such as, for example, a host device, a personal computer, a handheld or laptop device, a personal digital assistant, a multiprocessor system, a microprocessor-based system, a programmable consumer electronic device, a minicomputer, a server computer, a web server computer, a mainframe computer, and/or a distributed computing environment that includes any of the above systems or devices

In certain embodiments, input device 106 and output device 108 may be the same device. An interface 112 can also be provided for coupling the processing system 100 to one or more peripheral devices, for example interface 112 may be a PCI card or PC card. At least one storage device 114 which houses at least one database 116 can also be provided.

The memory 104 may be any form of memory device, for example, volatile or nonvolatile memory, solid state storage devices, magnetic devices, etc. For example, in some embodiments, the memory 104 may be a random access memory (RAM), a memory buffer, a hard drive, a read-only memory (ROM), an erasable programmable read-only memory (EPROM), a database, and/or the like.

The processor 102 can include more than one distinct processing device, for example to handle different functions within the processing system 100. The processor 100 may be any suitable processing device configured to run or execute a set of instructions or code (e.g., stored in the memory) such as a general-purpose processor (GPP), a central processing unit (CPU), an accelerated processing unit (APU), a graphics processor unit (GPU), an application specific integrated circuit (ASIC), and/or the like. Such a processor 100 can run or execute a set of instructions or code stored in the memory associated with using a personal computer application, a mobile application, an internet web browser, a cellular and/or wireless communication (e.g., via a network), and/or the like. More specifically, the processor can execute a set of instructions or code stored in the memory 104 associated with analyzing and classifying data, as described herein.

Input device 106 receives input data 118 and can comprise, for example, a keyboard, a pointer device such as a pen-like device or a mouse, audio receiving device for voice controlled activation such as a microphone, data receiver or antenna such as a modem or wireless data adaptor, data acquisition card, etc. Input data 118 can come from different sources, for example keyboard instructions in conjunction with data received via a network.

Output device 108 produces or generates output data 120 and can comprise, for example, a display device or monitor in which case output data 120 is visual, a printer in which case output data 120 is printed, a port for example a USB port, a peripheral component adaptor, a data transmitter or antenna such as a modem or wireless network adaptor, etc. Output data 120 may be distinct and derived from different output devices, for example a visual display on a monitor in conjunction with data transmitted to a network. A user can view data output, or an interpretation of the data output, on, for example, a monitor or using a printer.

In some embodiments, the input device 106 and/or the output device 108 may be a communication interface configured to send and/or receive data via a network. More specifically, in such embodiments, the processing system 100 can act as a host device to one or more client devices (not shown in Figure 7A). As such, the processing system 100 can send data to (e.g., output data 120) and receive data from (e.g., input data 118) the client devices. Such a communication interface may be any suitable module and/or device that can place the processing system 100 in communication with a client device such as one or more network interface cards or the like. Such a network interface card can include, for example, an Ethernet port, a WiFi^{®} radio, a Bluetooth^{®} radio, a near field communication (NFC) radio, and/or a cellular radio that can place the client device 150 in communication with the host device 110 via a network or the like.

The storage device 114 may be any form of data or information storage system or method, for example, volatile or non-volatile memory, solid state storage devices, magnetic devices, etc. For example, in some embodiments, the storage device 114 may be a random access memory (RAM), a memory buffer, a hard drive, a read-only memory (ROM), an erasable programmable read-only memory (EPROM), a database, and/or the like.

In use, the processing system 100 is adapted to allow data or information to be stored in and/or retrieved from, via a wired or wireless communication system or method, at least one database 116. The interface 112 may allow wired and/or wireless communication between the processing unit 102 and peripheral components that may serve a specialized purpose. In general, the processor 102 can receive instructions as input data 118 via input device 106 and can display processed results or other output to a user by utilizing output device 108. More than one input device 106 and/or output device 108 may be provided. The processing system 100 may be any suitable form of terminal, server, specialized hardware, or the like. The processing system 100 may be a part of a networked communications system.

Processing system 100 can connect to a network, for example, a local area network (LAN), a virtual network such as a virtual local area network (VLAN), a wide area network (WAN), a metropolitan area network (MAN), a worldwide interoperability for microwave access network (WiMAX), a cellular network, the Internet, and/or any other suitable network implemented as a wired and/or wireless network. For instance, when used in a LAN networking environment, the computing system environment 100 is connected to the LAN through a network interface or adapter. When used in a WAN networking environment, the computing system environment typically includes a modem or other system or method for establishing communications over the WAN, such as the Internet. The modem, which may be internal or external, may be connected to a system bus via a user input interface, or via another appropriate mechanism. In a networked environment, program modules depicted relative to the computing system environment 100, or portions thereof, may be stored in a remote memory storage device. It is to be appreciated that the illustrated network connections of Fig. 7 are examples and other systems and methods of establishing a communications link between multiple computers may be used.

Input data 118 and output data 120 may be communicated to other devices via the network. The transfer of information and/or data over the network may be achieved using wired or wireless systems and methods of communication. A server can facilitate the transfer of data between the network and one or more databases. A server and one or more databases provide an example of an information source.

Thus, the processing computing system environment 100 illustrated in Fig. 7A may operate in a networked environment using logical connections to one or more remote computers. The remote computer may be a personal computer, a server, a router, a network PC, a peer device, or other common network node, and typically includes many or all of the elements described above.

FIG. 7B illustrates the processor 102 of FIG. 7A in greater detail. The processor 102 may be configured to execute specific modules. The modules may be, for example, hardware modules, software modules stored in the memory 104 and/or executed in the processor 102, and/or any combination thereof. For example, as shown in FIG. 7B, the processor 102 includes and/or executes a pre-classifier analysis module 130, a classifier training module 132, a classifier analysis module 134 and a report module 136. As shown in FIG. 7B, the pre-classifier analysis module 130, the classifier training module 132, the classifier analysis module 134 and the report module 136 may be connected and/or electrically coupled. As such, signals may be sent between the pre-classifier analysis module 130, the classifier training module 132, the classifier analysis module 134 and the report module 136.

The classifier training module 132 may be configured to receive a corpora of data (e.g. gene expression data, sequencing data) and train a classifier. For example, clinical annotation data from samples previously identified as UIP and non-UIP (e.g., by an expert) may be received by the input device 106 and used by the classifier training module 132 to identify correlations between the samples previously identified as UIP and non-UIP. For example, expert TBB histopathology labels (i.e., UIP or non-UIP), expert HRCT labels, and/or expert patient-level clinical outcome labels may be obtained and used alone or in combination to train the classifier using microarray and/or sequencing data. The feature space used can include gene expression, variants, mutations, fusions, loss of heterozygoxity (LOH), biological pathway effect and/or any other dimension of the data that may be extracted as a feature for the purposes of training a machine-learning algorithm. In some embodiments, the feature space used for training a UIP vs. non-UIP classifier, a smoker vs. non-smoker classifier, or a UIP vs. non-UIP and smoker vs. non-smoker classifier includes gene expression, variants, mutations, fusions, loss of heterozygoxity (LOH), and biological pathway effect. In some embodiments, the feature space used for training a UIP vs. non-UIP classifier, a smoker vs. non-smoker classifier, or a UIP vs. non-UIP and smoker vs. non-smoker classifier includes gene expression and variant dimensions.

In some embodiments, the classifier training module 132 can train a smoker classifier and a non-smoker classifier based on an indication associated with whether a received sample is associated with a smoker or non-smoker. In other embodiments, the smoker/non-smoker may be used as an attribute (a model covariate) to train a single classifier. After the classifier is trained, it may be used to identify and/or classify newly received and unknown samples as described herein.

The pre-classifier analysis module 130 can identify whether a sample is associated with a smoker or a non-smoker. Specifically, the pre-classifier analysis module 130 can use any suitable method to identify and/or classify a sample as coming from an individual that smokes (or has a past history of heavy smoking) versus an individual that does not smoke (or has no smoking history). The classification may be done in any suitable manner such as, receiving an indication from a user, identification of genes that are susceptible to smoker-status bias, using a machine-learning classifier, and/or any other suitable method described herein.

The classifier analysis module 134 can input the sample into the classifier to identify and/or classify the received sample as associated with UIP and non-UIP. Specifically, the classifier analysis module 134 can use a trained classifier to identify whether the sample indicates UIP or non-UIP. In some embodiments, the classifier analysis module 134 can indicate a percentage or confidence score of the sample being associated with UIP or non-UIP. In some embodiments, the classifier analysis module 134 can execute two separate classifiers: one for smoker samples and the other for non-smoker samples (as determined by the pre-classifier analysis module 130). In other embodiments, a single classifier is executed for both smoker and non-smoker samples with an input for smoker status.

The report module 136 may be configured to generate any suitable report based on the outcome of the classifier analysis module 134 as described in further detail herein. In some cases, the report may include, but is not limited to, such information as one or more of the following: the number of genes differentially expressed, the suitability of the original sample, the number of genes showing differential alternative splicing, a diagnosis, a statistical confidence for the diagnosis, the likelihood the subject is a smoker, the likelihood of an ILD, and indicated therapies.

FIG. 7C illustrates a flow chart of one non-limiting embodiment of the present disclosure wherein gene product expression data for known UIP and non-UIP samples are used to train (e.g., using a classifier training module) a classifier for differentiating UIP vs. non-UIP, wherein the classifier in some cases considers smoker status as a covariant, and wherein gene product expression data from unknown samples are input into the trained classifier to identify the unknown samples as either UIP or non-UIP, and wherein the results of the classification via the classifier are defined and output via a report.

Certain embodiments may be described with reference to acts and symbolic representations of operations that are performed by one or more computing devices, such as the computing system environment 100 of Fig. 7A. As such, it will be understood that such acts and operations, which are at times referred to as being computer-executed, include the manipulation by the processor of the computer of electrical signals representing data in a structured form. This manipulation transforms the data or maintains them at locations in the memory system of the computer, which reconfigures or otherwise alters the operation of the computer in a manner understood by those skilled in the art. The data structures in which data is maintained are physical locations of the memory that have particular properties defined by the format of the data. However, while an embodiment is being described in the foregoing context, it is not meant to be limiting as those of skill in the art will appreciate that the acts and operations described hereinafter may also be implemented in hardware.

Embodiments may be implemented with numerous other general-purpose or special-purpose computing devices and computing system environments or configurations. Examples of other computing systems, environments, and configurations that may be suitable for use with an embodiment include, but are not limited to, personal computers, handheld or laptop devices, personal digital assistants, multiprocessor systems, microprocessor-based systems, programmable consumer electronics, network, minicomputers, server computers, web server computers, mainframe computers, and distributed computing environments that include any of the above systems or devices.

Embodiments may be described in a general context of computer-executable instructions, such as hardware and/or software modules. An embodiment may also be practiced in a distributed computing environment where tasks are performed by remote processing devices that are linked through a communications network. In a distributed computing environment, program modules may be located in both local and remote computer storage media including memory storage devices.

### Computer program products

The present disclosure provides computer program products that, when executed on a programmable computer such as that described above with reference to Fig. 7, can carry out the methods of the present disclosure. As discussed above, the subject matter described herein may be embodied in systems, apparatus, methods, and/or articles depending on the desired configuration. These various implementations may include implementation in one or more computer programs that are executable and/or interpretable on a programmable system including at least one programmable processor, which may be special or general purpose, coupled to receive data and instructions from, and to transmit data and instructions to, a storage system, at least one input device (e.g. video camera, microphone, joystick, keyboard, and/or mouse), and at least one output device (e.g. display monitor, printer, etc.).

Computer programs (also known as programs, software, software applications, applications, components, or code) include instructions for a programmable processor, and may be implemented in a high-level procedural and/or object-oriented programming language, and/or in assembly/machine language. As used herein, the term "machine-readable medium" refers to any computer program product, apparatus and/or device (e.g., magnetic discs, optical disks, memory, etc.) used to provide machine instructions and/or data to a programmable processor, including a machine-readable medium that receives machine instructions as a machine-readable signal.

It will be apparent from this description that aspects of the present disclosure may be embodied, at least in part, in software, hardware, firmware, or any combination thereof. Thus, the techniques described herein are not limited to any specific combination of hardware circuitry and/or software, or to any particular source for the instructions executed by a computer or other data processing system. Rather, these techniques may be carried out in a computer system or other data processing system in response to one or more processors, such as a microprocessor, executing sequences of instructions stored in memory or other computer-readable medium including any type of ROM, RAM, cache memory, network memory, floppy disks, hard drive disk (HDD), solid-state devices (SSD), optical disk, CD-ROM, and magnetic -optical disk, EPROMs, EEPROMs, flash memory, or any other type of media suitable for storing instructions in electronic format.

In addition, the processor(s) may be, or may include, one or more programmable general-purpose or special-purpose microprocessors, digital signal processors (DSPs), programmable controllers, application specific integrated circuits (ASICs), programmable logic devices (PLDs), trusted platform modules (TPMs), or the like, or a combination of such devices. In alternative embodiments, special- purpose hardware such as logic circuits or other hardwired circuitry may be used in combination with software instructions to implement the techniques described herein.

### Arrays and Kits

The present disclosure provides arrays and kits for use in carrying out a subject evaluating method or a subject diagnostic method.

### Arrays

A subject array can comprise a plurality of nucleic acids, each of which hybridizes to a gene differentially expressed in a cell present in a tissue sample obtained from an individual being tested for UIP, non-UIP, IPF, or an ILD.

A subject array can comprise a plurality of nucleic acids, each of which hybridizes to a gene differentially expressed in a cell present in a tissue sample obtained from an individual being tested for smoker status.

A subject array can comprise a plurality of nucleic acids, each of which hybridizes to a gene differentially expressed in a cell present in a tissue sample obtained from an individual being tested for both smoker status and UIP, non-UIP, IPF, or an ILD.

A subject array can comprise a plurality of member nucleic acids, each of which member nucleic acids hybridizes to a different gene product. In some cases, two or more member nucleic acids hybridize to the same gene product; e.g., in some cases 2, 3, 4, 5, 6, 7, 8, 9, 10, or more member nucleic acids hybridize to the same gene product. A member nucleic acid can have a length of from about 5 nucleotides (nt) to about 100 nt, e.g., 5, 6, 7, 8, 9, 10, 11, 12, 13, 14, 15, 16, 17 18, 19, 20, 20-25, 25-30, 30-40, 40-50, 50-60, 60-70, 70- 80, 80-90, or 90-100 nt. A nucleic acid can have one or more phosphate backbone modifications.

A subject array can include from about 10 to about 10⁵ unique member nucleic acids, or more than 10⁵ unique member nucleic acids. For example, a subject array can include from about 10 to about 10², from about 10² to about 10³, from about 10³ to about 10⁴, from about 10⁴ to about 10⁵, or more than 10⁵, unique member nucleic acids.

### Kits

A kit of the present disclosure can include an array, as described above; and a reagent for analyzing an expression level of a gene product.

Reagents for analyzing an expression level of a nucleic acid gene product include, e.g., reagents suitable for sequencing a nucleic acid; reagents suitable for amplifying a nucleic acid; and reagents suitable for nucleic acid hybridization.

The kit may include: a buffer; a detectable label; components for developing a detectable label (e.g., where a nucleic acid probe includes a detectable label); etc. The various components of the kit may be present in separate containers or certain compatible components may be pre-combined into a single container, as desired.

In addition to above-mentioned components, a subject kit can include instructions for using the components of the kit to practice a subject method. The instructions for practicing a subject method are generally recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or subpackaging) etc. In other embodiments, the instructions are present as an electronic storage data file present on a suitable computer readable storage medium, e.g. compact disc-read only memory (CD-ROM), digital versatile disk (DVD), diskette, etc. In yet other embodiments, the actual instructions are not present in the kit, but methods for obtaining the instructions from a remote source, e.g. via the internet, are provided. An example of this embodiment is a kit that includes a web address where the instructions can be viewed and/or from which the instructions can be downloaded. As with the instructions, this method for obtaining the instructions is recorded on a suitable substrate.

### Abbreviations

| | |
|---|---|
| adj.P.Value.edgeR: | False discovery rate adjusted p value of RNAseq gene expression data using edgeR analysis. |
| adj.P.Value.microarray | False discovery rate adjusted p value of RNAseq gene expression data using microarray analysis |
| adj.P.Value.npSeq: | False discovery rate adjusted p value of RNAseq gene expression data using npSeq analysis |
| BRONCH: | Broncholitis |
| CIF-NOC | Chronic Interstitial Fibrosis Not Otherwise Classified |
| edgeR: | an R package for the significance analysis of sequencing data |
| Ensembl ID: | Gene Identifier from Ensembl Genome Browser database |
| FDR: | False Discovery Rate, an adjusted p value that limits the possibility that the results are random due to the large number of genes simultaneously evaluated. |
| Gene Symbol: | Gene Identifier from HUGO Gene Nomenclature Committee |
| logFC.edgeR: | Log2 fold change of RNAseq gene expression data using edgeR analysis |
| logFC.microarray: | Log2 fold change of RNAseq gene expression data using LIMMA microarray analysis |
| logFC.npSeq: | Log2 fold change of RNAseq gene expression data using npSeq analysis |
| microarray: | Gene expression analysis using gene arrays such as from Affymetrix. |
| NML: | Normal Lung, usually obtained from human lung donor tissue that was ultimately never transplanted |
| npSeq: | an R package for the significance analysis of sequencing data |
| NSIP: | Non Specific Interstitial Pneumonia |
| OP: | Organizing Pneumonia |
| P.value.edgeR: | p value of RNAseq gene expression data using edgeR analysis |
| P.value.microarray: | p value of RNAseq gene expression data using LIMMA microarray analysis |
| P.value.npSeqp: | value of RNAseq gene expression data using npSeq analysis |
| RB: | Respiratory Broncholitis |
| REST: | A combination of all other ILDs except the subtype it is being compared to. Usually HP and NSIP, BRONCH, CIF-NOC, OP, RB and SARC. |
| SARC: | Sarcoidosis |
| SQC: | Squamous Cell Carcinoma |
| TCID: | "TCID" or "Transcript Cluster Identifier" refers to a gene level identifier used by all Affymetrix microarrays. Each TCID is associated with a fixed reference number that identifies a set of specific probes having sequences for a specific gene. Such specific probes are present on a given array commercially available from Affymetrix. TCID numbers thus refer to a gene product(s) of a specific gene, and may be found, e.g., at the following world wide web address: affymetrix.com/ the sequences of which probes and gene products are hereby incorporation herein in their entirety. |
| UIP: | Usual Interstitial Pneumonia; the HRCT or histopathology pattern observed in IPF |
| LIMMA: | Linear Models for Microarray Data; an R package for the significance analysis of microarray data. |

"ENSEMBL ID" refers to a gene identifier number from the Ensembl Genome Browser database (see World Wide Web address: ensembl.org/index.html, which is entirely incorporated herein by reference). Each identifier begins with the letters ENSG to denote "Ensembl Gene". Each ENSEMBL ID number (i.e., each "gene" in the Ensembl database) refers to a gene defined by a specific start and stop position on a particular human chromosome, and therefore defines a specific locus of the human genome. As one of skill in the art may fully appreciate, all of the gene symbols disclosed herein refer to gene sequences, which are readily available on publically available databases, e.g., UniGene database (Pontius JU, Wagner L, Schuler GD. UniGene: a unified view of the transcriptome. In: The NCBI Handbook. Bethesda (MD): National Center for Biotechnology Information; 2003, available at the World Wide Web address ncbi.nlm.nih.gov/unigene, incorporated herein), RefSeq (The NCBI handbook [Internet]. Bethesda (MD): National Library of Medicine (US), National Center for Biotechnology Information; 2002 Oct. Chapter 18, The Reference Sequence (RefSeq) Project, available at the World Wide Web address: ncbi.nlm.nih.gov/refseq/, incorporate herein), Ensembl (EMBL, available at the world wide web address: ensembl.org/index.html, incorporated herein), and the like. The sequences of the genes disclosed herein via their gene symbols, Ensembl IDs, and Entrez IDs are herein incorporated in their entirety.

All references, patents, and patent applications cited herein are incorporated in their entirety for all purposes.

### EXAMPLES

The diagnostic approach to ILD remains quite challenging given the complexity of diffuse parenchymal disorders. Diagnostic approaches have emphasized multidisciplinary evaluation of clinical, radiological, and pathological data. The latter has traditionally emphasized SLB to maximize the yield in sampling lung tissue. The development of molecular markers that could serve as a diagnostic surrogate is of interest. In order to be clinically useful in the diagnosis of ILD, a surrogate test for pathology needs to distinguish UIP from among similar but pathologically distinct disease processes.

We hypothesized that a genomic classifier can detect a UIP gene expression signature in TBBs with high accuracy in a diverse patient population. In the following examples, we used machine learning on exome enriched transcriptional data to train a classifier to differentiate UIP from among the wide variety of ILDs encountered in clinical practice. We then demonstrated that this classifier accurately predicts the presence of UIP in an independent multi-center validation cohort. Further, we surprisingly demonstrate that sample pooling enables improved sensitivity and specificity for diagnosis, and classifier performance is agnostic to cellular heterogeneity. This was surprising because prior studies had indicated that that the cell of interest in IPF is the alveolar cell; thus it may be expected that all the biology is contained within alveolar cells. However, our results demonstrate that signals outside the alveolar cells are sufficient to inform on IPF classification, and this has not been previously described.

Thus, the genomic classifiers disclosed herein may reduce the need for surgical lung biopsy in the diagnosis of ILD, and may eventually be used to inform the diagnosis and treatment of patients with IPF.

The following items are also disclosed.
1. A method of detecting whether a lung tissue sample is positive for usual interstitial pneumonia (UIP) or non-usual interstitial pneumonia (non-UIP), comprising:
   (a) assaying the expression level of each of a first group of transcripts and a second group of transcripts in a test sample of a subject, wherein the first group of transcripts includes one or more sequences corresponding to any one of the genes overexpressed in UIP and listed in Table 1 and/or Table 15 and the second group of transcripts includes one or more sequences corresponding to any one of the genes under-expressed in UIP and listed in Table 1 and/or Table 15; and
   (b) comparing the expression level of each of the first group of transcripts and the second group of transcripts with reference expression levels of the corresponding transcripts to (1) classify said lung tissue as usual interstitial pneumonia (UIP) if there is (i) an increase in an expression level corresponding to the first group and/or (ii) a decrease in an expression level corresponding to the second group as compared to the reference expression levels, or (2) classify the lung tissue as non-usual interstitial pneumonia (non-UIP) if there is (i) an increase in the expression level corresponding to the second group and/or (ii) a decrease in the expression level corresponding to the first group as compared to the reference expression levels.
2. A method of detecting whether a lung tissue sample is positive for usual interstitial pneumonia (UIP) or non-usual interstitial pneumonia (non-UIP), comprising:
   (a) assaying by sequencing, array hybridization, or nucleic acid amplification the expression level of each of a first group of transcripts and a second group of transcripts in a test sample from a lung tissue of a subject, wherein the first group of transcripts includes one or more sequences corresponding to any one of the genes overexpressed in UIP and listed in Table 1 and/or Table 15 and the second group of transcripts includes one or more sequences corresponding to any one of the genes under-expressed in UIP and listed in any of Table 1 and/or Table 15; and
   (b) comparing the expression level of each of the first group of transcripts and the second group of transcripts with reference expression levels of the corresponding transcripts to (1) classify said lung tissue as usual interstitial pneumonia (UIP) if there is (i) an increase in an expression level corresponding to the first group and/or (ii) a decrease in an expression level corresponding to the second group as compared to the reference expression levels, or (2) classify the lung tissue as non-usual interstitial pneumonia (non-UIP) if there is (i) an increase in the expression level corresponding to the second group and/or (ii) a decrease in the expression level corresponding to the first group as compared to the reference expression levels.
3. A method of detecting whether a lung tissue sample is positive for UIP or non-UIP, comprising:
   (a) assaying the expression level of two or more transcripts expressed in a test sample; and
   (b) using a computer generated classifier to classify the sample as UIP or non-UIP;

   wherein the classifier was trained using a heterogeneous spectrum of non-UIP pathology subtypes comprising HP, NSIP, sarcoidosis, RB, bronchiolitis, and organizing pneumonia (OP); and
   wherein the two or more transcripts expressed in the test sample are selected from transcripts of two or more genes listed in Table 1 and/or Table 15, or any two or more of SEQ ID NOs: 1-320.
4. The method of any one of the preceding items, wherein the test sample is a pool of a plurality of samples obtained from the subject.
5. The method of any one of items 1-3, wherein the method comprises pooling the expression level data from a plurality of individual samples obtained from the subject.
6. The method of any one of items 1-3, comprising synthesizing double-stranded cDNA from the cDNA prior to assaying the expression level.
7. The method of any one of items 1-3, comprising synthesizing non-natural RNA from the double stranded cDNA prior to assaying the expression level.
8. The method of any one of items 1-2, further comprising using smoking status as a covariate to the classification step of (1) or (2).
9. The method of item 8, wherein smoking status is determined by detecting an expression profile indicative of the subject's smoker status.
10. The method of any one of the preceding items, wherein classification of the sample comprises detection of the expression levels of one or more transcripts that are susceptible to smoker status bias, and wherein the transcripts that are susceptible to smoker status bias are weighted differently than transcripts that are not susceptible to smoker bias.
11. The method of any one of the preceding items, wherein classification of the sample comprises detection of the expression levels of one or more transcripts that are susceptible to smoker status bias, and wherein the transcripts that are susceptible to smoker status bias are excluded from the classification step.
12. The method of any one of the preceding items, wherein the classification step further comprises detecting sequence variants in the test sample and comparing the sequence variants to the respective sequences in a reference sample to classify the sample as UIP or non-UIP.
13. The method of any one of the preceding items, wherein the expression data used to classify the sample as UIP or non-UIP comprises expression data for at least two transcripts of genes selected from SEQ ID NOs: 1-320.
14. The method of any one of items 1-3, further comprising (i) obtaining a sample from the subject, (ii) subjecting a first portion of the sample to cytological analysis that indicates that the first portion of the sample is ambiguous or indeterminate, and (iii) assaying a second portion of the sample as the test sample.
15. The method of item 14, wherein the first portion and the second portion are different portions.
16. The method of item 14, wherein the first portion and the second portion are the same portions.
17. The method of any one of items 1 or 2, wherein (b) is performed using a trained algorithm that is trained with a plurality of samples, wherein said test sample is independent of said plurality of samples.
18. A method of treating a subject with undiagnosed idiopathic pulmonary fibrosis (IPF) comprising,
   (a) measuring by array, sequencing, or qRT-PCR the level of expression of at least two genes in one or more samples obtained from a subject's airway, wherein the genes are selected from those listed in Table 1 and/or Table 15, and wherein the method comprises:
      (i) pooling at least two samples prior to the measuring step;
      (ii) pooling at least two sets of expression data independently measured from two separate samples; or
      (iii) a combination of (i) and (ii);
   (b) administering a compound effective for treating IPF if:
      (i) the expression level of each of the at least two genes is increased as compared to reference expression levels of the corresponding transcripts; and/or
      (ii) the expression level of each of the at least two genes is decreased as compared to reference expression levels of the corresponding transcripts; and/or
      (iii) the expression level of at least one of the at least two genes increased as compared to reference expression levels of the corresponding transcripts and at least one of the at least two genes is decreased as compared to reference expression levels of the corresponding transcripts.
19. A method of detecting whether a pooled lung tissue test sample is positive for UIP or non-UIP, comprising:
   (a) assaying the expression level of one or more transcripts expressed in a test sample; and
   (b) classifying the test sample as UIP or non-UIP using a computer generated trained classifier;
   wherein the computer generated trained classifier is trained using expression levels of one or more transcripts expressed in a plurality of individual training samples obtained from a plurality of subjects, each training sample having a confirmed diagnoses of UIP or non-UIP, wherein at least two of said training samples were obtained from a single subject; and wherein the test sample is pooled prior to the classifying.
20. The method of item 19, wherein the classifier training uses expression levels of one or more transcripts listed in Table 1 and/or Table 15.
21. The method of item 19, wherein the classifier training uses expression levels of transcripts of all of the genes listed in Table 1 and/or Table 15.
22. The method of any one of items 19-21, wherein the computer generated trained classifier classifies the test sample as UIP or non-UIP based upon the expression level of one or more transcripts of genes listed in Table 1 and/or Table 15.
23. A method of detecting whether a pooled lung tissue test sample is positive for a disease or condition comprising:
   (a) assaying the expression level of one or more transcripts expressed in a test sample; and
   (b) classifying the test sample as either positive for, or negative for, the disease or condition using a computer generated trained classifier;
   wherein the computer generated trained classifier is trained using expression levels of one or more transcripts expressed in a plurality of individual training samples obtained from a plurality of subjects, each training sample having a confirmed diagnoses of positive or negative for the disease or condition, wherein at least two of said training samples were obtained from a single subject; and wherein the test sample is pooled prior to the classifying.
24. The method of item 23, wherein the disease or condition is selected from: a lung disorder, lung cancer, interstitial lung disease (ILD), idiopathic pulmonary fibrosis (IPF), nonspecific interstitial pneumonia (NSIP), Favor NSIP, usual interstitial pneumonia (UIP) or non-usual interstitial pneumonia (non-UIP), acute lung injury, bronchiolitis, desquamative interstitial pneumonia, diffuse alveolar damage, emphysema, eosinophilic pneumonia, nonspecific interstitial pneumonia (including subtypes of cellular, mixed, or Favor), granulomatous disease, hypersensitivity pneumonitis (HP), Favor subtype hypersensitivity pneumonitis (Favor HP), organizing pneumonia, pneumocystis pneumonia, pulmonary hypertension, respiratory bronchiolitis, pulmonary sarcoidosis, smoking-related interstitial fibrosis, chronic obstructive pulmonary disease (COPD), a history of exposure to smoke, long-term exposure to smoke, short-term exposure to smoke, and chronic interstitial fibrosis.
25. A method of treating a subject in need thereof with a therapeutic effective for treating idiopathic pulmonary fibrosis (IPF) comprising,
   administering an effective dose of a compound effective for treating IPF to the subject in need thereof; wherein the subject in need thereof has an expression level of one or more genes in Table 1 and/or Table 15 that indicates the subject is in need of treatment for IPF as determined by a computer generated trained classifier.
26. The method of item 25, wherein the computer-generated trained classifier was trained using expression levels of one or more transcripts expressed in a plurality of individual training samples obtained from a plurality of subjects, each training sample having a confirmed diagnoses of UIP or non-UIP, wherein at least two of said training samples were obtained from a single subject; and wherein the test sample is pooled prior to the classifying.
27. The method of item 25, wherein the computer-generated trained classifier identified a sample obtained from the subject as UIP.
28. The method of item 25, wherein the computer-generated trained classifier identified a sample obtained from the subject as IPF.
29. A method of detecting whether a lung tissue sample is positive for usual interstitial pneumonia (UIP) or non-usual interstitial pneumonia (non-UIP), comprising:
   (a) assaying the expression level of each of a first group of transcripts and a second group of transcripts in a test sample of a subject, wherein the first group of transcripts includes one or more sequences corresponding to any one of the genes overexpressed in UIP and listed in Table 5 and the second group of transcripts includes one or more sequences corresponding to any one of the genes under-expressed in UIP and listed in Table 5; and
   (b) comparing the expression level of each of the first group of transcripts and the second group of transcripts with reference expression levels of the corresponding transcripts to (1) classify said lung tissue as usual interstitial pneumonia (UIP) if there is (i) an increase in an expression level corresponding to the first group and/or (ii) a decrease in an expression level corresponding to the second group as compared to the reference expression levels, or (2) classify the lung tissue as non-usual interstitial pneumonia (non-UIP) if there is (i) an increase in the expression level corresponding to the second group and/or (ii) a decrease in the expression level corresponding to the first group as compared to the reference expression levels.
30. A method of detecting whether a lung tissue sample is positive for usual interstitial pneumonia (UIP) or non-usual interstitial pneumonia (non-UIP), comprising:
   (a) assaying by sequencing, array hybridization, or nucleic acid amplification the expression level of each of a first group of transcripts and a second group of transcripts in a test sample from a lung tissue of a subject, wherein the first group of transcripts includes one or more sequences corresponding to any one of the genes overexpressed in UIP and listed in Table 5 and the second group of transcripts includes one or more sequences corresponding to any one of the genes under-expressed in UIP and listed in Table 5; and
   (b) comparing the expression level of each of the first group of transcripts and the second group of transcripts with reference expression levels of the corresponding transcripts to (1) classify said lung tissue as usual interstitial pneumonia (UIP) if
   there is (i) an increase in an expression level corresponding to the first group and/or (ii) a decrease in an expression level corresponding to the second group as compared to the reference expression levels, or (2) classify the lung tissue as non-usual interstitial pneumonia (non-UIP) if there is (i) an increase in the expression level corresponding to the second group and/or (ii) a decrease in the expression level corresponding to the first group as compared to the reference expression levels.
31. A method of detecting whether a lung tissue sample is positive for UIP or non-UIP, comprising:
   (a) assaying the expression level of two or more genes expressed in a test sample; and
   (b) using a computer generated classifier to classify the sample as UIP or non-UIP; wherein the classifier was trained using a heterogeneous spectrum of non-UIP pathology subtypes comprising HP, NSIP, sarcoidosis, RB, bronchiolitis, and organizing pneumonia (OP); and
   wherein the two or more genes expressed in the test sample are selected from any two or more genes listed in Table 5.
32. The method of any one of items 29-31, wherein the test sample is a pool of a plurality of samples obtained from the subject.
33. The method of any one of items 29-31, wherein the method comprises pooling the expression level data from a plurality of individual samples obtained from the subject.
34. The method of any one of items 29-33, wherein the test sample is a biopsy sample or a bronchoalveolar lavage sample.
35. The method of any one of items 29-34, wherein the biopsy sample is a transbronchial biopsy sample.
36. The method of any one of the preceding items, wherein assaying the expression level is accomplished using qRT-PCR, DNA microarray hybridization, RNAseq, or a combination thereof.
37. The method of any one of items 29-36, comprising synthesizing cDNA from RNA expressed in the test sample prior to assaying the expression level.
38. The method of item 37, comprising synthesizing double-stranded cDNA from the cDNA prior to assaying the expression level.
39. The method of item 38, comprising synthesizing non-natural RNA from the double-stranded cDNA prior to assaying the expression level.
40. The method of any one of items 29-36, comprising amplification of the nucleotide prior to assaying the expression level.
41. The method of any one of items 29-36, wherein one or more of the transcripts are labeled.
42. The method of any one of items 29-32, further comprising measuring the expression level of at least one control nucleic acid in the test sample.
43. The method of any one of items 29-32, wherein the lung tissue is classified as any one of interstitial lung diseases (ILD), a particular type of ILD, a non-ILD, or non-diagnostic.
44. The method of any one of items 29-30, further comprising using smoking status as a covariate to the classification step of (1) or (2).
45. The method of item 44, wherein smoking status is determined by detecting an expression profile indicative of the subject's smoker status.
46. The method of item 3 or 31, further comprising using smoking status as a covariate to the classification step.
47. The method of any one of items 9, 44, or 46, wherein the method uses smoking status as a covariate prior to the classification step.
48. The method of any one of the preceding items, comprising implementing a classifier trained using one or more features selected from gene expression, variants, mutations, fusions, loss of heterozygoxity (LOH), and biological pathway effect.
49. The method of any one of the preceding items, wherein the classifying results in a specificity of at least about 90% and a sensitivity of at least about 70%.
50. The method of any one of the preceding items, wherein the expression data used to classify the sample as UIP or non-UIP comprises expression data for at least two transcripts corresponding to genes selected from the genes listed in Table 5.
51. The method of item 50, wherein the expression data used comprises each of the genes listed in Table 5.
52. A method of treating a subject with undiagnosed idiopathic pulmonary fibrosis (IPF) comprising,
   (a) measuring by array, sequencing, or qRT-PCR the level of expression of at least two genes in one or more samples obtained from a subject's airway, wherein the genes are selected from those listed in Table 5, and wherein the method comprises:
      (i) physical pooling at least two samples prior to the measuring step;
      (ii) pooling at least two sets of expression data independently measured from two separate samples; or
      (iii) a combination of (i) and (ii);
   (b) administering a compound effective for treating IPF if:
      (i) the expression level of each of the at least two genes is increased as compared to reference expression levels of the corresponding transcripts; and/or
      (ii) the expression level of each of the at least two genes is decreased as compared to reference expression levels of the corresponding transcripts; and/or
      (iii) the expression level of at least one of the at least two genes increased as compared to reference expression levels of the corresponding transcripts and at least one of the at least two genes is decreased as compared to reference expression levels of the corresponding transcripts.
53. The method of item 52, wherein the administering step is performed only if the increase in (i) and/or the decrease in (ii) is significant.
54. A method of detecting whether a pooled lung tissue test sample is positive for UIP or non-UIP, comprising:
   (a) assaying the expression level of one or more transcripts expressed in a test sample; and
   (b) classifying the test sample as UIP or non-UIP using a computer generated trained classifier;
   wherein the computer generated trained classifier is trained using expression levels of one or more transcripts expressed in a plurality of individual training samples obtained from a plurality of subjects, each training sample having a confirmed diagnoses of UIP or non-UIP, wherein at least two of said training samples were obtained from a single subject; and wherein the test sample is pooled prior to the classifying.
55. The method of any item 54, wherein the classifier training uses expression levels of one or more genes listed in Table 5.
56. The method of any one of items 54-55, wherein the classifier training uses expression levels of all of the genes listed in Table 5.
57. The method of any one of items 54-56, wherein the computer-generated trained classifier classifies the test sample as UIP or non-UIP based upon the expression level of one or more genes listed in Table 5.
58. The method of item 57, wherein the classifier classifies the test sample as UIP or non-UIP based upon the expression level of transcripts of all of the genes listed in Table 5.
59. A method of detecting whether a pooled lung tissue test sample is positive for a disease or condition comprising:
   (a) assaying the expression level of one or more transcripts expressed in a test sample; and
   (b) classifying the test sample as either positive for, or negative for, the disease or condition using a computer-generated trained classifier;
   wherein the computer-generated trained classifier is trained using expression levels of one or more transcripts expressed in a plurality of individual training samples obtained from a plurality of subjects, each training sample having a confirmed diagnoses of positive or negative for the disease or condition, wherein at least two of said training samples were obtained from a single subject; and wherein the test sample is pooled prior to the classifying.
60. A method of treating a subject in need thereof with a therapeutic effective for treating idiopathic pulmonary fibrosis (IPF) comprising,
   administering an effective dose of a compound effective for treating IPF to the subject in need thereof; wherein the subject in need thereof has an expression level of one or more genes in Table 5 that indicates the subject is in need of treatment for IPF as determined by a computer-generated trained classifier.
61. The method of item 60, wherein the computer-generated trained classifier was trained using expression levels of one or more transcripts expressed in a plurality of individual training samples obtained from a plurality of subjects, each training sample having a confirmed diagnoses of UIP or non-UIP, wherein at least two of said training samples were obtained from a single subject; and wherein the test sample is pooled prior to the classifying.
62. The method of item 61, wherein the computer-generated trained classifier identified a sample obtained from the subject as UIP.
63. The method of item 61, wherein the computer-generated trained classifier identified a sample obtained from the subject as IPF.
64. A method for identifying whether a subject is positive for a lung disorder, comprising:
   (a) obtaining a tissue sample of said subject;
   (b) subjecting a first portion of said tissue sample to cytological testing that indicates that said first portion is ambiguous or suspicious;
   (c) upon identifying that said first portion is ambiguous or suspicious, assaying a second portion of said tissue sample for an expression level of one or more markers associated with said lung disorder;
   (d) processing said expression level with a trained algorithm to generate a classification of said tissue sample as being positive for said lung disorder at an accuracy of at least about 90%, wherein said trained algorithm is trained with a training set comprising a plurality of training samples, and wherein said tissue sample is independent of said plurality of training samples; and
   (e) electronically outputting said classification, thereby identifying whether said subject is positive for said lung disorder.
65. The method of item 64, wherein said tissue sample is a lung tissue sample.
66. The method of item 64, wherein said tissue sample is a non-lung tissue sample.
67. The method of item 66, wherein said non-lung tissue sample is a respiratory epithelium sample.
68. The method of item 67, wherein said respiratory epithelium sample is from a nose or mouth of said subject.
69. The method of item 64, wherein said expression level is of a plurality of markers associated with UIP.
70. The method of item 64, wherein said accuracy is at least about 95%.
71. The method of item 64, wherein said classification is generated at a specificity of at least about 90%.
72. The method of item 64, wherein said classification is generated at a sensitivity of at least about 70%.
73. The method of item 64, wherein said trained algorithm is configured to classify a tissue sample at an accuracy of at least about 90% across at least 100 independent test samples.
74. The method of item 64, wherein said classification is electronically outputted on a graphical user interface of an electronic display of a user.
75. The method of item 64, wherein said lung disorder is usual interstitial pneumonia (UIP) or non-usual interstitial pneumonia (non-UIP).
76. The method of item 64, wherein the first portion is different than the second portion.

### EXAMPLE 1

### Sample Collection, Pathology Diagnosis, and Labeling

Video-assisted thoracoscopic surgery (VATS) specimens were prospectively collected as a part of an Institutional Review Board (IRB) approved ongoing multi-center clinical protocol, BRonchial sAmple collection for a noVel gEnomic test (BRAVE), sponsored by Veracyte, Inc. (South San Francisco, CA). Additional VATS and surgical lung biopsy specimens were obtained from banked sources. High resolution computed tomography (HRCT) scans collected during usual clinical care were reviewed by an expert radiologist, when available. Radiology diagnoses were summarized according to ATS guidelines (Raghu G, et al., Am J Respir Crit Care Med 2011,183:788-824, incorporated herein by reference in its entirety). Pathological diagnoses were determined by expert pathologists (A-LK, TC, JM, and SG) according to a centralized review process.

Following surgery, histology slides were prepared by study sites from surgical lung biopsies (SLB), bronchoscopic lung cryobiopsies (BLC) or transbronchial biopsies (TBB), de-identified, and submitted to two pathologists for blinded, independent expert pathology review. Selected slides were scanned to construct a permanent digital file of microscopic images (Aperio, Vista, CA). Slides were evaluated according to the according to a centralized review process described in Kim SY, et al., The Lancet Respiratory Medicine 2015;3:473-482, incorporated herein by reference in its entirety.

Each pathologist determined diagnoses for the patient as a whole (patient level) and for the specific lung lobe(s) sampled for pathology (sample or lobe level). Diagnoses were evaluated, with agreement defined as subtype concordance. In the event of agreement, a categorical UIP or non-UIP 'truth' label was defined, otherwise blinded review by a third pathologist was used to achieve 2 of 3 ('tiebreaker') consensus. In the absence of agreement, an unblinded conferral process was used. This process is also described in Figure 1, resulting in both sample-level and patient-level pathology diagnoses.

Truth labels for algorithm training and development were assigned to TBBs using pathology diagnoses made on surgical lung biopsy (SLB) from the same lung lobe. Pathology subtypes were translated to sample and patient labels of UIP or non-UIP for use in algorithm training and validation as described in *Kim SY et al., supra,* with the exception that three patients with UIP pattern detected in a lower lobe, but non-UIP or non-diagnostic labels assigned in the upper lobe, were assigned UIP labels at the patient level (Table 14).

Up to 5 TBB samples (two upper lobe, three lower lobe) were collected from each patient for molecular testing. Sampling was performed at the discretion of the treating physician, with guidance to obtain visible tissue from areas adjacent to pathology sampling. Labels of UIP or non-UIP were assigned to TBB samples at lobe level resolution for algorithm training and sample scoring. A patient can have more than one sample-level diagnosis (i.e. one per VATS sample per patient, most often one from each of the lower and upper lobes of the right lung), but can only have one patient-level diagnosis. For mixtures (see Example 6), truth labels were inferred from sample labels so that all patients in training could be scored.

In total, 283 TBB samples from 84 patients were collected at 17 clinical sites and utilized in the studies reported herein. The following pathology diagnoses were defined as non-UIP for purposes of algorithm training and scoring: acute lung injury, bronchiolitis, desquamative interstitial pneumonia, diffuse alveolar damage, emphysema, eosinophilic pneumonia, nonspecific interstitial pneumonia (NSIP) (including subtypes of cellular, mixed, or Favor), granulomatous disease, hypersensitivity pneumonitis (including Favor subtype), organizing pneumonia, pneumocystis pneumonia, pulmonary hypertension, respiratory bronchiolitis, sarcoidosis, and smoking-related interstitial fibrosis.

UIP, for purposes of algorithm training and scoring, was defined as any UIP subtype (classic UIP, difficult UIP, Favor UIP, or UIP).

Diagnostic concordance was defined as subtype agreement for non-UIP pathologies or any UIP subtype for UIP. In the event of subtype disagreement (e.g. Favor HP and HP, Favor NSIP and NISP), consensus diagnoses (e.g., HP and NSIP, respectively) were accepted after consultation. Diagnoses of chronic interstitial fibrosis, not otherwise classified, non-diagnostic, or 'Other' were not assigned training labels and were excluded from training.

As mentioned above, mixtures from patients with concordant UIP or non-UIP diagnoses across lung lobes were assigned UIP or non-UIP labels for mixture scoring. Three patients with a lower-lobe UIP pattern, but a non-UIP or non-diagnostic label in their upper lobe, were assigned UIP labels for mixture scoring purposes.

Most diagnostic terminologies follow American Thoracic Society (ATS) 2011 or 2013 guidelines^{5,6} but a few changes were made by the expert pathologist panel to better characterize features at the lobe level. In particular, `Classic UIP' and 'Difficult UIP' were included instead of `Definite UIP' and `Probable UIP' as described in the ATS 2011 guidelines. Chronic interstitial fibrosis, not otherwise classified (CIF/NOC) corresponds to unclassifiable fibrotic ILD. Three subcategories of CIF/NOC, `Favor UIP', `Favor NSIP', and `Favor HP', were defined to specify cases of unclassifiable fibrosis which, in the judgment of the expert pathology panel, exhibit features suggestive of UIP, non-specific interstitial pneumonia (NSIP), or hypersensitivity pneumonitis (HP). A diagnosis of smoking-related interstitial fibrosis (SRIF) is also included²⁰.

For classification, sample-level pathology diagnoses were converted into binary class labels (UIP and non-UIP). Among the pathology diagnosis categories, the 'UIP' class includes (1) UIP, (2) Classic UIP, (3) Difficult UIP, and (4) Favor UIP. All other pathology diagnoses except non-diagnostic (ND) were assigned to the 'non-UIP' class.

### EXAMPLE 2

### Sample Processing

Pre- or intra-operative transbronchial biopsy specimens were collected from patients for molecular testing, packaged and transported at 4°C in a nucleic acid preservative, and stored long-term in Veracyte facilities at -80°C until processing. Briefly, frozen tissue samples were mounted for sectioning using Tissue-Tek O.C.T. medium (Sakura Finetek U.S.A.) and 2 × 20µm sections generated using a CM1800 cryostat (Leica Biosystems, Buffalo Grove, Illinois). Tissue curls were immediately immersed in RNAprotect (QIAGEN, Valencia, California), incubated overnight at 4°C and stored at -80°C until extraction. Whenever possible, adjacent 5µm tissue curls were mounted onto glass slides and processed for hematoxylin and eosin (H&E) staining following standard procedures.

Nucleic acids were extracted from preserved TBB samples using a modified AllPrep^{™} Micro Kit (QIAGEN, Valencia, CA) procedure. Briefly, TBB tissues were thoroughly disrupted and homogenized using a TissueLyzer^{™} and QIAshredder^{™} prior to column-based isolation of DNA and RNA fractions per manufacturer's instructions (QIAGEN). Total RNA sample quantity and quality was determined using QuantiFluor^{™} RNA System (Promega, Madison, WI) and Agilent RNA 6000 Pico assay (Agilent Technologies, Santa Clara, CA), respectively. We also obtained total RNAs derived from human brain, heart, lung, placenta, and testes (Life Technologies, Carlsbad, CA), thyroid and lung tumors (Takara Bio USA, Mountain View, CA) (Asterand USA; Cooperative Human Tissue Network), and lung epithelial cell lines (HBEC, NL-20, Beas2b; a kind gift from Dr. Avrum Spira). In addition, total RNAs extracted from the surgical lung biopsies of 22 BRAVE I patients were also used (Kim SY *et al., supra*)*.*

RNA libraries enriched for exonic sequences were prepared using the TruSeq^{™} RNA Access Library Prep Kit (Illumina, San Diego, CA) according to manufacturer's instructions. Briefly, RNA samples were fragmented into small pieces using divalent cations under elevated temperature, and random hexamer primers were used to convert fragmented RNAs into cDNAs via reverse transcriptase. cDNA libraries were subsequently used as templates for second strand synthesis; thus, producing libraries of double-stranded cDNAs, which were ligated to sequencing adapters according to the manufacturer's protocol. Finally, enriched libraries of high-specificity, adapter-ligated cDNAs were produced by two rounds of PCR amplification, validation, and capture probe hybridization, as per manufacturer's protocol.

### EXAMPLE 3

### Next-Generation RNA Sequencing

In this example, exome-enriched next-generation RNA sequencing was performed on select samples that met in-process PCR yield criteria using a NextSeq^{™} 500 instrument (Illumina), per manufacturer's instructions, at a targeted read depth of up to 25 million paired-end reads per sample, and, after data quality filtering, expression counts for 17,601 Ensembl genes was normalized and input to machine learning algorithms. Machine learning was used to train an elastic net logistic regression model. Performance was evaluated by cross-validation and on an independent set of 31 patients. The sequencing and algorithm development was performed as follows.

Briefly, 10ng of total RNA was amplified using the Ovation^{™} RNASeq System v2 (NuGEN, San Carlos, California) and TruSeq^{™} (Illumina, San Diego, California) sequencing libraries were prepared and sequenced on an Illumina HiSeq according to manufacturer's instructions (as described in Example 2). Raw sequencing (FASTQ) files were aligned to the Human Reference assembly 37 (Genome Reference Consortium) using the STAR RNAseq aligner software (Dobin A, et al., Bioinformatics 2013 Jan 1;29(1):15-21, incorporated herein by reference in its entirety). Read counts for up to 26,268 Ensembl annotated gene-level features were determined using HTSeq (Anders S, et al., Bioinformatics 2015; 31:166-169, incorporated herein by reference in its entirety).

Sequencing data quality metrics were generated using RNA-SeQC (DeLuca DS, et al., Bioinformatics 2012;28:1530-153222, incorporated herein by reference in its entirety). Quality metrics in each replicate were evaluated against acceptance metrics for total reads, mapped unique reads, mean per-base coverage, base duplication rate, the percentage of bases aligned to coding regions, the base mismatch rate, and uniformity of coverage within genes. Sequencing data was filtered to exclude features not targeted for enrichment by the library assay, and genes annotated in Ensembl as pseudogenes, non-expressed exons in T-cell receptor or immunoglobulin genes, or rRNAs, resulting in 17,601 Ensembl genes with high confidence of specific enrichment.

For the 84 patient classifier (see Figure 2), genes with variable expression across multiple assays (total inter-assay SD > 0.3) were also excluded, resulting in 14,811 genes with reproducible expression run-to-run. Expression count data was scaled by gene dispersion function and VST transformed, prior to downstream analysis. Principal component analysis was performed in R using the 'princomp' function (https://www.r-project.org). Model feature selection and parameter estimation were performed by logistic regression with elastic net penalty as described in Friedman J, et al., Journal of statistical software 2010;33:1-22, incorporated herein by reference in its entirety. Parameter tuning and performance evaluations were determined by leave-one-patient-out cross validation (LOPO CV).

### EXAMPLE 4

### Patient cohort characteristics

Samples from 113 ILD patients enrolled at 18 clinical sites as part of the BRAVE study (see Example 9) were screened for use in developing the molecular test for ILD. FIG. 2 shows a flow diagram of the 113 patients and associated TBB samples screened for use in this study, and it illustrates the cohorts (central squares), processing steps (trapezoids), and exclusions (lateral squares), of patients and samples at each sequential step of processing. Patients were assigned to training and test sets prospectively, prior to the availability of pathology diagnoses. Laboratory and analytical personnel remained blinded to the pathology diagnoses and labels of the test set until after algorithm lock and scoring.

We obtained pathology diagnoses specific to individual lobes of the lung for 95 of these patients using the central pathology review process described in Example 1.

We excluded diagnoses which required un-blinded review (i.e., conferral) and one patient diagnosed with lung cancer, resulting in 89 patients with high confidence ILD pathology in at least one lung lobe.

We extracted total RNA from 496 TBB samples collected from the 113 patients, and ultimately generated high quality RNAseq data for 407 samples derived from 108 patients.

The union of diagnostic patients and high-quality sample data represents 283 samples from 84 patients (52 UIP and 32 non-UIP) (Figure 2, Table 2).

We prospectively assigned 53 patients to algorithm training and 31 patients to a validation cohort, targeting an equivalent UIP prevalence between training and test cohorts (Table 2).

| **Table 2: Demographics and UIP prevalence** | | | | | |
|---|---|---|---|---|---|
| | | **Training Set** | **Test Set** | **Total** | |
| Number of subjects | | 53 | 31 | 84 | |

| **Clinical factors** | | | | | |
|---|---|---|---|---|---|
| | age, median (range) | 63.5 (31-88) | 62 (18-78) | 63 (18-88) | |
| | male gender, no. (%) | 26 (49%) | 14 (45%) | 40 (48%) | |
| | smoking history, yes, no. (%) | 34 (64%) | 19 (61%) | 53 (63%) | |

| **UIP prevalence by pathology** | | | | | |
|---|---|---|---|---|---|
| | | 26 of 38 | 17 of 22 | 43 of 60 | |
| | by surgical lung biopsy, no. UIP (%) | (68%) | (77%) | (72%) | |
| | | Classic UIP | 11 | 6 | 17 |
| | | UIP | 9 | 6 | 15 |
| | | Difficult UIP | 5 | 5 | 10 |
| | | Favor UIP | 1 | 0 | 1 |
| | by cryobiopsy, no. UIP (%) | | 6 of 11 (55%) | 2 of 6 (33%) | 8 of 17 (47%) |
| | | UIP | 2 | 0 | 2 |
| | | Difficult UIP | 0 | 1 | 1 |
| | | Favor UIP | 4 | 1 | 5 |
| | by transbronchial biopsy, no. UIP (%) | | 1 of 4 (25%) | 0 of 3 (0%) | 1 of 7 (14%) |
| | | Difficult UIP | 1 | 0 | 1 |
| | | | 33 of 53 | 19 of 31 | 52 of 84 |
| | total UIP prevalence, no. UIP (%) | | (62%) | (61%) | (62%) |

| **UIP prevalence by radiology** | | | | | |
|---|---|---|---|---|---|
| | | Definite UIP | 4 | 2 | 6 |
| | | UIP | 4 | 2 | 6 |
| | | Probable UIP | 0 | 1 | 1 |
| | | | | | 13 of 79 |
| | total UIP prevalence, no. UIP (%) | | 8 of 52 (15%) | 5 of 27 (19%) | (16%) |

Due to several rare non-UIP II,Ds in our prospective collections, some subtypes are represented by single cases in the patient cohort (Table 14, Figure 3). Single cases of cellular NSIP, Favor HP, emphysema and pneumocystis pneumonia were assigned to the training cohort, whereas single cases of diffuse alveolar damage, pulmonary hypertension and eosinophilic pneumonia were assigned to the test set. The diversity and paucity of ILD subtypes prevalent in these patients illustrates the challenge of training a genomic classifier on a balanced spectrum of II,Ds as encountered in clinical practice.

Radiology performed on our patient cohort as part of routine clinical care provides an independent estimate of UIP prevalence. We performed expert review of available HRCT scans and summarized the radiology findings according to ATS criteria for the UIP pattern (*Raghu G.,* 2011, *supra*)*.* The prevalence of HRCT UIP pattern in our cohort was 16%, compared to 62% by all pathology biopsy types (Table 2). The prevalence of UIP was higher in SLB than in bronchoscopic biopsies (72% vs. 47% [cryobiopsy] vs. 14% [transbronchial biopsy]), with definitive UIP typically identified in SLBs (Table 2).

### EXAMPLE 5

### Classifier development and performance using individual TBB samples

We evaluated multiple normalization schemes, feature selection and machine learning algorithms on our training set of 170 TBB samples from 53 patients, using a variety of genomic and clinical features. In cross validation, we observed the highest and most stable classification performance from a logistic regression model with elastic net penalty trained on expression count data, which uses 169 genes as features (Table 15). The model achieves a receiver-operator characteristic area under the curve (ROC-AUC) in cross-validation on the training set of 0.85 (FIG. 3A, FIG. 3B) based on sample level data.

We defined a decision boundary targeting high (92%) specificity, and observed a corresponding sensitivity of 65% (FIG. 3A, FIG. 3B).

Using this classifier, TBB samples from the independent test set of 113 samples from 31 patients was prospectively scored, and the classifier showed a ROC-AUC of 0.86 with sensitivity of 63% [95% CI: 43-87] and specificity of 86% [95% CI: 73-97] (FIG. 3C, FIG. 3D) based on sample level data. Cross-validation performance that generalizes to a validation cohort suggests that robust training was achieved despite the relatively modest cohort size.

Algorithm re-training on the combined cohort of 283 TBB samples from 84 patients resulted in a cross-validated ROC-AUC of 0.87 [CI: 0.82-0.91] (sensitivity of 63% [CI: 54-72], specificity of 91% [CI: 80-98]) when all TBB samples from each patient are scored separately. Similar cross-validation results (as observed in this case) on the larger set of samples is promising, and will need to be evaluated on an additional independent test set, currently planned as prospective patients are accrued in the BRAVE studies.

Thus, we have demonstrated that a UIP genomic classifier using gene expression signature can effectively distinguish the spatially and temporally heterogeneous fibrotic disease pattern characteristic of UIP from the uniform and typically active fibrosis associated with immune responses (RB-ILD/DIP, eosinophilic pneumonia, granulomatous disease), inflammation (NSIP, HP), or as an acute response to injury¹⁰.

All statistical analyses were carried out using R version 3.0.1²¹. For the microarray classifier, genes differentially expressed between UIP and non-UIP classes were ranked by limma²⁶, then the top 200 genes with lowest false discovery rate (FDR) (< 0·0003) were carried forward as candidate genes for model building. Several models were built using different methods, and the one with the lowest error was chosen. Feature selection and model estimation were performed by logistic regression with *lasso* penalty using *glmnet²⁷.* For the RNAseq classifier, genes were ranked by FDR resulting from a Wald-style test implemented in the *DESeq2²²* package on the raw count data. The top features (N ranging from 10 to 200) were used to train a linear support vector machine (SVM)²³ using the *e1071* library²⁴ on the normalized expression data.

Classifier performance was evaluated by CV and, when available, by an independent test set. To minimize over-fitting, a single patient was maintained as the smallest unit when defining the training/test set and the CV partition; i.e. all samples belonging to the same patient were held together as a group in the training/test set or in CV partitions. The CV methods used include leave-one-patient-out (LOPO) and 10-fold patient-level CV.

Figure 3 shows the results of single-sample classification performances.

Performance was reported as the area under the curve (AUC), and specificity (1.0 - false positive rate) and sensitivity (1.0 - false negative rate) at a given score threshold. We set the score threshold to require at least >90% specificity. For each performance measurement, 95% confidence intervals were computed using 2000 stratified bootstrap replicates and the *pROC* package²² and reported as [CI lower-upper].

### EXAMPLE 6

### Classifier development and performance using pooled samples

While overall single-sample performance achieved in Example 5 was excellent, the classifier did not detect UIP in some samples from some UIP patients (FNs). As UIP was frequently detected in other samples from the same patient, sampling effects, either insufficient tissue sampling or disease heterogeneity, came under suspicion as a source of FNs.

We did not observe a systematic reduction in alveolar content in false negative samples, ruling out inadequate sampling of alveolar tissue as the cause (see Example 7). Thus, disease heterogeneity or technical sample quality effects remain possible explanations for false negatives. Importantly, we chose expert pathology review as the reference standard for the presence of UIP. Despite known issues of inter-operator disagreement^{4,29,30}, we achieved blinded agreement between two expert pathologists at the subtype level for 83% of our patients.

By design, our clinical study collects multiple TBB samples per patient, typically two to three per lung lobe, to mitigate possible disease and sampling heterogeneity effects, which could result in training error or false test calls. For most patients, our sample-level classifier correctly detects disease in more than one sample of the available TBBs per patient, consistent with overall high sample-level test accuracy (Figure 3). This raises the possibility that patient-level reporting of UIP based on mixtures of multiple TBB samples is feasible by pooling multiple samples per patient, and we hypothesized that such mixtures may improve detection accuracy overall at the patient level. We therefore evaluated test designs involving combinations of multiple TBB samples.

We first used an *in silico* approach to derive models that simulate mixing multiple TBB samples from the same patient to yield a single test result. Herein, this approach is referred to as "*in silico* mixing" or, interchangeably, "*in silico* pooling". *In silico* within-patient mixtures were modeled from multiple samples by averaging scaled gene count data prior to variance stabilized transformation (VST). Simulations were performed 100 fold at each condition with gene-level technical variability added at the VST level.

The scores from the *in silico* simulated mixtures were then compared to actual mixtures generated in vitro for eight patients, as well as to the corresponding individual (e.g. unmixed) TBB sample scores (Figure 4A). The results indicate that our *in silico* modeling reasonably approximates scores observed from actual mixtures and individual TBB samples.

We then used this analytical method to simulate mixtures of two through five TBBs per patient, selected at random within each patient (Figure 4B). By simulation, mixtures of two or three samples per patient show increasing classification accuracy versus a single sample per patient selected at random (Figure 4B). Furthermore, mixtures of 4 or 5 TBBs show reduced variability (i.e., higher confidence) in the performance estimate with similar maximal accuracy (Figure 4B). At a targeted specificity of ~90%, test sensitivity in mixtures improves to ~72%, with reduced variability (AUC=0.90 [CI 0.88-0.93], sensitivity=72% at 90% specificity [CI 60-81]) ( Figure 4C). In a set of 33 subjects with two upper lobe and three lower lobe TBBs available for every subject, mixture simulation shows no improvement in performance when sampling is restricted to the upper or lower lobes (Figure 4D). This analysis suggests that mixtures of up to five TBB samplings per patient can maximize the accurate detection of the UIP pattern, using a single molecular test. Such a result may be surprising because pooling is expected to introduce more variability due to cellular heterogeneity.

Thus, physical or *in silico* mixing studies suggest that combining multiple samplings per patient results in increased accuracy.

By training on all samples separately (i.e., as described in Example 5), we maximized representation and sampling diversity, and mitigated a priori sub-sampling bias of available samples. By testing on sample mixtures, we appear to mitigate sampling effects, as demonstrated by improved test accuracy.

### EXAMPLE 7

### Sampling heterogeneity and performance

Given that there is established disease heterogeneity in the lungs of patients with ILD^{4, 21-23}, the finding that strong classifier performance may be obtained with variable sampling of the lungs prompted the question of whether adequate alveolar sampling is necessary during the TBB procedure. We hypothesized that if accurate classification of UIP versus non-UIP required gene signals from alveolar cells, then those samples with a paucity of alveolar cells should give rise to more classifier errors (particularly FNs) than samples with greater alveolar content. To address whether classifier accuracy depends upon adequate alveolar sampling, we tested the correlation between classifier accuracy and alveolar-specific genes.

Specifically, we first developed a semi-quantitative genomic measure of alveolar content in the TBBs and then used this metric to determine whether it was correlated with classifier accuracy. TBB samples were evaluated for the expression of 44 lung specific genes, reported in the literature to be markers of bronchiolar, alveolar, and lung progenitor cells^{E5-E9}(Table 16). Unsupervised clustering by principal components using the 44 markers suggests that this TBB cohort represents a continuous spectrum of sampled lung tissue, a subset of which overlaps with surgical lung biopsies (Figure 5A; TBB samples in blue, SLB samples in orange).

We developed two alveolar metrics, one for type I and one for type II alveolar cells.

For the type I alveolar statistic we summed the expression of two genes, PDPN and AQP5. These genes show a continuous pattern of gene expression amongst the sample set.

Our second approach, used for the type II alveolar metric, was to examine markers that showed evidence of bimodal expression within the population of TBBs. This pattern is seen for nine genes, five of which (SFTPB, SFTPC, SFTPD, ABCA3, CEBPA) are alveolar type II (ATII) specific, three (AGER, GPRC5A, HOPX) are alveolar type I (ATI) specific, and one (SFTPA1) is seen in both type I and II cells (Figure 5B; TBB expression counts in blue, SLB expression counts in orange). Correlated, directionally consistent expression is seen between SFTPA1, SFTPB, SFTPC, and SFTPD, but not between PDPN and AQP5, or between members of these two groups (Figure 5C; TBB expression counts in blue, SLB expression counts in orange). We therefore selected the four surfactant proteins SFTPA1, SFTPB, SFTPC and SFTPD as markers of type II alveolar content, and summed their expression within samples as a proxy measure of alveolar content within each sample.

While these metrics show a wide range of type I and type II alveolar specific gene expression across various samples types, with high expression in SLBs and many TBBs, and low expression in a variety of non-lung tissue types and in three bronchial epithelial cell lines (Beas2b, HBEC, and NL-20) (FIG. 6A), the expression of these transcripts did not correlate with classifier accuracy (Pearson's correlation, 0.03, p-value=0.61). Thus, these results show that neither false negative nor false positive errors are associated with lower type I or II alveolar I content, suggesting that accurate classification results may be achieved in TBB samples with variable cellular composition (FIG. 6B).

We also found no significant correlation between classifier accuracy on individual samples and TBB alveolar gene expression, RNA quality, or RNA yield (Table 3).

**Table 3: Pearson's correlations of TBB sample properties to classification accuracy**

| **Sample property** | | **Correlation** | **p-value** |
|---|---|---|---|
| Alveolar I expression statistic | | -0.07 | 0.27 |
| Alveolar II expression statistic | | 0.03 | 0.61 |
| RNA quality | | | |
| | RIN | -0.07 | 0.24 |
| | DV₂₀₀ | -0.10 | 0.09 |
| RNA yield in nanograms | | 0.06 | 0.32 |

These results suggest that accurate classification results can be achieved in TBB samples with variable alveolar content.

### EXAMPLE 8

### Biological Pathways Associated With Genes Used By The Classifiers

### PANTHER^{™} Pathway Analysis

We used DESeq2¹⁹ to identify differential expression between UIP and non-UIP TBBs derived from 84 patients with pathology truth. Ensembl genes significantly upregulated in UIP (n=926) and in non-UIP (n=1330) at false-discovery rate (FDR) adjusted p-values ≤ 0.05 were used as input to the PANTHER^{™} classification system for pathway over-representation analysis (web version 11.0, released 2016-07-15)(Mi H, Lazareva-Ulitsky B. et al., Nucleic Acids Res 2005;33:D284-D288, incorporated herein by reference in its entirety). PANTHER^{™} pathways were curated to remove general or redundant pathway classifications, and ordered by significance (Table 4). We found that TBBs with UIP are significantly enriched for the expression of markers of cellular metabolism, adhesion and developmental processes while non-UIP TBBs show evidence of immune activation, lipid metabolism, stress responses and cell death (Table 4). Aberrant re-activation of developmental pathways and cellular proliferation are hallmarks of IPF²⁴⁻²⁷.

| **Table 4: Biological Processes Over Represented in UIP and non-UIP TBB Samples** | | | | |
|---|---|---|---|---|
| **Biological Process** | **Number expecte d** | **Number observe d** | **Fold Increase** | **P-value** |
| **Over Represented in UIP** | | | | |
| Cell-cell adhesion | 13 | 44 | 3.4 | <0.0001 |
| Cellular component morphogenesis | 23 | 53 | 2.3 | <0.0001 |
| Nervous system development | 29 | 63 | 2.2 | <0.0001 |
| Transcription, DNA-dependent | 65 | 122 | 1.9 | <0.0001 |
| RNA metabolic process | 88 | 144 | 1.6 | <0.0001 |
| Nucleobase metabolic process | 135 | 189 | 1.4 | 0.0002 |
| Nitrogen compound metabolic process | 86 | 129 | 1.5 | 0.0008 |
| Ectoderm development | 17 | 39 | 2.3 | 0.0010 |
| Visual perception | 8 | 23 | 2.8 | 0.0036 |
| Mesoderm development | 19 | 37 | 1.9 | 0.0371 |
| Muscle contraction | 7 | 18 | 2.7 | 0.0398 |

| **Over Represented in non-UIP** | | | | |
|---|---|---|---|---|
| Antigen processing and presentation | 4 | 20 | 5.3 | <0.0001 |
| Cellular defense response | 13 | 39 | 3.0 | <0.0001 |
| Lipid metabolic process | 33 | 68 | 2.1 | <0.0001 |
| Immune system process | 79 | 131 | 1.7 | <0.0001 |
| Cholesterol metabolic process | 5 | 19 | 3.8 | 0.0003 |
| Steroid metabolic process | 11 | 30 | 2.7 | 0.0004 |
| Immune response | 44 | 74 | 1.7 | 0.0054 |
| Apoptotic process | 26 | 49 | 1.9 | 0.0057 |
| Phosphate-containing compound metabolism | 77 | 114 | 1.5 | 0.0076 |
| I-kappaB kinase/NF-kappaB cascade | 4 | 15 | 3.4 | 0.0157 |
| Response to stress | 53 | 83 | 1.6 | 0.0172 |
| Transmembrane tyrosine kinase signaling | 12 | 28 | 2.3 | 0.0213 |
| Catabolic process | 49 | 77 | 1.6 | 0.0222 |
| Hemopoiesis | 6 | 17 | 2.9 | 0.0328 |

### EXAMPLE 9

### BRAVE study design

The purpose of the BRAVE (BRonchial sAmple collection for a noVel genomic test) study is to collect bronchoscopic specimens, clinical data, and associated pathology slides for external review in order to optimize a molecular profiling test that will provide a range of diagnostic and prognostic information about interstitial lung disease (ILD).

BRAVE is divided into three arms: BRAVE-1 is intended to enroll patients scheduled for a diagnostic surgical lung biopsy (SLB) as part of their usual care clinical diagnosis. BRAVE-2 is intended for patients scheduled for diagnostic bronchoscopy only. BRAVE-3 is intended for patients scheduled for a diagnostic cryobiopsy.

Bronchoalveolar lavage, blood, serum and buccal swabs are also collected. Subjects will be enrolled until a sufficient number of samples are collected to satisfy power and sample size requirements for the development and prospective validation of a molecular test for ILD.

Subjects are followed for up to one year after sample collection in order to assess progression of disease. Patients aged less than 18 years, or for whom SLB is not medically indicated, or who are undergoing SLB for non-ILD medical conditions, are not eligible for study enrollment. Patients with medical conditions which are contraindications to performing bronchoscopic biopsy, or for whom bronchoscopic sampling is not recommended or difficult, are also excluded from the BRAVE study.

### EXAMPLE 10

### Generation of the Envisia Classifier

Having demonstrated that machine learning can detect a UIP histopathologic pattern in lung tissue obtained by SLB and TBB (see Examples 1-9), we sought to extend the classifier training in a larger and more diverse group of patients, and to validate a locked algorithm on an independent, prospectively collected set of subjects.

### Methods

A total of 201 subjects were enrolled in a prospective, multi-center study at 18 U.S. and European sites. We collected up to five TBBs per subject, paired at the lobe level with standard-of-care lung tissue biopsy samples. A histologic pattern diagnosis, made using a panel of three expert pathologists, was obtained on 139 subjects. Exome-enriched RNA sequencing was performed on pooled TBBs and the resulting sequences were aligned and transcript counts extracted for xyx genes. We trained and locked a machine learning algorithm, the Envisia Genomic Classifier, using approximately 90 patients and then validated the test on an independent set of 49 subjects with histology reference labels. We optimized the test decision boundary to give high specificity, *i.e*., to reduce false positives as this may create harm by overcalling the UIP pattern, potentially leading to the unnecessary risk and expense of IPF therapy. We locked all classifier parameters, and defined the patient and sample characteristics that are within indication for testing. We report here the prospective clinical validation of the Envisia Genomic Classifier in TBBs from an independent cohort of 49 subjects, and compare its classification performance to HRCT.

### Study design and oversight

For this independent validation study, a total of 88 subjects were enrolled into three separate BRAVE studies (Fig 1). In BRAVE-1, subjects underwent a clinically-indicated SLB (n=43); BRAVE-2 subjects underwent a clinically indicated TBB (n=9); and BRAVE-3 subjects underwent a clinically indicated cryobiopsy (n=36). BRAVE-2 subjects had only TBB for histopathology evaluation, BRAVE-1 and 3 subjects were diagnosed by SLB or cryobiopsy, respectively.

Up to five dedicated transbronchial biopsies (TBB) (two upper lobe and three lower lobe, typically) were collected for molecular testing from the same lung lobes identified by participating physicians for clinically-indicated biopsy for histopathologic diagnosis. The study-indicated TBB specimens; study site-prepared histopathology slides and de-identified patient clinical data; HRCT of the chest; local clinical diagnoses; and one year and two year follow up, where available, were provided to Veracyte. Results of molecular testing were not provided to participating physicians, nor were they used to inform patient diagnosis or treatment.

HRCT scans were reviewed and classified by an expert thoracic radiologist (D. Lynch) as Definite UIP, Probable UIP, or Possible UIP9, desquamative interstitial pneumonia (DIP), hypersensitivity pneumonitis (HP), Langerhans cell histiocytosis (LCH), nonspecific interstitial pneumonia (NSIP), organizing pneumonia (OP), respiratory bronchiolitis (RB), Sarcoidosis or 'other' (unclassifiable). Veracyte clinical personnel reviewed and interpreted study site radiology descriptions using the same criteria, but with "Inconsistent with UIP" in place of the specific non-UIP diagnoses³⁵.

Histopathology slides from the clinically-indicated SLB, cryobiopsy, or TBB were independently reviewed by two or three expert lung pathologists blinded to patient clinical information, as previously described^{E1,E10} Each pathologist independently determined a histopathologic pattern diagnosis for each lung lobe sampled. We defined consensus as blinded agreement at the histologic pattern level between two of two or two of three reviewing pathologists, or by agreement after unblinded consultation between three pathologists (conferral), if blinded agreement was not achieved.

Veracyte personnel assigned reference labels of UIP or non-UIP to each study subject based on the consensus of the lobe-level diagnoses, according to the following categories (Figure 9). If any lobe was diagnostic of UIP by pathology, that subject was assigned a UIP label⁴. A subject diagnostic for non-UIP pathology in any lobe was assigned a non-UIP reference label if all other lobes were also non-UIP or non-diagnostic (Figure 9). All Veracyte laboratory and analysis personnel were blinded to the reference labels during testing and algorithm development.

### Laboratory test procedure

Study-indicated TBB from 88 BRAVE patients were collected into a dedicated nucleic acid preservative (RNAprotect, QIAGEN, Valencia, CA), stored cold onsite for up to 14 days and shipped to Veracyte for processing. We extracted total RNA using a modified AllPrep Micro procedure (QIAGEN), followed by quantitation using RNA-binding dye fluorescence (QuantiFluor, Promega, Madison, WI). We pre-specified that a minimum of three and maximum of five TBBs per subject, each yielding at least 31ng of total RNA, were required for study inclusion. Nine subjects were excluded due to insufficient numbers of samples or RNA yields (Figure 8). In addition, specimens containing a foreign object (toothpicks, one subject), specimens delivered to Veracyte with missing preservative (one subject), and specimens in shipment beyond the shipping container cooling limit of 48 hours (five subjects), were also prospectively excluded (Figure 8). The individual TBBs for 72 subjects thus satisfied our pre-specified study inclusion criteria.

Pooled RNA for each subject was input to a partially automated TruSeq RNA Access Library Prep procedure (Illumina, San Diego, CA) to enrich for expressed exonic sequences, and sequenced to a targeted depth of ≥ 25M paired-end reads on NextSeq 500 instruments (Illumina). Count data were evaluated against criteria for total numbers of sequenced and uniquely mapped reads, the overall proportions of mapped reads and of exonic reads, the mean per-base coverage, the uniformity of base coverage, and base duplication and mismatch rates. Data from one subject did not meet these criteria and was excluded, leaving 71 subjects (Figure 8). Expression count data was normalized with respect to sequencing depth (scale factors) and transformed by variance stabilized transformation using DESeq216, prior to classification.

### Algorithm development

354 individual TBB samples from 90 subjects previously enrolled in the BRAVE studies from December of 2012 to July of 2015^{E10} were used exclusively to train the machine learning algorithm (the classification model). Feature selection and hyperparameter optimization were performed by the algorithm using elastic net logistic regression. Performance of the model was evaluated in the training set using receiver-operator characteristic areas under the curve (ROC-AUCs), determined by leave-one-patient-out cross validation (CV). A test decision boundary was selected that optimized specificity (minimizes UIP false positive calls) in the training set. A penalized logistic classifier using 190 genes as features, with a locked decision boundary (the Envisia Genomic Classifier) was thus defined (Table 5). Envisia reports a molecular diagnosis of UIP or non-UIP for each pool of TBBs. Subjects with classification scores above the decision boundary are called UIP by Envisia while subjects with scores equal or below the decision boundary are called non-UIP. The validation was scored both internally and independently by third parties not involved in the development of the test, prior to the unveiling of the reference labels.

**Table 5: 190 genes used by the Envisia Genomic Classifier**

| Gene ID | Gene Symbol |
|---|---|
| ENSG00000005381 | MPO |
| ENSG00000005955 | GGNBP2 |
| ENSG00000007908 | SELE |
| ENSG00000007933 | FMO3 |
| ENSG00000010379 | SLC6A13 |
| ENSG00000012232 | EXTL3 |
| ENSG00000022556 | NLRP2 |
| ENSG00000026950 | BTN3A1 |
| ENSG00000033050 | ABCF2 |
| ENSG00000038295 | TLL1 |
| ENSG00000048052 | HDAC9 |
| ENSG00000054803 | CBLN4 |
| ENSG00000054938 | CHRDL2 |
| ENSG00000060688 | SNRNP40 |
| ENSG00000071909 | MYO3B |
| ENSG00000072310 | SREBF1 |
| ENSG00000073605 | GSDMB |
| ENSG00000078070 | MCCC1 |
| ENSG00000079385 | CEACAM1 |
| ENSG00000081041 | CXCL2 |
| ENSG00000081985 | IL12RB2 |
| ENSG00000082781 | ITGB5 |
| ENSG00000083814 | ZNF671 |
| ENSG00000086544 | ITPKC |
| ENSG00000089902 | RCOR1 |
| ENSG00000092295 | TGM1 |
| ENSG00000099251 | HSD17B7P2 |
| ENSG00000099974 | DDTL |
| ENSG00000100376 | FAM118A |
| ENSG00000100557 | C14orf105 |
| ENSG00000101544 | ADNP2 |
| ENSG00000102837 | OLFM4 |
| ENSG00000103044 | HAS3 |
| ENSG00000103257 | SLC7A5 |
| ENSG00000104812 | GYS1 |
| ENSG00000105255 | FSD1 |
| ENSG00000105559 | PLEKHA4 |
| ENSG00000105696 | TMEM59L |
| ENSG00000105784 | RUNDC3B |
| ENSG00000105983 | LMBR1 |
| ENSG00000106018 | VIPR2 |
| ENSG00000106178 | CCL24 |
| ENSG00000107929 | LARP4B |
| ENSG00000108312 | UBTF |
| ENSG00000108551 | RASD1 |
| ENSG00000109205 | ODAM |
| ENSG00000110092 | CCND1 |
| ENSG00000110900 | TSPAN11 |
| ENSG00000110975 | SYT10 |
| ENSG00000111218 | PRMT8 |
| ENSG00000111321 | LTBR |
| ENSG00000111328 | CDK2AP1 |
| ENSG00000112164 | GLP1R |
| ENSG00000112299 | VNN1 |
| ENSG00000112852 | PCDHB2 |
| ENSG00000114248 | LRRC31 |
| ENSG00000114923 | SLC4A3 |
| ENSG00000115415 | STAT1 |
| ENSG00000115607 | IL18RAP |
| ENSG00000116285 | ERRFI1 |
| ENSG00000116761 | CTH |
| ENSG00000119711 | ALDH6A1 |
| ENSG00000119725 | ZNF410 |
| ENSG00000120217 | CD274 |
| ENSG00000120738 | EGR1 |
| ENSG00000120903 | CHRNA2 |
| ENSG00000121380 | BCL2L14 |
| ENSG00000121417 | ZNF211 |
| ENSG00000122497 | NBPF14 |
| ENSG00000124205 | EDN3 |
| ENSG00000124702 | KLHDC3 |
| ENSG00000124935 | SCGB1D2 |
| ENSG00000125255 | SLC10A2 |
| ENSG00000128016 | ZFP36 |
| ENSG00000128266 | GNAZ |
| ENSG00000128791 | TWSG1 |
| ENSG00000128891 | C15orf57 |
| ENSG00000130164 | LDLR |
| ENSG00000130487 | KLHDC7B |
| ENSG00000130598 | TNNI2 |
| ENSG00000131095 | GFAP |
| ENSG00000131142 | CCL25 |
| ENSG00000132199 | ENOSF1 |
| ENSG00000132204 | LINC00470 |
| ENSG00000132915 | PDE6A |
| ENSG00000132938 | MTUS2 |
| ENSG00000133636 | NTS |
| ENSG00000133794 | ARNTL |
| ENSG00000134028 | ADAMDEC1 |
| ENSG00000134245 | WNT2B |
| ENSG00000135148 | TRAFD1 |
| ENSG00000135447 | PPP1R1A |
| ENSG00000135625 | EGR4 |
| ENSG00000136881 | BAAT |
| ENSG00000136883 | KIF12 |
| ENSG00000136928 | GABBR2 |
| ENSG00000136933 | RABEPK |
| ENSG00000137285 | TUBB2B |
| ENSG00000137463 | MGARP |
| ENSG00000137573 | SULF1 |
| ENSG00000137709 | POU2F3 |
| ENSG00000137968 | SLC44A5 |
| ENSG00000138166 | DUSP5 |
| ENSG00000138308 | PLA2G12B |
| ENSG00000140274 | DUOXA2 |
| ENSG00000140279 | DUOX2 |
| ENSG00000140323 | DISP2 |
| ENSG00000140450 | ARRDC4 |
| ENSG00000140465 | CYP1A1 |
| ENSG00000140505 | CYP1A2 |
| ENSG00000140718 | FTO |
| ENSG00000141279 | NPEPPS |
| ENSG00000142178 | SIK1 |
| ENSG00000142661 | MYOM3 |
| ENSG00000143185 | XCL2 |
| ENSG00000143195 | ILDR2 |
| ENSG00000143320 | CRABP2 |
| ENSG00000143322 | ABL2 |
| ENSG00000143367 | TUFT1 |
| ENSG00000143379 | SETDB1 |
| ENSG00000143603 | KCNN3 |
| ENSG00000144655 | CSRNP1 |
| ENSG00000145248 | SLC10A4 |
| ENSG00000145284 | SCD5 |
| ENSG00000145358 | DDIT4L |
| ENSG00000145736 | GTF2H2 |
| ENSG00000148541 | FAM13C |
| ENSG00000148700 | ADD3 |
| ENSG00000148702 | HABP2 |
| ENSG00000149043 | SYT8 |
| ENSG00000149289 | ZC3H12C |
| ENSG00000151012 | SLC7A11 |
| ENSG00000151572 | ANO4 |
| ENSG00000152672 | CLEC4F |
| ENSG00000153404 | PLEKHG4B |
| ENSG00000154227 | CERS3 |
| ENSG00000154451 | GBP5 |
| ENSG00000156414 | TDRD9 |
| ENSG00000157103 | SLC6A1 |
| ENSG00000157680 | DGKI |
| ENSG00000158457 | TSPAN33 |
| ENSG00000159231 | CBR3 |
| ENSG00000159674 | SPON2 |
| ENSG00000161609 | CCDC155 |
| ENSG00000162594 | IL23R |
| ENSG00000163029 | SMC6 |
| ENSG00000163110 | PDLIM5 |
| ENSG00000163285 | GABRG1 |
| ENSG00000163412 | EIF4E3 |
| ENSG00000163635 | ATXN7 |
| ENSG00000163644 | PPM1K |
| ENSG00000163735 | CXCL5 |
| ENSG00000163817 | SLC6A20 |
| ENSG00000163884 | KLF15 |
| ENSG00000164604 | GPR85 |
| ENSG00000164821 | DEFA4 |
| ENSG00000165948 | IFI27L1 |
| ENSG00000165973 | NELL1 |
| ENSG00000165983 | PTER |
| ENSG00000166923 | GREM1 |
| ENSG00000167748 | KLK1 |
| ENSG00000168004 | HRASLS5 |
| ENSG00000168036 | CTNNB1 |
| ENSG00000168062 | BATF2 |
| ENSG00000168394 | TAP1 |
| ENSG00000168661 | ZNF30 |
| ENSG00000168938 | PPIC |
| ENSG00000169248 | CXCL11 |
| ENSG00000170113 | NIPA1 |
| ENSG00000170442 | KRT86 |
| ENSG00000170509 | HSD17B13 |
| ENSG00000170837 | GPR27 |
| ENSG00000171016 | PYGO1 |
| ENSG00000171408 | PDE7B |
| ENSG00000171649 | ZIK1 |
| ENSG00000171714 | ANO5 |
| ENSG00000172137 | CALB2 |
| ENSG00000172183 | ISG20 |
| ENSG00000172215 | CXCR6 |
| ENSG00000172667 | ZMAT3 |
| ENSG00000173809 | TDRD12 |
| ENSG00000173812 | EIF1 |
| ENSG00000173926 | MARCH3 |
| ENSG00000175764 | TTLL11 |
| ENSG00000175806 | MSRA |
| ENSG00000176046 | NUPR1 |
| ENSG00000177182 | CLVS1 |
| ENSG00000177294 | FBXO39 |
| ENSG00000178187 | ZNF454 |
| ENSG00000178229 | ZNF543 |

### Statistical analysis

Statistical analysis was performed using R software, version 3.2.3 (https://www.r-project.org). Continuous variables were compared by Student's t-test and categorical variables were compared by chi-squared test. All confidence intervals [CI] are two-sided 95% unless otherwise noted. We assessed test performance using standard measures of prediction accuracy. We used the alveolar type I and II gene expression scores developed previously¹⁴ to assess whether test accuracy correlated with alveolar cell gene expression. We performed a biological pathway analysis on a combined set of the top 1000 genes differentially expressed in the training cohort TBBs (UIP vs. non-UIP) and the 190 classifier genes using GeneTrail software available at http://genetrail.bioinf.uni-sb.de/.

### Results

### Demographic and pathological characteristics of study subjects

A total of 88 subjects were enrolled into one of 3 BRAVE studies at 18 US and European clinical sites between August 2014 and May 2016 (Figure 8). We excluded 16 subjects prior to analytical testing due to specimen mis-handling or insufficient material, and one subject that failed analytical QC during testing. A total of 71 subjects satisfied study inclusion criteria. Of these, two subjects with missing histopathology slides and one subject with adenocarcinoma of the lung were subsequently excluded, leaving a total of 68 subjects for pathology review. We were unable to assign UIP/non-UIP pathology reference labels to 12 subjects with non-diagnostic pathology and 7 subjects with non-classifiable fibrosis, thus they could not be included in the final validation (Figure 8). Final histopathologic pattern diagnoses were determined and UIP/non-UIP reference labels provided for the remaining 49 subjects. These 49 subjects became the final validation group (Figure 8).

The 49 subjects in the final validation group showed no significant differences in subject age, gender, or smoking status when compared to the 88 enrolled subjects and the 39 excluded subjects (Table 6). The final validation set includes a diversity of UIP subtypes as well as non-UIP II,Ds that may be encountered in clinical practice (Table 7).

**Table 6: Clinical characteristics of study subjects.**

| | | Study eligible group N, (%) | Final validation group N, (%) | P-va |
|---|---|---|---|---|
| Gender - n (%) | | | | |
| | Female | 38 (43%) | 21 (43%) | |
| | Male | 50 (57%) | 28 (57%) | |
| Mean age (SD) - yr | | 63.0 (11.7) | 64.1 (10.3) | 0. |

| Smoking status - n (%) | | | | 0. |
|---|---|---|---|---|
| | Yes | 56 (64%) | 33 (67%) | |
| | No | 28 (32%) | 15 (31%) | |
| | Unknown | 4 (5%) | 1 (2%) | |

| Site - n (%) | | | | 0. |
|---|---|---|---|---|
| | Academic | 36 (41%) | 20 (41%) | |
| | Community | 37 (42%) | 24 (49%) | |
| | European | 15 (17%) | 5 (10%) | |

| Study - n (%) | | | | 0. |
|---|---|---|---|---|
| | BRAVE 1 | 43 (49%) | 26 (53%) | |
| | BRAVE 2 | 9 (10%) | 2 (4%) | |
| | BRAVE 3 | 36 (41%) | 21 (43%) | |
| UIP prevalence by pathology, n (%) | | N/A | 24 (49%) | |
| UIP prevalence by radiology, n/n (%) | | N/A | 9/46 (20%) | |
| Radiology missing, n (%) | | N/A | 3 (6%) | |
| **Total subjects** | | 88 | 49 | |

**Table 7: ILD pathologic patterns represented in the validation.**

| **Pathology Pattern Diagnosis** | Final validatio N, (%) |
|---|---|
| **UIP Classification labels** | |
| UIP (Classic UIP, Difficult UIP or Favor UIP) | 19 (39%) |
| UIP with Favor HP; UIP with CIF,NOC; UIP with NSIP; UIP with Pulmonary hypertension | 5 (10%) |
| UIP Total | 24 (49%) |

| **Non-UIP Classification labels** | |
|---|---|
| OP; OP with CIF,NOC; OP with Acute lung injury | 1 (2%) |
| Respiratory bronchiolitis; SRIF; RB with SRIF; RB with CIF,NOC | 6 (12%) |
| Bronchiolitis; Bronchiolitis with Favor bronchiolitis | 1 (2%) |
| Sarcoidosis | 3 (6%) |
| NSIP; Cellular NSIP; Favor NSIP; Cellular NSIP with Favor HP; NSIP with Favor NSIP; Favor NSIP with CIF,NOC | 3 (6%) |
| Hypersensitivity pneumonitis; Favor HP | 4 (8%) |
| DAD; DAD with hemosiderosis | 2 (4%) |
| Eosinophilic pneumonia | 1 (2%) |
| Organizing alveolar hemorrhage | 1 (2%) |
| Exogenous lipid pneumonia | 1 (2%) |
| Amyloid or light chain deposition | 1 (2%) |
| Emphysema; Emphysema with Probable infection; Emphysema with RB | 1 (2%) |
| Non-UIP Total | 25 (51%) |
| **Total** | 49 |

| | |
|---|---|
| * Subjects with different diagnoses across lung lobes are noted as "with". | |

### Envisia Genomic Classifier performance

The Envisia Genomic Classifier for molecular diagnosis of UIP achieved a high specificity of 88% [CI: 68%-97%] and moderate sensitivity of 67% [CI: 45%-84%] in the validation group. The ROC-AUC was 0.85 (Figure 10). Test performance remains within the confidence intervals when the analysis is restricted to 26 subjects with pathology derived exclusively from surgical lung biopsy or 21 subjects with pathology from cryobiopsy (data not shown). Of the 21 cryobiopsy subjects, five were from a single European study site and one, seven, and eight subjects were from three US study sites, respectively.

In examining errors made by the classifier, three of the 25 subjects with non-UIP pathology were classified as molecular UIP (FPs) (Figure 11). One FP had a pathologic pattern diagnosis of advanced small airway disease with follicular bronchiolitis but had a study site-derived clinical diagnosis of probable IPF. A second FP was initially diagnosed with HP by central radiology and cellular NSIP by central pathology, but showed dense fibrosis by HRCT on long term follow-up. A case of NSIP with severe emphysema by radiology and noted on histopathology to exhibit amyloid or light chain deposition was also called molecular UIP (Table 8).

**Table 8: Clinical factors associated with three Envisia Genomic Classifier false positive subjects**

| | | **Radiology** | | | **Pathology** | | **Local Clinical Diagnosis** | |
|---|---|---|---|---|---|---|---|---|
| **FP subject** | **Envisia Call** | Local | Central | Local long term follow-up | Local | Central | Initial Diagnosis | Updated Diagnosis |
| 1 | UIP | IPF | Other (LIP) | N/A | Advanced chronic small airway disease with follicular bronchiolitis | SLB: | Probable IPF | N/A |
| | | | | | | Bronchiolitis (upper lobe) | | |
| | | | | | | Bronchiolitis (lower lobe) | | |
| 2 | UIP | Bibasilar infiltrates w/o honeycombing | HP | N/A | CIF,NOC | SLB: | Cellular NSIP | Dense fibrosis |
| | | | | | | NSIP (lower lobe) | | |
| 3 | UIP | Pulmonary fibrosis | Other (Emphysema) | Severe Emphysema | UIP | Cryobiopsy: | NSIP | NSIP |
| | | | | | | Other- amyloid or light chain deposition (upper lobe) | | |

Eight subjects with pathologic UIP were classified as molecular non-UIP by the Envisia test (FNs) (Figure 11). While the final reference label for these subjects was UIP, half of these cases had a non-UIP diagnosis either by study site pathology, radiology or clinical diagnosis. Study site diagnoses included, HP, RB, NSIP/DIP (later updated to SRIF), and unclassifiable ILD (Table 9). The remaining four cases had a study site diagnosis of IPF, two of which had HRCT pattern diagnoses of UIP, one with NSIP, and one with HP associated with possible underlying autoimmune disease (Table 9).

**Table 9: Clinical details associate with eight Envisia Genomic Classifier FN subjects**

| | | **Radiology** | | | **Pathology** | | **Local Clinical Diagnosis** | |
|---|---|---|---|---|---|---|---|---|
| **FN subject** | **Envisia Call** | Local Non-specific alveolitis | Central Organizing pneumonia | Local long term follow-up SLB-associated changes | Local NSIP | Central | Initial Diagnosis NSIP/DIP | Updated Diagnosis SRIF |
| 1 | Non-UIP | | | | | SLB: | | |
| | | | | | | Classic UIP (middle lobe) | | |
| | | | | | | Classic UIP (lower lobe) | | |
| 2 | Non-UIP | Possible UIP | NSIP | N/A | UIP | Cryobiopsy: | Possible IPF | N/A |
| | | | | | | UIP (lower lobe) | | |
| 3 | Non-UIP | Bilateral pulmonary fibrosis | HP | N/A | UIP | SLB: | Possible IPF | UIP + NSIP with autoimmune disease (possible RA) |
| | | | | | | UIP (upper lobe) | | |
| | | | | | | UIP (middle lobe) | | |
| | | | | | | Classic UIP (lower lobe) | | |
| 4 | Non-UIP | UIP | Definite UIP | N/A | UIP | Cryobiopsy: | Definite IPF | N/A |
| | | | | | | Favor UIP (upper lobe) | | |
| | | | | | | UIP (middle lobe) | | |
| | | | | | | Non-Diagnostic (lower lobe) | | |
| 5 | Non-UIP | NSIP | HP | N/A | hronic interstitial fibrosis, not otherwise classified | SLB: | Unclassifiable ILD | N/A |
| | | | | | | UIP (upper lobe) | | |
| | | | | | | UIP (lower lobe) | | |
| 6 | Non-UIP | IPF | Probable UIP | UIP | UIP | SLB: | Definite IPF | Definite IPF |
| | | | | | | Classic UIP (upper lobe) | | |
| 7 | Non-UIP | Acute exacerbation of chronic ILD | HP | N/A | Chronic HP | SLB: | HP | N/A |
| | | | | | | UIP (upper lobe) | | |
| | | | | | | Classic UIP (middle lobe) | | |
| | | | | | | Classic UIP (lower lobe) | | |
| 8 | Non-UIP | UIP | N/A | N/A | SRIF | Cryobiopsy: | RB | N/A |
| | | | | | | Non-diagnostic (upper lobe) | | |
| | | | | | | Non-diagnostic (middle lobe) | | |
| | | | | | | Favor UIP (lower lobe) | | |

Consistent with guidelines, HRCT is used to evaluate suspect ILD patients with a goal of assessing the presence or absence of UIP pattern (e.g. "HRCT - UIP"). In the absence of a definitive UIP diagnoses by HRCT, patients should be considered for SLB to obtain a histopathologic pattern diagnosis of UIP or non-UIP⁵. To establish a performance baseline for Envisia molecular UIP calls, we evaluated the predictive value of HRCT-UIP, using histopathologic UIP as the reference standard. We examined HRCT pattern diagnoses from expert review (D. Lynch) as well as study site pattern diagnoses. In the final validation set, central radiology shows perfect specificity and positive predictive value (PPV), with marginal sensitivity (Figure 12). This is consistent with previous reports of high specificity, but low sensitivity of expert HRCT - UIP¹⁷. The specificity and PPV of local radiology in this group of patients is substantially lower than expert central review, at 70% and 67%, respectively (Figure 12).

The PPV of molecular UIP among subjects with central HRCT - UIP is 100%, similar to the overall PPV of expert radiology (versus pathology), but far superior to the overall PPV of study site HRCT - UIP (Figure 12). The PPV of molecular UIP decreases to 73% among subjects with study site HRCT - UIP, but remains 100% among central HRCT - UIP cases (Figure 12). Interestingly, molecular UIP calls are highly accurate among subjects with a study site radiology diagnosis of Inconsistent with UIP, showing a PPV of 100% and a NPV of 89%, similar to the 100% PPV observed by central radiology (Figure 12). Moreover, molecular UIP shows improved sensitivity over expert radiology, at 67% versus 41%. Among 15 subjects with a specific central radiology diagnosis of HP, nine had a UIP histopathologic pattern (Table 10). Molecular UIP correctly identified histopathologic UIP in six of the nine HP patients with a UIP histopathologic pattern (Table 10), suggesting that molecular diagnosis by the Envisia test can help identify the presence of histopathologic UIP in HP patients.

**Table 10: Performance of Envisia, relative to pathology, for 15 subjects with central radiology diagnosis of hypersensitivity pneumonitis**

| Subject | Pathology | Radiology | Envisia Call |
|---|---|---|---|
| 1 | UIP | HP | UIP |
| 2 | NSIP | HP | UIP |
| 3 | UIP | HP | UIP |
| 4 | UIP | HP | UIP |
| 5 | UIP | HP | UIP |
| 6 | UIP | HP | UIP |
| 7 | UIP | HP | UIP |
| 8 | UIP | HP | nonUIP |
| 9 | DAD | HP | nonUIP |
| 10 | UIP | HP | nonUIP |
| 11 | UIP | HP | nonUIP |
| 12 | DAD | HP | nonUIP |
| 13 | SRIF | HP | nonUIP |
| 14 | HP | HP | nonUIP |
| 15 | HP | HP | nonUIP |

The recognized challenge of achieving diagnostic pathology meant that labels of UIP or non-UIP may not be determined for 19 subjects (Table 11). Patients similar to these may be encountered in the clinic and thus potentially tested by Envisia. Therefore, we compared Envisia test results to the available clinical information associated with these subjects. Among six subjects with molecular UIP by Envisia, there are two with a HRCT - UIP pattern, and two with clinical diagnoses of IPF (Table 11). Among the 13 subjects with molecular non-UIP by Envisia, seven have HRCT non-UIP pattern; three of whom have a clinical diagnosis of a non-UIP condition (Table 11).

**Table 11: Envisia classification of subjects with non diagnostic pathology or unclassifiable fibrosis**

| (secondary analysis group). | | | | | | |
|---|---|---|---|---|---|---|
| Subject | Envisia score | Envisia call | Central Pathology | Central Radiology | Local Clinical Diagnosis | Local Pathology Diagnosis |
| 1 | -2.05 | **NonUIP** | CIF,NOC | **Other- Aspiration** | **Bronchiolitis** | |
| 2 | -1.43 | **NonUIP** | Non diagnostic | **HP** | Other | |
| 3 | -0.91 | **NonUIP** | Non diagnostic | **Other- Aspiration** | Other | Other |
| 4 | -0.49 | **NonUIP** | Non diagnostic | **Asbestosis** | | |
| 5 | -0.39 | NonUIP | CIF,NOC | | | |
| 6 | -0.25 | **NonUIP** | Non diagnostic | **Other- Aspiration** | Other | |
| 7 | 0.13 | NonUIP | Non diagnostic | | | |
| 8 | 0.15 | **NonUIP** | CIF,NOC | **HP** | **Mixed NSIP** | |
| 9 | 0.17 | NonUIP | CIF,NOC | Definite UIP | Mixed NSIP | |
| 10 | 0.36 | NonUIP | Non diagnostic | Probable UIP | Other | Other |
| 11 | 0.56 | **NonUIP** | Non diagnostic | **HP** | **HP** | Non-diagnostic |
| 12 | 0.74 | NonUIP | Non diagnostic | | Probable IPF | Other |
| 13 | 0.87 | NonUIP | CIF,NOC | Definite UIP | Probable IPF | CIF,NOC |
| 14 | 0.98 | UIP | Non diagnostic | RB | Other | Non-diagnostic |
| 15 | 0.98 | **UIP** | CIF,NOC | HP | **Probable IPF** | |
| 16 | 1.26 | UIP | Non diagnostic | | | Other |
| 17 | 1.39 | **UIP** | Non diagnostic | **Possible UIP** | Favor NSIP | |
| 18 | 1.42 | **UIP** | Non diagnostic | **Definite UIP** | | |
| 19 | 2.59 | **UIP** | Non diagnostic | | **Definite IPF** | |

Of the 190 genes used by the Envisia Genomic Classifier, 124 are among the top 1000 genes differentially expressed between UIP and non-UIP TBBs. The classifier features and genes upregulated in UIP are enriched for members of four biological pathways, three of which were previously identified in SLB using a microarray gene expression platform¹³ (Table 12).

**Table 12: Pathway enrichment analysis of 389 genes up-regulated in UIP in TBBs and 92 Envisia Genomic Classifier genes (55 genes are common to both sets). Pathways marked in bold are significantly enriched in surgical lung biopsies (Kim SY et al, 2015).**

| Category | Number of genes expected | Number of genes observed | P-value (corrected) | Direction of enrichment |
|---|---|---|---|---|
| **Dilated cardiomyopathy** | 1.9 | 11 | 0.000126 | More pathway genes than expected |
| **Hypertrophic cardiomyopathy (HCM)** | 1.7 | 9 | 0.003087 | More pathway genes than expected |
| **Focal adhesion** | 4.1 | 13 | 0.012032 | More pathway genes than expected |
| Neuroactive ligand-receptor interaction | 5.5 | 15 | 0.021914 | More pathway genes than expected |

The KEGG dilated and hypertrophic cardiomyopathy networks include genes involved in extracellular matrix interactions, growth factor response, and cytoskeletal remodeling, all reported to be upregulated in IPF^{18,19}.

Similarly, features and genes upregulated in non-UIP TBBs are enriched for multiple pathways also upregulated in non-UIP SLB, including immune response, cell-cell signaling and developmental pathways (Table 13). Differential upregulation of cell proliferation, immune response genes has been shown for HP in comparison to IPF²⁰, although some genes are co-regulated in these diseases²¹.

**Table 13: Pathway enrichment analysis of 611 genes up-regulated in non-UIP in TBBs and 98 Envisia Genomic Classifier genes (69 genes are common to both sets). Pathways marked in bold are significantly enriched in surgical lung biopsies (Kim SY et al, 2015).**

| Category | Number of genes expected | | Number of genes observed | P-value (corrected) | Direction of enrichment |
|---|---|---|---|---|---|
| **Antigen processing and presentation** | | 3.6 | 22 | 4.05E-10 | More pathway genes than expected |
| **Leishmaniasis** | | 3.4 | 20 | 7.47E-09 | More pathway genes than expected |
| Graft-versus-host disease | | 2.0 | 15 | 4.75E-08 | More pathway genes than expected |
| **Type I diabetes mellitus** | | 2.1 | 15 | 9.94E-08 | More pathway genes than expected |
| **Allograft rejection** | | 1.8 | 14 | 1.22E-07 | More pathway genes than expected |
| **Viral myocarditis** | | 3.5 | 18 | 5.90E-07 | More pathway genes than expected |
| **Toll-like receptor signaling pathway** | | 4.8 | 21 | 7.52E-07 | More pathway genes than expected |
| **Autoimmune thyroid disease** | | 2.5 | 14 | 1.39E-05 | More pathway genes than expected |
| **Phagosome** | | 7.4 | 23 | 0.000118 | More pathway genes than expected |
| Olfactory transduction | | 18.1 | 2 | 0.000145 | Fewer pathway genes than expected |
| Cytokine-cytokine receptor interaction | | 12.4 | 29 | 0.001704 | More pathway genes than expected |
| Chagas disease | | 4.8 | 16 | 0.002929 | More pathway genes than expected |
| **Cell adhesion molecules (CAMs)** | | 6.3 | 18 | 0.006574 | More pathway genes than expected |
| NOD-like receptor signaling pathway | | 2.9 | 11 | 0.015921 | More pathway genes than expected |
| Chemokine signaling pathway | | 8.8 | 21 | 0.023401 | More pathway genes than expected |

### Discussion

It is common for the combination of the clinical context and radiologic pattern seen on HRCT scan of the chest to fail to provide a confident diagnosis in patients undergoing evaluation for ILD. While a histopathologic pattern diagnosis from SLB may provide a definitive diagnosis in these patients, many patients are unwilling or too ill to undergo a surgical diagnostic procedure. Even in those that do, the challenge associated with the pathologic interpretation of the biopsy findings may leave significant clinical uncertainty. An accurate and available test associated with minimal risk and not dependent on the visual and subjective skill of an experienced pulmonary pathologist to confirm the presence of histologic UIP may be very useful.

The significant challenges in accruing meaningful numbers of patients and samples to support the machine learning efforts needed to develop Envisia mirrors the challenges faced by clinicians when investigating patients with newly diagnosed ILD. Out of 201 subjects accrued through our BRAVE sample collection studies, we identified only 140 subjects with diagnostic histopathology results, despite the use of a panel of expert pulmonary pathologists. This poor yield highlights the challenge of achieving diagnostic pathology with which clinicians in the community are confronted. We trained and locked the Envisia Genomic Classifier using the first 90 subjects, and validated the test using subsequently accrued subjects. This Genomic Classifier for UIP in conventional TBBs showed high performance in both cohorts.

The accuracy of a molecular UIP call among 25 subjects with a histologic UIP pattern that was not predicted by the study site radiologists is high, with 78% of subjects with a UIP histopathologic pattern successfully identified, with no false positives. In this group of subjects, Envisia functions as a true rule-in test that recovers almost 4 in 5 of the UIP histopathologic pattern cases that were unable to be identified by HRCT. Furthermore, this subgroup is enriched with patients with HP, 60% of whom (9 of 15) in the current study had evidence of advanced fibrotic disease. Envisia detected UIP in HP patients with the same 67% sensitivity as UIP was detected overall in the 49 subject validation cohort. The NPV of the Envisia molecular UIP call in subjects with an inconsistent with UIP radiologic pattern diagnosis is >80%, suggesting substantial utility for both positive and negative Envisia test results.

### EXAMPLE 11

### Sample clinical and technical factors and Envisia performance

Envisia test performance shows some correlation to subject clinical and sample technical factors. UIP disease is missed at a higher rate in male subjects and subjects with a history of smoking (Figure 13). Gene expression consistent with alveolar type II cells does not correlate strongly with Envisia test accuracy (Figure 14), suggesting that alveolar sampling is not critical to test performance, consistent with previous observation in a cohort of 90 ILD subjects^{E10}. There is a slight correlation between stronger (more negative) classification scores and higher sample quality, defined by sample size and RNA quality, among non-UIP samples that is not evident in UIP samples (Figure 14).

The various embodiments described above may be combined to provide further embodiments. All of the U.S. patents, U.S. patent application publications, U.S. patent application, foreign patents, foreign patent application and non-patent publications referred to in this specification and/or listed in the Application Data Sheet are incorporated herein by reference, in their entirety. Aspects of the embodiments may be modified, if necessary to employ concepts of the various patents, application and publications to provide yet further embodiments.

These and other changes may be made to the embodiments in light of the above-detailed description. In general, in the following claims, the terms used should not be construed to limit the claims to the specific embodiments disclosed in the specification and the claims, but should be construed to include all possible embodiments along with the full scope of equivalents to which such claims are entitled. Accordingly, the claims are not limited by the disclosure.

Some embodiments described herein relate to a computer storage product with a non-transitory computer-readable medium (also may be referred to as a non-transitory processor-readable medium) having instructions or computer code thereon for performing various computer-implemented operations. The computer-readable medium (or processor-readable medium) is non-transitory in the sense that it does not include transitory propagating signals per se (e.g., a propagating electromagnetic wave carrying information on a transmission medium such as space or a cable). The media and computer code (also may be referred to as code) may be those designed and constructed for the specific purpose or purposes. Examples of non-transitory computer-readable media include, but are not limited to, magnetic storage media such as hard disks, floppy disks, and magnetic tape; optical storage media such as Compact Disc/Digital Video Discs (CD/DVDs), Compact Disc-Read Only Memories (CD-ROMs), and holographic devices; magneto-optical storage media such as optical disks; carrier wave signal processing modules; and hardware devices that are specially configured to store and execute program code, such as Application-Specific Integrated Circuits (ASICs), Programmable Logic Devices (PLDs), Read-Only Memory (ROM) and Random-Access Memory (RAM) devices. Other embodiments described herein relate to a computer program product, which can include, for example, the instructions and/or computer code discussed herein.

Some embodiments and/or methods described herein may be performed by software (executed on hardware), hardware, or a combination thereof. Hardware modules may include, for example, a general-purpose processor, a field programmable gate array (FPGA), and/or an application specific integrated circuit (ASIC). Software modules (executed on hardware) may be expressed in a variety of software languages (e.g., computer code), including C, C++, Java^{™}, Ruby, Visual Basic^{™}, R, and/or other object-oriented, procedural, statistical, or other programming language and development tools. Examples of computer code include, but are not limited to, micro-code or micro-instructions, machine instructions, such as produced by a compiler, code used to produce a web service, and files containing higher-level instructions that are executed by a computer using an interpreter. For example, embodiments may be implemented using imperative programming languages (e.g., C, FORTRAN, etc.), functional programming languages (e.g., Haskell, Erlang, etc.), logical programming languages (e.g., Prolog), object-oriented programming languages (e.g., Java, C++, etc.), statistical programming languages and/or environments (e.g., R, etc.) or other suitable programming languages and/or development tools. Additional examples of computer code include, but are not limited to, control signals, encrypted code, and compressed code.

### REFERENCES.

*All of the following references and all references cited herein are incorporated herein in their entirety.*
1. Travis WD, Costabel U, Hansell DM, King TE, Lynch DA, Nicholson AG, Ryerson CJ, Ryu JH, Selman M, Wells AU, Behr J, Bouros D, Brown KK, Colby TV, Collard HR, Cordeiro CR, Cottin V, Crestani B, Drent M, Dudden RF, Egan J, Flaherty K, Hogaboam C, Inoue Y, Johkoh T, Kim DS, Kitaichi M, Loyd J, Martinez FJ, Myers J, Protzko S, Raghu G, Richeldi L, Sverzellati N, Swigris J, Valeyre D. An Official American Thoracic Society/European Respiratory Society Statement: Update of the international multidisciplinary classification of the idiopathic interstitial pneumonias. Am J Respir Crit Care Med 2013;188:733-748.
2. Raghu G, Rochwerg B, Zhang Y, Garcia CAC, Azuma A, Behr J, Brozek JL, Collard HR, Cunningham W, Homma S, Johkoh T, Martinez FJ, Myers J, Protzko SL, Richeldi L, Rind D, Selman M, Theodore A, Wells AU, Hoogsteden H, Schünemann HJ. An Official ATS/ERS/JRS/ALAT Clinical Practice Guideline: Treatment of idiopathic pulmonary fibrosis. An update of the 2011 clinical practice guideline. Am J Respir Crit Care Med 2015;192:e3-e19.
3. Bjoraker JA, Ryu JH, Edwin MK, Myers JL, Tazelaar HD, Schroeder DR, Offord KP. Prognostic significance of histopathologic subsets in idiopathic pulmonary fibrosis. Am J Respir Crit Care Med 1998;157:199-203.
4. Flaherty KR, Travis WD, Colby TV, Toews GB, Kazerooni EA, Gross BH, Jain A, Strawderman RL, Flint A, Lynch JP, Martinez FJ. Histopathologic variability in usual and nonspecific interstitial pneumonias. Am J Respir Crit Care Med 2001; 164: 1722-1727.
5. Flaherty KR, Toews GB, Travis WD, Colby TV, Kazerooni EA, Gross BH, Jain A, Strawderman RL, Paine R, Flint A, Lynch JP, Martinez FJ. Clinical significance of histological classification of idiopathic interstitial pneumonia. Eur Respir J 2002;19:275-283.
6. Flaherty K, Thwaite E, Kazerooni E, Gross B, Toews G, Colby T, Travis W, Mumford J, Murray S, Flint A, Lynch J, Martinez F. Radiological versus histological diagnosis in UIP and NSIP: Survival implications. Thorax 2003;58:143-148.
7. Katzenstein A-LA, Mukhopadhyay S, Myers JL. Diagnosis of usual interstitial pneumonia and distinction from other fibrosing interstitial lung diseases. Hum Pathol 2008;39:1275-1294.
8. Raghu G, Collard HR, Egan JJ, Martinez FJ, Behr J, Brown KK, Colby TV, Cordier J-F, Flaherty KR, Lasky JA, Lynch DA, Ryu JH, Swigris JJ, Wells AU, Ancochea J, Bouros D, Carvalho C, Costabel U, Ebina M, Hansell DM, Johkoh T, Kim DS, King TE, Kondoh Y, Myers J, Müller NL, Nicholson AG, Richeldi L, Selman M, Dudden RF, Griss BS, Protzko SL, Schünemann HJ. An Official ATS/ERS/JRS/ALAT Statement: Idiopathic Pulmonary Fibrosis: Evidence-based guidelines for diagnosis and management. Am J Respir Crit Care Med 2011;183:788-824.
9. American Thoracic S, European Respiratory S. American Thoracic Society/European Respiratory Society International Multidisciplinary Consensus Classification of the Idiopathic Interstitial Pneumonias. This joint statement of the American Thoracic Society (ATS) and the European Respiratory Society (ERS) was adopted by the ATS board of directors, June 2001 and by the ERS Executive Committee, June 2001. Am J Respir Crit Care Med 2002;165:277-304.
10. Katzenstein A-LA, Myers JL. Idiopathic Pulmonary Fibrosis. Am J Respir Crit Care Med 1998;157:1301-1315.
11. Berbescu EA, Katzenstein A-LA, Snow JL, Zisman DA. Transbronchial biopsy in usual interstitial pneumonia. Chest 2006;129:1126-1131.
12. Tomassetti S, Cavazza A, Colby TV, Ryu JH, Nanni O, Scarpi E, Tantalocco P, Buccioli M, Dubini A, Piciucchi S, Ravaglia C, Gurioli C, Casoni GL, Gurioli C, Romagnoli M, Poletti V. Transbronchial biopsy is useful in predicting UIP pattern. Respir Res 2012;13:96-96.
13. Shim HS, Park MS, Park IK. Histopathologic findings of transbronchial biopsy in usual interstitial pneumonia. Pathol Int 2010;60:373-377.
14. Tomassetti S, Wells AU, Costabel U, Cavazza A, Colby TV, Rossi G, Sverzellati N, Carloni A, Carretta E, Buccioli M, Tantalocco P, Ravaglia C, Gurioli C, Dubini A, Piciucchi S, Ryu JH, Poletti V. Bronchoscopic lung cryobiopsy increases diagnostic confidence in the multidisciplinary diagnosis of idiopathic pulmonary fibrosis. Am J Respir Crit Care Med 2016;193:745-752.
15. Dhooria S, Sehgal IS, Aggarwal AN, Behera D, Agarwal R. Diagnostic yield and safety of cryoprobe transbronchial lung biopsy in diffuse parenchymal lung diseases: Systematic review and meta-analysis. Respir Care 2016;61:700-712.
16. Poletti V, Ravaglia C, Gurioli C, Piciucchi S, Dubini A, Cavazza A, Chilosi M, Rossi A, Tomassetti S. Invasive diagnostic techniques in idiopathic interstitial pneumonias. Respirology 2016;21:44-50.
17. Kim SY, Diggans J, Pankratz D, Huang J, Pagan M, Sindy N, Tom E, Anderson J, Choi Y, Lynch DA, Steele MP, Flaherty KR, Brown KK, Farah H, Bukstein MJ, Pardo A, Selman M, Wolters PJ, Nathan SD, Colby TV, Myers JL, Katzenstein A-LA, Raghu G, Kennedy GC. Classification of usual interstitial pneumonia in patients with interstitial lung disease: Assessment of a machine learning approach using high-dimensional transcriptional data. Lancet Respir Med 2015;3:473-482.
18. Friedman J, Hastie T, Tibshirani R. Regularization paths for generalized linear models via coordinate descent. J Stat Softw 2010;33:1-22.
19. Love MI, Huber W, Anders S. Moderated estimation of fold change and dispersion for RNA-seq data with DESeq2. Genome Biol 2014;15:550.
20. Mi H, Lazareva-Ulitsky B, Loo R, Kejariwal A, Vandergriff J, Rabkin S, Guo N, Muruganujan A, Doremieux O, Campbell MJ, Kitano H, Thomas PD. The PANTHER database of protein families, subfamilies, functions and pathways. Nucleic Acids Res 2005;33:D284-D288.
21. Katzenstein A-LA, Zisman DA, Litzky LA, Nguyen BT, Kotloff RM. Usual interstitial pneumonia: Histologic study of biopsy and explant specimens. Am J Surg Pathol 2002;26:1567-1577.
22. Trahan S, Hanak V, Ryu JH, Myers JL. Role of surgical lung biopsy in separating chronic hypersensitivity pneumonia from usual interstitial pneumonia/idiopathic pulmonary fibrosis: Analysis of 31 biopsies from 15 patients. Chest 2008; 134: 126-132.
23. Akashi T, Takemura T, Ando N, Eishi Y, Kitagawa M, Takizawa T, Koike M, Ohtani Y, Miyazaki Y, Inase N, Yoshizawa Y. Histopathologic analysis of sixteen autopsy cases of chronic hypersensitivity pneumonitis and comparison with idiopathic pulmonary fibrosis/usual interstitial pneumonia. Am J Clin Pathol 2009;131:405-415.
24. Selman M, Pardo A, Barrera L, Estrada A, Watson SR, Wilson K, Aziz N, Kaminski N, Zlotnik A. Gene expression profiles distinguish idiopathic pulmonary fibrosis from hypersensitivity pneumonitis. Am J Respir Crit Care Med 2006;173:188-198.
25. Lockstone HE, Sanderson S, Kulakova N, Baban D, Leonard A, Kok WL, McGowan S, McMichael AJ, Ho LP. Gene set analysis of lung samples provides insight into pathogenesis of progressive, fibrotic pulmonary sarcoidosis. Am J Respir Crit Care Med 2010; 181: 1367-1375.
26. Selman M, Pardo A. Revealing the pathogenic and aging-related mechanisms of the enigmatic idiopathic pulmonary fibrosis. An integral model. Am J Respir Crit Care Med 2014;189:1161-1172.
27. Bauer Y, Tedrow J, de Bernard S, Birker-Robaczewska M, Gibson KF, Guardela BJ, Hess P, Klenk A, Lindell KO, Poirey S, Renault B, Rey M, Weber E, Nayler O, Kaminski N. A novel genomic signature with translational significance for human idiopathic pulmonary fibrosis. Am J Respir Cell Mol Biol 2015;52:217-231.
28. Jonigk D, Izykowski N, Rische J, Braubach P, Kuhnel M, Warnecke G, Lippmann T, Kreipe H, Haverich A, Welte T, Gottlieb J, Laenger F. Molecular profiling in lung biopsies of human pulmonary allografts to predict chronic lung allograft dysfunction. Am J Pathol 2015;185:3178-3188.
29. Nicholson AG, Fulford LG, Colby TV, du Bois RM, Hansell DM, Wells AU. The relationship between individual histologic features and disease progression in idiopathic pulmonary fibrosis. Am J Respir Crit Care Med 2002;166:173-177.
30. Walsh SL, Wells AU, Desai SR, Poletti V, Piciucchi S, Dubini A, Nunes H, Valeyre D, Brillet PY, Kambouchner M, Morais A, Pereira JM, Moura CS, Grutters JC, van den Heuvel DA, van Es HW, van Oosterhout MF, Seldenrijk CA, Bendstrup E, Rasmussen F, Madsen LB, Gooptu B, Pomplun S, Taniguchi H, Fukuoka J, Johkoh T, Nicholson AG, Sayer C, Edmunds L, Jacob J, Kokosi MA, Myers JL, Flaherty KR, Hansell DM. Multicentre evaluation of multidisciplinary team meeting agreement on diagnosis in diffuse parenchymal lung disease: A case-cohort study. Lancet Respir Med 2016;4:557-565.
31. Flaherty KR, King TE, Raghu G, Lynch JP, Colby TV, Travis WD, Gross BH, Kazerooni EA, Toews GB, Long Q, Murray S, Lama VN, Gay SE, Martinez FJ. Idiopathic Interstitial Pneumonia. Am J Respir Crit Care Med 2004;170:904-910.
32. Tominaga J, Sakai F, Johkoh T, Noma S, Akira M, Fujimoto K, Colby TV, Ogura T, Inoue Y, Taniguchi H, Homma S, Taguchi Y, Sugiyama Y. Diagnostic certainty of idiopathic pulmonary fibrosis/usual interstitial pneumonia: The effect of the integrated clinico-radiological assessment. Eur J Radiol 2015;84:2640-2645.
33. The Idiopathic Pulmonary Fibrosis Clinical Research Network. Prednisone, azathioprine, and n-acetylcysteine for pulmonary fibrosis. N Engl J Med 2012;366:1968-77.
34. Sumikawa H, Johkoh T, Colby TV, Ichikado K, Suga M, Taniguchi H, Kondoh Y, Ogura T, Arakawa H, Fujimoto K, Inoue A, Mihara N, Honda O, Tomiyama N, Nakamura H, Muller NL. Computed tomography findings in pathological usual interstitial pneumonia. Am J Respir Crit Care Med 2008;177:433-439.
35. Chung JH, Chawla A, Peljto AL, Cool CD, Groshong SD, Talbert JL, McKean DF, Brown KK, Fingerlin TE, Schwarz MI, Schwarz DA, Lynch DA. CT scan findings of probable usual interstitial pneumonitis have a high predictive value for histologic usual interstitial pneumonitis. Chest 2015;147:450-459.
36. Brownell R, Moua T, Henry TS, Elicker BM, White D, Vittinghoff E, Jones KD, Urisman A, Aravena C, Johannson KA, Golden JA, King TE Jr, Wolters PJ. Collard HR, Ley B. The use of pretest probability increases the value of high-resolution CT in diagnosing usual interstitial pneumonia. Thorax 2017;72(5):424-429.
37. DiBardino DM, Haas AR, Lanfranco AR, Litzky LA, Sterman D, Bessich JL. High complication rate after introduction of transbronchial cryobiopsy into clinical practice at an academic medical center. Annals Am Thorac Soc 2017;14(6):851-857.
38. Hutchinson JP, McKeever TM, Fogarty AW, Navaratnam V, Hubbard RB. Surgical lung biopsy for the diagnosis of interstitial lung disease in England: 1997-2008. Eur Respir J 2016;48:1453-61.

E1. Kim SY, Diggans J, Pankratz D, Huang J, Pagan M, Sindy N, Tom E, Anderson J, Choi Y, Lynch DA, Steele MP, Flaherty KR, Brown KK, Farah H, Bukstein MJ, Pardo A, Selman M, Wolters PJ, Nathan SD, Colby TV, Myers JL, Katzenstein A-LA, Raghu G, Kennedy GC. Classification of usual interstitial pneumonia in patients with interstitial lung disease: assessment of a machine learning approach using high-dimensional transcriptional data. Lancet Respir Med 2015;3:473-482.
E2. Dobin A, Davis CA, Schlesinger F, Drenkow J, Zaleski C, Jha S, Batut P, Chaisson M, Gingeras TR. STAR: ultrafast universal RNA-seq aligner. Bioinformatics 2012.
E3. Anders S, Pyl PT, Huber W. HTSeq-a Python framework to work with high-throughput sequencing data. Bioinformatics 2015;31:166-169.
E4. DeLuca DS, Levin JZ, Sivachenko A, Fennell T, Nazaire M-D, Williams C, Reich M, Winckler W, Getz G. RNA-SeQC: RNA-seq metrics for quality control and process optimization. Bioinformatics 2012;28:1530-1532.
E5. Wuenschell CW, Sunday ME, Singh G, Minoo P, Slavkin HC, Warburton D. Embryonic mouse lung epithelial progenitor cells co-express immunohistochemical markers of diverse mature cell lineages. J Histochem Cytochem 1996;44:113-123.
E6. Nielsen S, King LS, Christensen BM, Agre P. Aquaporins in complex tissues. II. Subcellular distribution in respiratory and glandular tissues of rat. Am J Physiol 1997;273:C1549-1561.
E7. Kim CF, Jackson EL, Woolfenden AE, Lawrence S, Babar I, Vogel S, Crowley D, Bronson RT, Jacks T. Identification of bronchioalveolar stem cells in normal lung and lung cancer. Cell 2005;121:823-835.
E8. Zemke AC, Snyder JC, Brockway BL, Drake JA, Reynolds SD, Kaminski N, Stripp BR. Molecular staging of epithelial maturation using secretory cell-specific genes as markers. Am J Respir Cell Mol Biol 2009;40:340-348.
E9. Treutlein B, Brownfield DG, Wu AR, Neff NF, Mantalas GL, Espinoza FH, Desai TJ, Krasnow MA, Quake SR. Reconstructing lineage hierarchies of the distal lung epithelium using single-cell RNA-seq. Nature 2014;509:371-375.
E10. Pankratz DG, Choi Y, Imtiaz U, Fedorowicz GM, Anderson JD, Colby TV, Myers JL, Lynch DA, Brown KK, Flaherty KR, Steele MP, Groshong SD, Raghu G, Barth NM, Walsh PS, Huang J, Kennedy GC, Martinez FJ. Usual interstital pneumonia can be detected in transbronchial biopsies using machine learning. Annals Am Thorac Soc 2017.

All of the above U.S. patents, U.S. patent application publications, U.S. patent applications, foreign patents, foreign patent applications and non-patent publications referred to in this specification and/or listed in the Application Data Sheet or in the above REFERENCES list, are incorporated herein by reference, in their entirety.

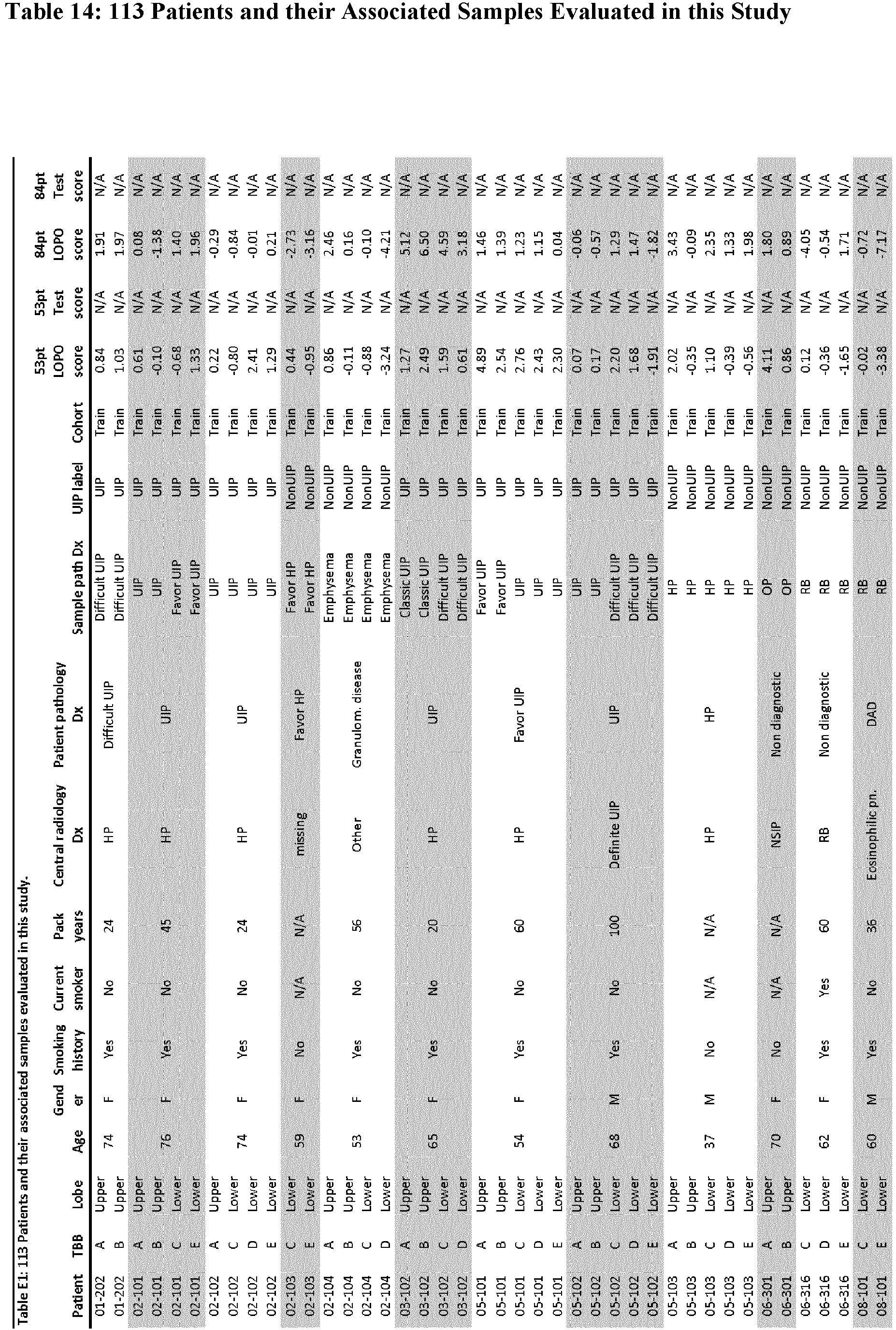

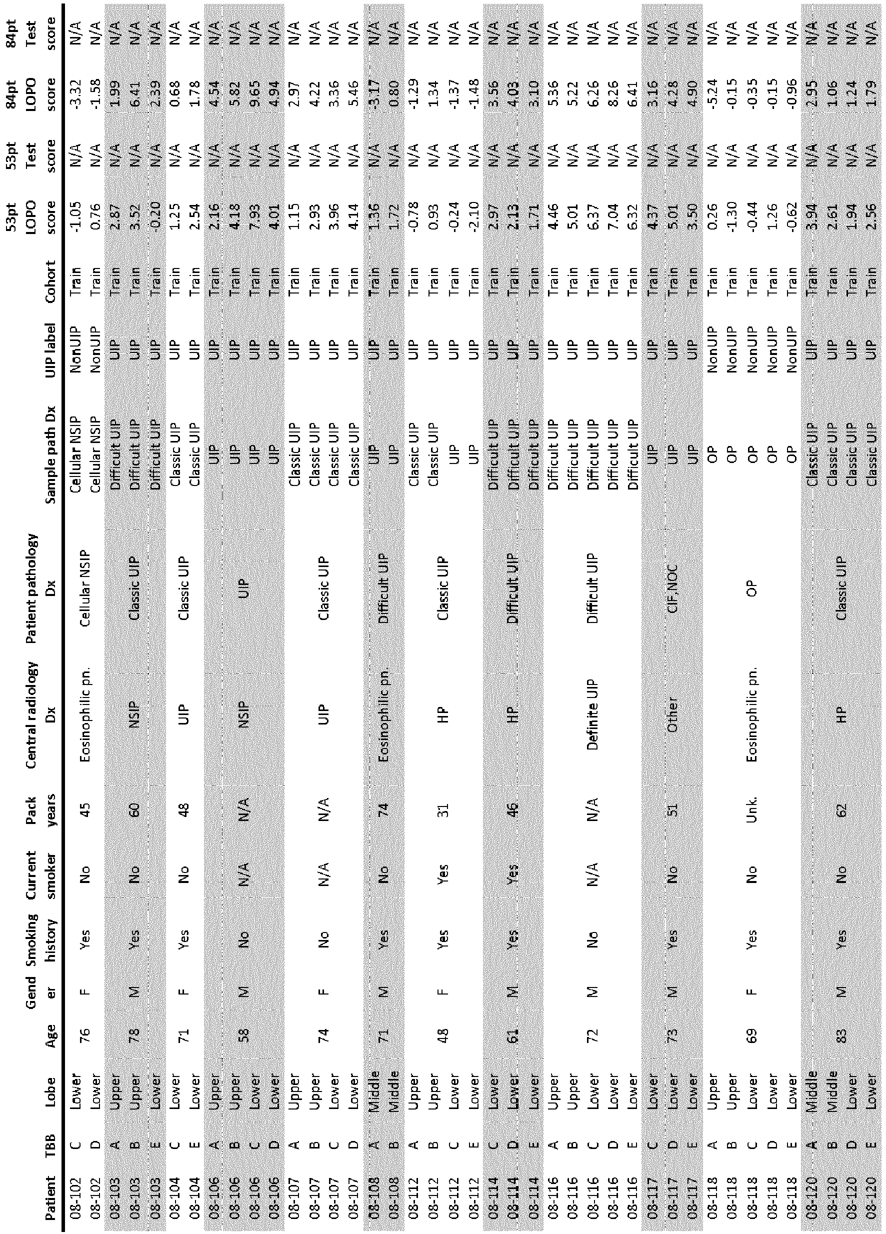

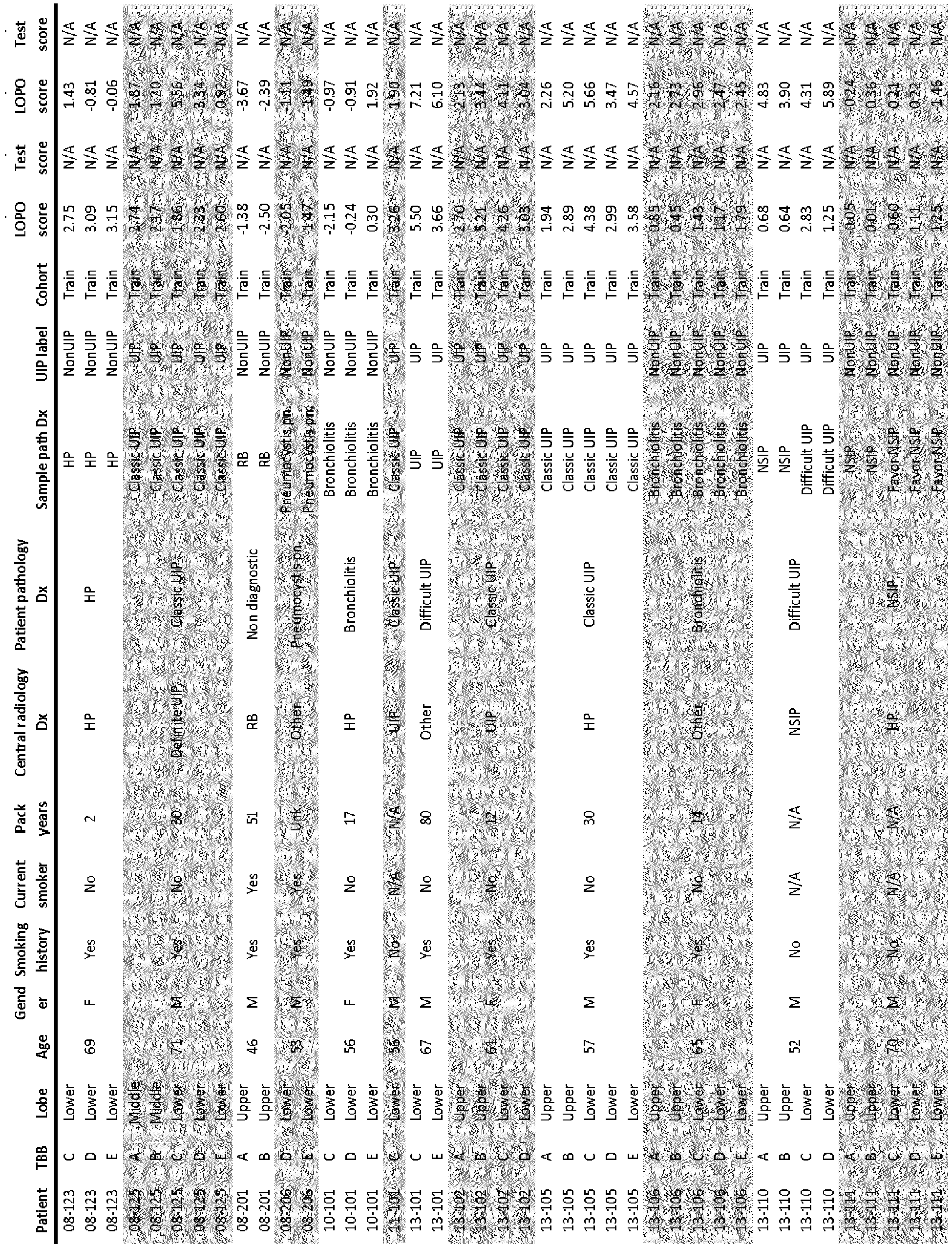

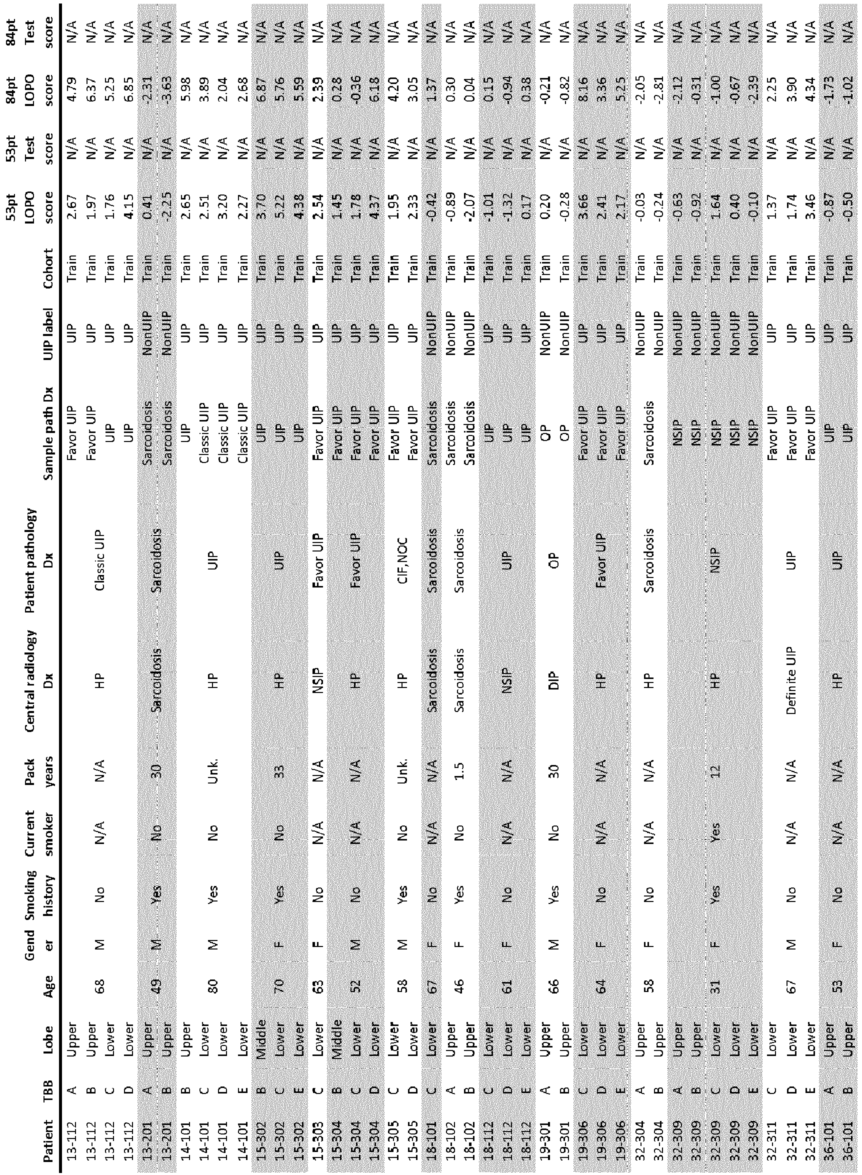

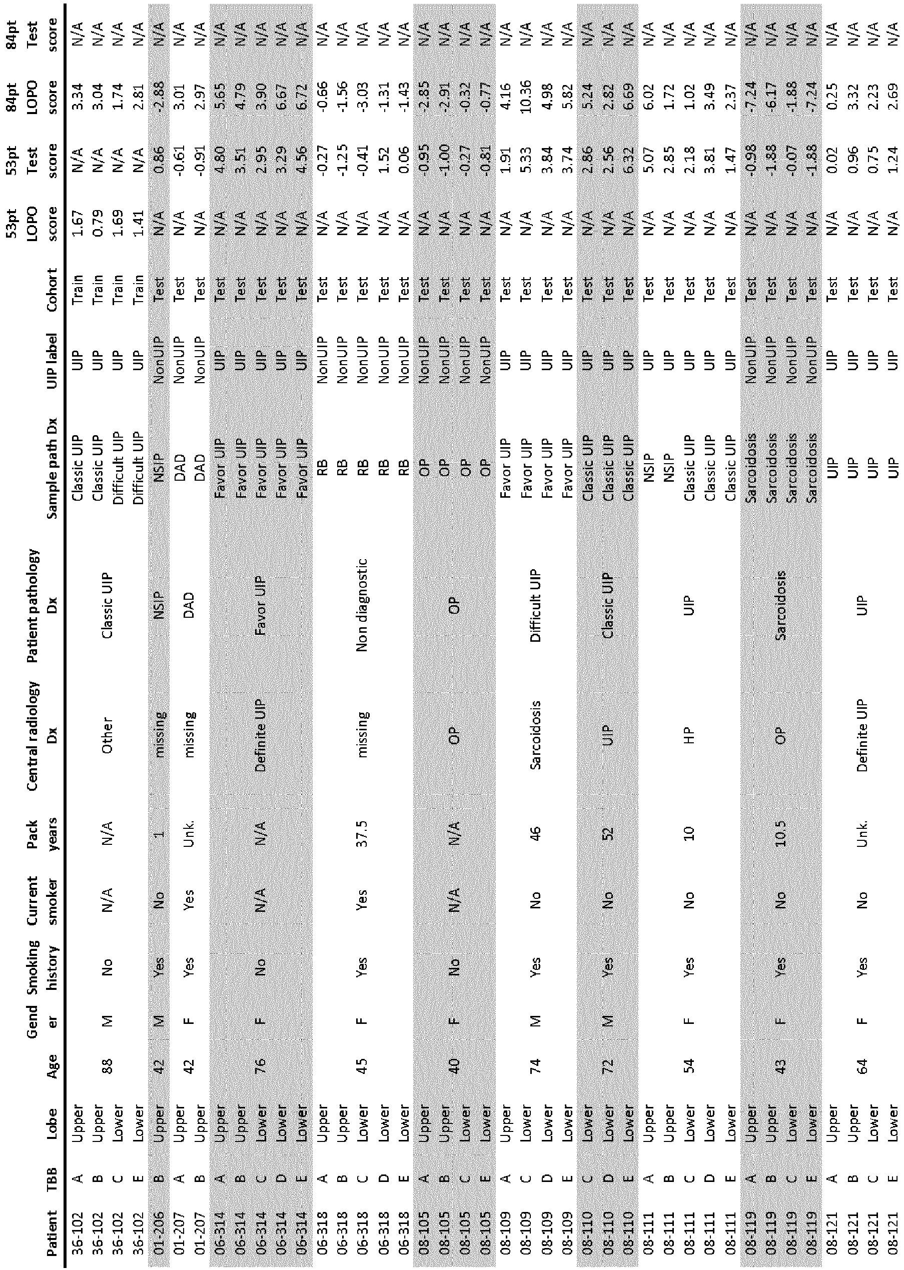

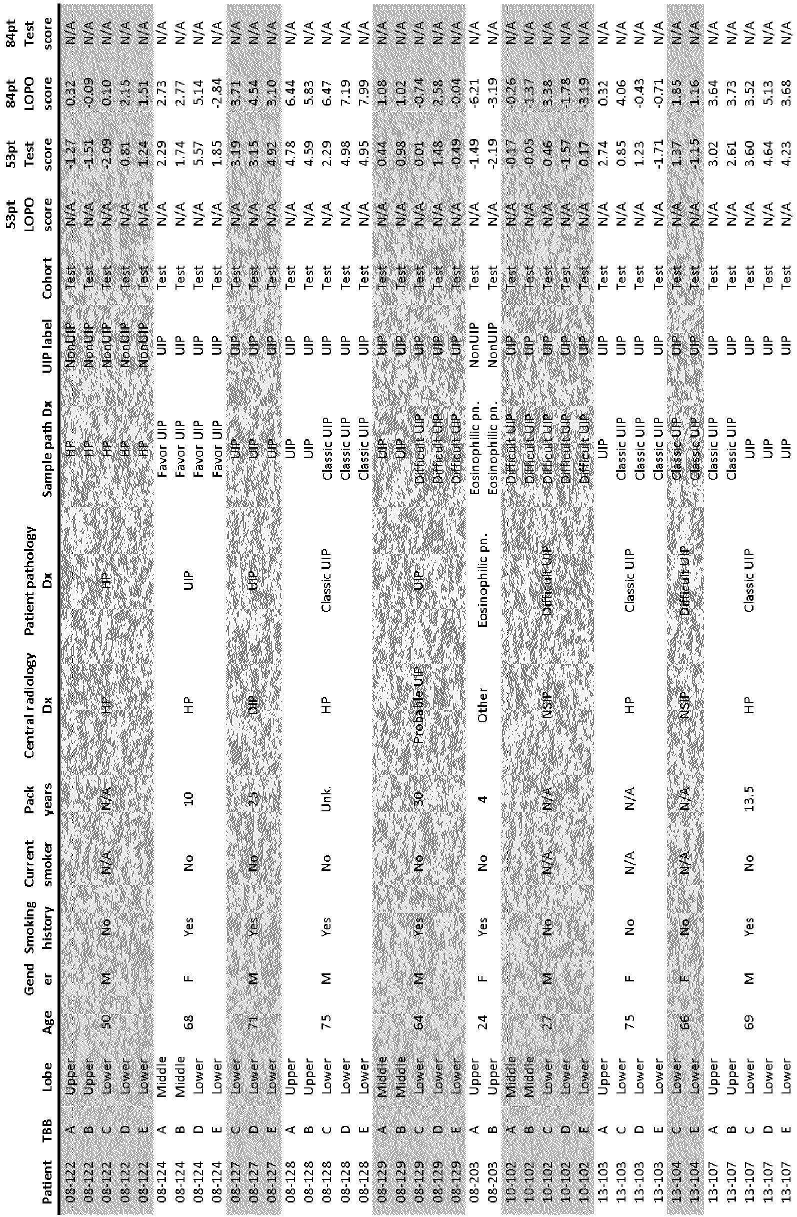

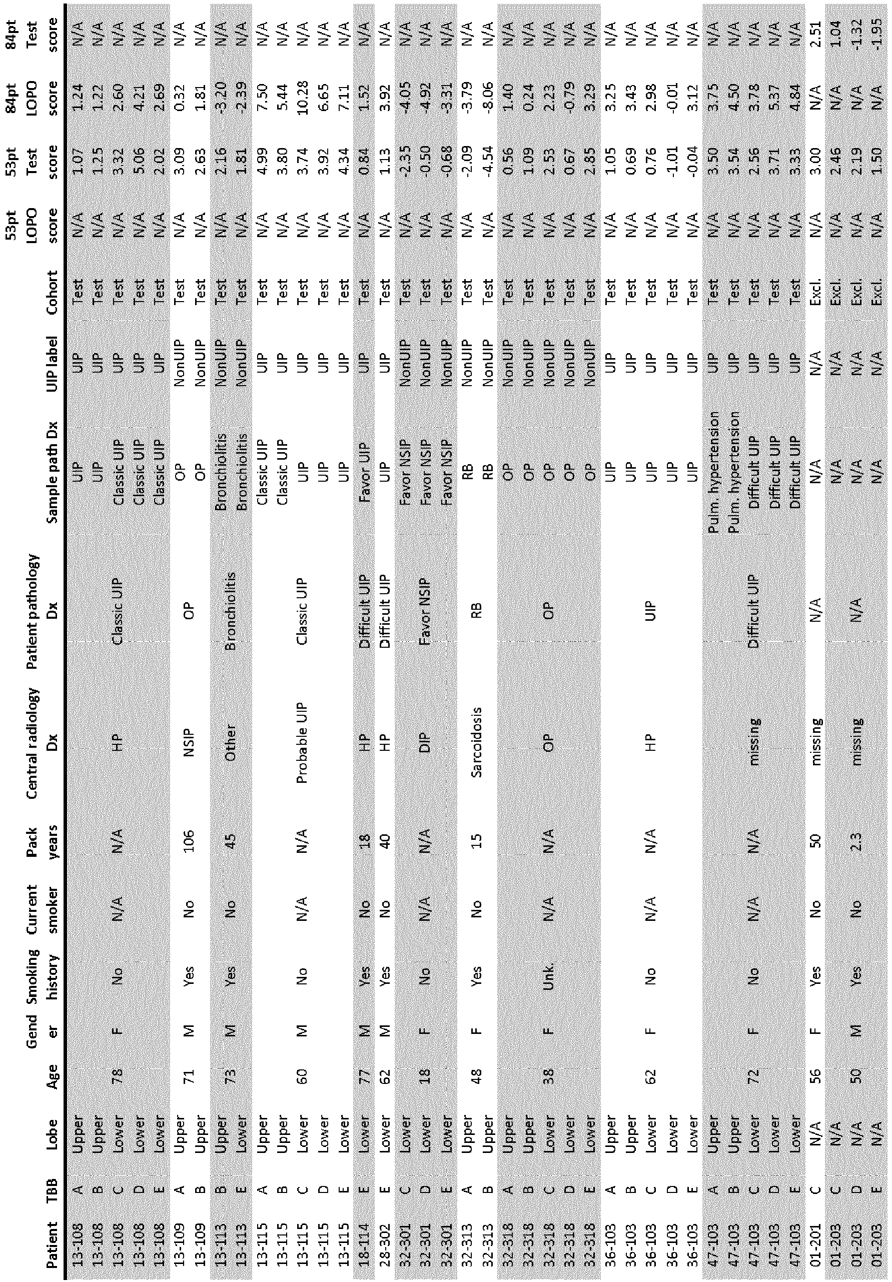

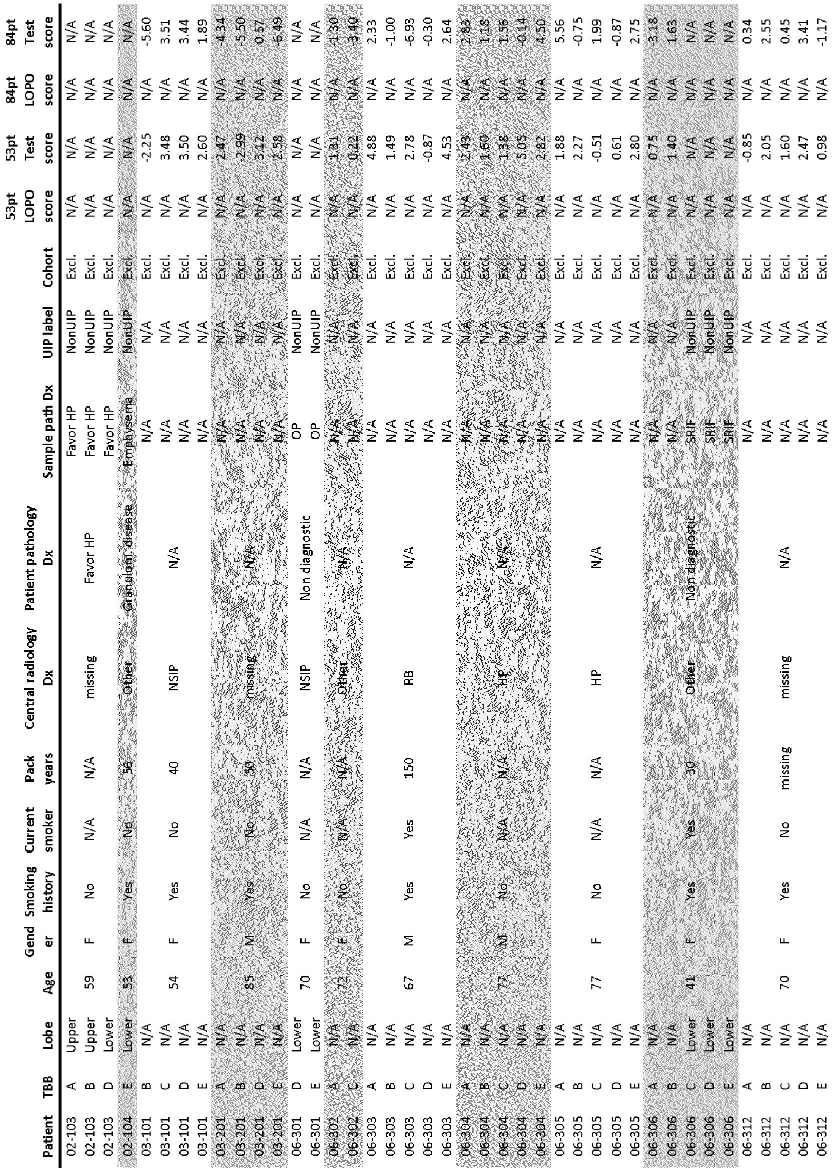

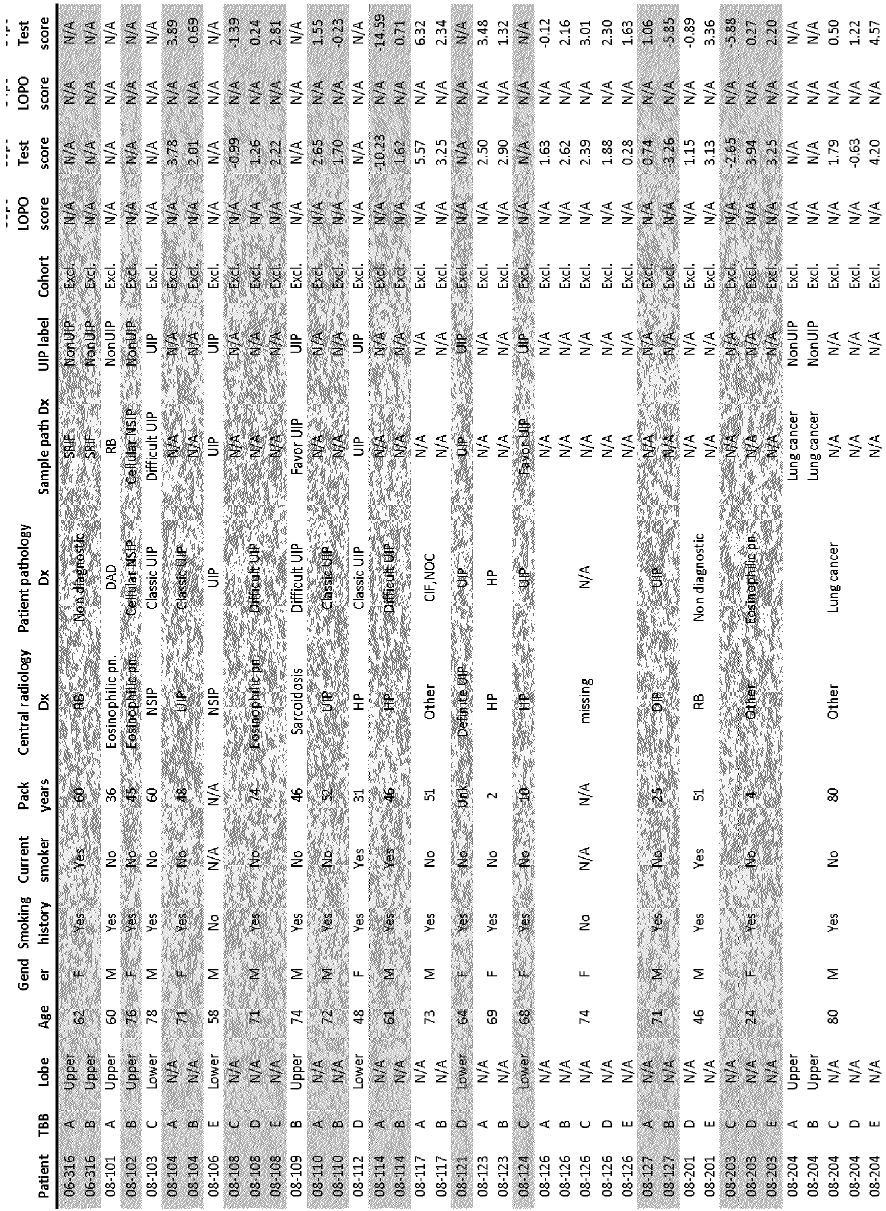

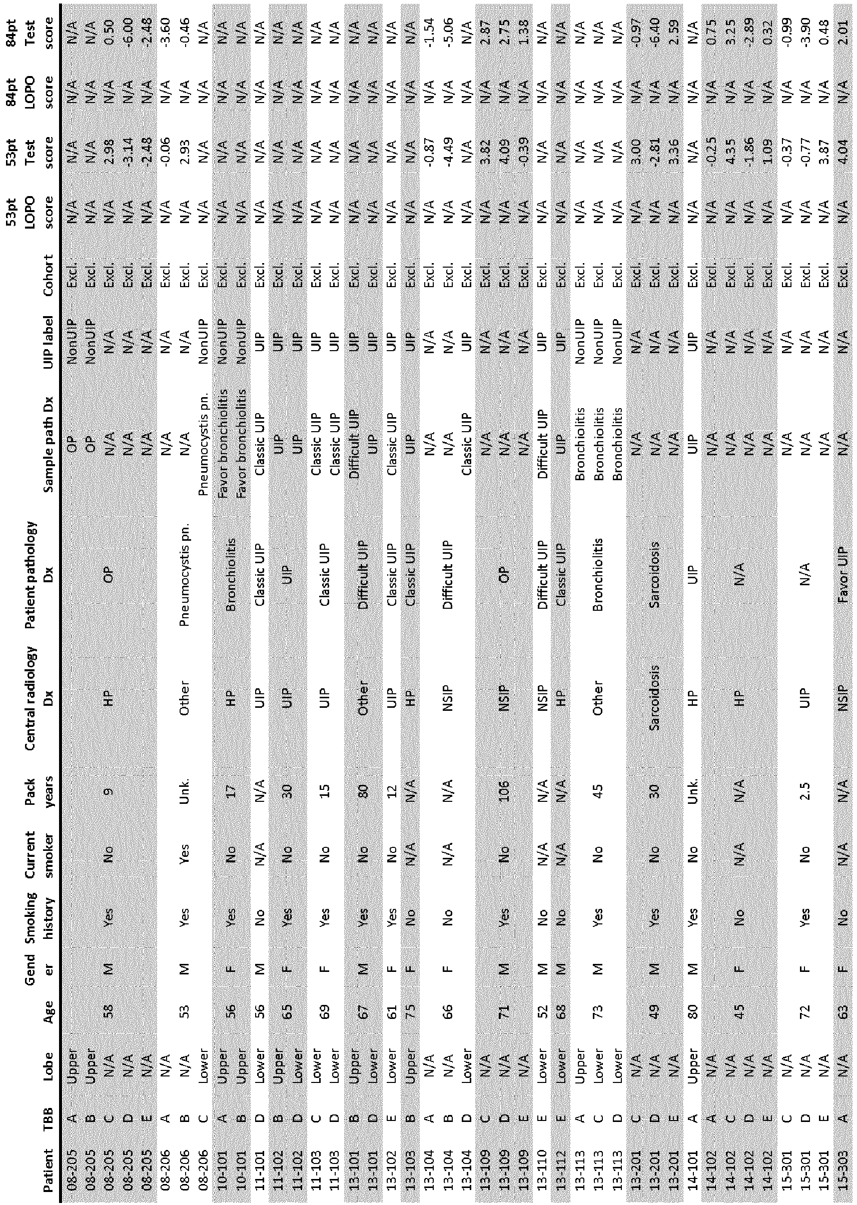

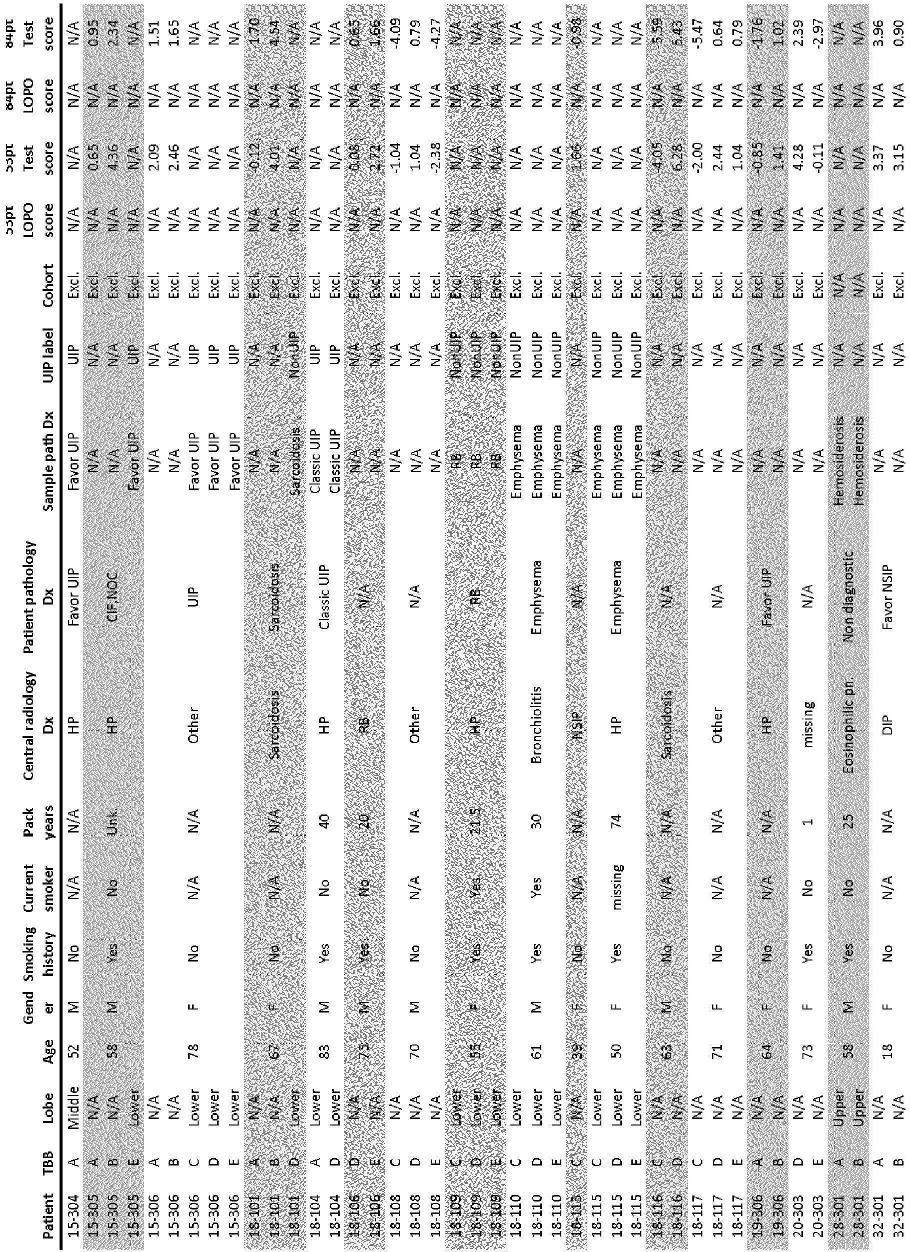

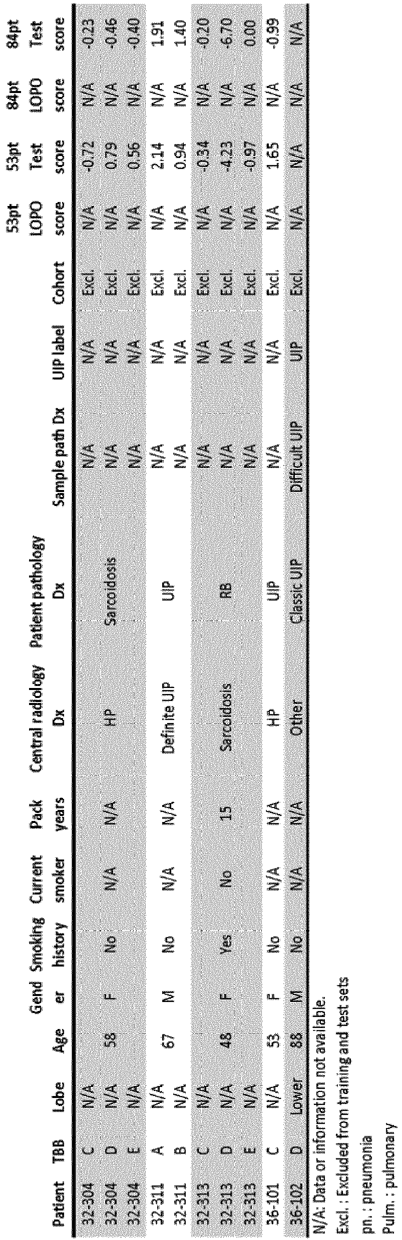

**Table 15: 169 Ensembl Gene IDs used in classification (53-patient classifier).**

| **SEQ ID NO** | **gene_id** | **gene_biotype** | **SEQ ID NO** | **gene_id** | **gene_biotype** | **SEQ ID** | **gene_id** | **gene_biotype** |
|---|---|---|---|---|---|---|---|---|
| 152. | ENSG00000189339 | prot_coding | 153. | ENSG00000105991 | prot_coding | 154. | ENSG00000248713 | prot_coding |
| 155. | ENSG00000116285 | prot_coding | 156. | ENSG00000136275 | prot_coding | 157. | ENSG00000138795 | prot_coding |
| 158. | ENSG00000219481 | prot_coding | 159. | ENSG00000146707 | prot_coding | 160. | ENSG00000172399 | prot_coding |
| 161. | ENSG00000204219 | prot_coding | 162. | ENSG00000221305 | miRNA | 163. | ENSG00000109471 | prot_coding |
| 164. | ENSG00000142661 | prot_coding | 165. | ENSG00000012232 | prot_coding | 166. | ENSG00000151005 | prot_coding |
| 167. | ENSG00000157131 | prot_coding | 168. | ENSG00000104381 | prot_coding | 169. | ENSG00000145736 | prot_coding |
| 170. | ENSG00000116761 | prot_coding | 171. | ENSG00000204844 | lineRNA | 172. | ENSG00000168938 | prot_coding |
| 173. | ENSG00000134245 | prot_coding | 174. | ENSG00000136928 | prot_coding | 175. | ENSG00000169194 | prot_coding |
| 176. | ENSG00000122497 | prot_coding | 177. | ENSG00000136881 | prot_coding | 178. | ENSG00000113621 | prot_coding |
| 179. | ENSG00000159164 | prot_coding | 180. | ENSG00000136883 | prot_coding | 181. | ENSG00000253910 | prot_coding |
| 182. | ENSG00000232671 | prot_coding | 183. | ENSG00000148200 | prot_coding | 184. | ENSG00000261934 | prot_coding |
| 185. | ENSG00000143367 | prot_coding | 186. | ENSG00000148339 | prot_coding | 187. | ENSG00000145888 | prot_coding |
| 188. | ENSG00000143320 | prot_coding | 189. | ENSG00000176919 | prot_coding | 190. | ENSG00000055163 | prot_coding |
| 191. | ENSG00000143195 | prot_coding | 192. | ENSG00000107929 | prot_coding | 193. | ENSG00000184845 | prot_coding |
| 194. | ENSG00000007908 | prot_coding | 195. | ENSG00000207937 | miRNA | 196. | ENSG00000234284 | prot_coding |
| 197. | ENSG00000171806 | prot_coding | 198. | ENSG00000188234 | prot_coding | 199. | ENSG00000198518 | prot_coding |
| 200. | ENSG00000007933 | prot_coding | 201. | ENSG00000148541 | prot_coding | 202. | ENSG00000261839 | lincRNA |
| 203. | ENSG00000162782 | prot_coding | 204. | ENSG00000204020 | prot_coding | 205. | ENSG00000235109 | prot_coding |
| 206. | ENSG00000177489 | prot_coding | 207. | ENSG00000148702 | prot_coding | 208. | ENSG00000204701 | prot_coding |
| 209. | ENSG00000138075 | prot_coding | 210. | ENSG00000149043 | prot_coding | 211. | ENSG00000204632 | prot_coding |
| 212. | ENSG00000135625 | prot_coding | 213. | ENSG00000130598 | prot_coding | 214. | ENSG00000204110 | IincRNA |
| 215. | ENSG00000115317 | prot_coding | 216. | ENSG00000171987 | prot_coding | 217. | ENSG00000124641 | prot_coding |
| 218. | ENSG00000183281 | prot_coding | 219. | ENSG00000166796 | prot_coding | 220. | ENSG00000124702 | prot_coding |
| 221. | ENSG00000144057 | prot_coding | 222. | ENSG00000183908 | prot_coding | 223. | ENSG00000112818 | prot_coding |
| 224. | ENSG00000257207 | prot_coding | 225. | ENSG00000166004 | prot_coding | 226. | ENSG00000174156 | prot_coding |
| 227. | ENSG00000144320 | prot_coding | 228. | ENSG00000183560 | prot_coding | 229. | ENSG00000118402 | prot_coding |
| 230. | ENSG00000188282 | prot_coding | 231. | ENSG00000149289 | prot_coding | 232. | ENSG00000112299 | prot_coding |
| 233. | ENSG00000074582 | prot_coding | 284. | ENSG00000254842 | lineRNA | 235. | ENSG00000048052 | prot_coding |
| 236. | ENSG00000054356 | prot_coding | 287. | ENSG00000010379 | prot_coding | 238. | ENSG00000129204 | prot_coding |
| 239. | ENSG00000114923 | prot_coding | 240. | ENSG00000111321 | prot_coding | 241. | ENSG00000129221 | prot_coding |
| 242. | ENSG00000115009 | prot_coding | 243. | ENSG00000212126 | prot_coding | 244. | ENSG00000108551 | prot_coding |
| 245. | ENSG00000181798 | Proces'd_transc | 246. | ENSG00000110900 | prot_coding | 247. | ENSG00000108342 | prot_coding |
| 248. | ENSG00000144712 | prot_coding | 249. | ENSG00000139211 | prot_coding | 250. | ENSG00000131095 | prot_coding |
| 251. | ENSG00000168329 | prot_coding | 252. | ENSG00000187166 | prot_coding | 253. | ENSG00000167105 | prot_coding |
| 254. | ENSG00000168036 | prot_coding | 255. | ENSG00000086159 | prot_coding | 256. | ENSG00000258890 | prot_coding |
| 257. | ENSG00000179152 | prot_coding | 258. | ENSG00000170374 | prot_coding | 259. | ENSG00000141562 | prot_coding |

| **SEQ ID NO** | **gene_id** | **gene_biotype** | **SEQ ID NO** | **gene_id** | **gene_biotype** | **SEQ ID NO** | **gene_id** | **gene_biotype** |
|---|---|---|---|---|---|---|---|---|
| 260. | ENSG00000256097 | prot_coding | 261. | ENSG00000221479 | miRNA | 262. | ENSG00000128791 | prot_coding |
| 263. | ENSG00000227124 | prot_coding | 264. | ENSG00000139352 | prot_coding | 265. | ENSG00000170558 | prot_coding |
| 266. | ENSG00000184500 | prot_coding | 267. | ENSG00000122966 | prot_coding | 268. | ENSG00000075643 | prot_coding |
| 269. | ENSG00000206531 | prot_coding | 270. | ENSG00000125255 | prot_coding | 271. | ENSG00000166573 | prot_coding |
| 272. | ENSG00000163884 | prot_coding | 273. | ENSG00000134905 | prot_coding | 274. | ENSG00000256463 | prot_coding |
| 275. | ENSG00000180697 | prot_coding | 276. | ENSG00000187630 | prot_coding | 277. | ENSG00000125827 | prot_coding |
| 278. | ENSG00000198685 | prot_coding | 279. | ENSG00000257365 | prot_coding | 280. | ENSG00000182931 | prot_coding |
| 281. | ENSG00000034533 | prot_coding | 282. | ENSG00000133997 | prot_coding | 283. | ENSG00000198768 | prot_coding |
| 284. | ENSG00000172667 | prot_coding | 285. | ENSG00000119725 | prot_coding | 286. | ENSG00000101188 | prot_coding |
| 287. | ENSG00000078070 | prot_coding | 288. | ENSG00000198208 | prot_coding | 289. | ENSG00000131142 | prot_coding |
| 290. | ENSG00000159674 | prot_coding | 291. | ENSG00000258945 | prot_coding | 292. | ENSG00000086544 | prot_coding |
| 293. | ENSG00000174123 | prot_coding | 294. | ENSG00000169918 | prot_coding | 295. | ENSG00000188293 | prot_coding |
| 296. | ENSG00000109158 | prot_coding | 297. | ENSG00000198838 | prot_coding | 298. | ENSG00000167748 | prot_coding |
| 299. | ENSG00000145248 | prot_coding | 300. | ENSG00000140323 | prot_coding | 301. | ENSG00000189013 | prot_coding |
| 302. | ENSG00000035720 | prot_coding | 303. | ENSG00000167014 | prot_coding | 304. | ENSG00000022556 | prot_coding |
| 305. | ENSG00000081041 | prot_coding | 306. | ENSG00000137875 | prot_coding | 307. | ENSG00000273311 | sense_intronic |
| 308. | ENSG00000145284 | prot_coding | 309. | ENSG00000067141 | prot_coding | 310. | ENSG00000183066 | prot_coding |
| 311. | ENSG00000170509 | prot_coding | 312. | ENSG00000095917 | prot_coding | 313. | ENSG00000189306 | prot_coding |
| 314. | ENSG00000170502 | prot_coding | 315. | ENSG00000155714 | prot_coding | 316. | ENSG00000142192 | prot_coding |
| 317. | ENSG00000163644 | prot_coding | 318. | ENSG00000166848 | prot_coding | | | |
| 319. | ENSG00000163110 | prot coding | 320. | ENSG00000166509 | prot_coding | | | |

| | | | | | | | | |
|---|---|---|---|---|---|---|---|---|
| Prot_coding=Protein Coding; Proces'd_transc=Processed Transcript | | | | | | | | |

**Table 16: 44 bronchiolar and alveolar cell literature markers used in this study**

| **Gene** | **Gene name** | **Cell types** | **Evidence** | **Ensembl Gene ID** |
|---|---|---|---|---|
| SFTPC | Surfactant protein C | Epithelial precursor, alveolar type II | IHC², qPCR², in-situ hyb⁴, timecourse² | ENSG00000168484 |
| PDPN | Podoplanin | Epithelial precursor, alveolar type I | IHC⁴ | ENSG00000162493 |
| CGRP | CGRP receptor component | Epithelial precursor | IHC^{1,2} | ENSG00000241258 |
| CD34 | CD34 molecule | Epithelial precursor | IHC³ | ENSG00000174059 |
| ATXN1 | Ataxin 1 | Epithelial precursor | IHC³ | ENSG00000124788 |
| SOX11 | SRY-box 11 | Epithelial precursor | RNAseq⁴ | ENSG00000176887 |
| TUBA1A | Tubulin alpha 1a | Epithelial precursor | RNAseq⁴ | ENSG00000167552 |
| FOXJ1 | Forkhead box J1 | Ciliated bronchiolar epithelial cells | IHC^{2,4} | ENSG00000129654 |
| AQP4 | Aquaporin 4 | Ciliated bronchiolar epithelial cells | IHC⁵ | ENSG00000171885 |
| ITGB4 | Integrin subunit beta 4 | Ciliated bronchiolar epithelial cells | qPCR⁴ | ENSG00000132470 |
| TOP2A | Topoisomerase DNA II alpha | Ciliated bronchiolar epithelial cells | qPCR⁴ | ENSG00000131747 |
| SCGB1A1 | Secretoglobin family 1A member 1 | Ciliated bronchiolar epithelial cells, Clara cells | injury timecourse², IHC^{1,2,4} | ENSG00000149021 |
| CLDN10 | Claudin 10 | Bronchiolar Clara cells | injury timecourse², IHC² | ENSG00000134873 |
| KRT15 | Keratin 15 | Bronchiolar Clara cells | IHC⁴ | ENSG00000171346 |
| AQP3 | Aquaporin 3 | Bronchiolar Clara cells | in-situ EM⁵ | ENSG00000165272 |
| CYP2F2P | Cytochrome P450 family 2 subfamily F member 2, pseudogene | Bronchiolar Clara cells | injury timecourse² | ENSG00000237118 |
| FMO3 | Flavin containing monooxygenase 3 | Bronchiolar Clara cells | injury timecourse² | ENSG00000007933 |
| PON1 | Paraoxonase 1 | Bronchiolar Clara cells | injury timecourse² | ENSG00000005421 |
| AOX3P | Aldehyde oxidase 3, pseudogene | Bronchiolar Clara cells | injury timecourse² | ENSG00000244301 |
| SCGB3A2 | Secretoglobin family 3A member 2 | Bronchiolar Clara cells | microarray² | ENSG00000164265 |
| CES1 | Carboxylesterase 1 | Bronchiolar Clara cells | microarray² | ENSG00000198848 |
| GABRP | Gamma-aminobutyric acid type A receptor pi subunit | Bronchiolar Clara cells | microarray² | ENSG00000094755 |
| SFTPA1 | Surfactant protein A1 | Alveloar type I and II | IHC¹ | ENSG00000122852 |
| HOPX | HOP homeobox | Alveolar type I | Tg-IF⁴ | ENSG00000171476 |
| AGER | Advanced glycosylation end product-specific receptor | Alveolar type I | IHC⁴ | ENSG00000204305 |
| AQP5 | Aquaporin 5 | Alveolar type I | qPCR⁴, RNAseq⁴, IHC⁵ | ENSG00000161798 |
| VEGFA | Vascular endothelial growth factor A | Alveolar type I | qPCR⁴, RNAseq⁴ | ENSG00000112715 |
| HES1 | Hes family bHLH transcription factor 1 | Alveolar type I | RNAseq⁴ | ENSG00000114315 |

| **Gene** | **Gene name** | **Cell types** | **Evidence** | **Ensembl Gene ID** |
|---|---|---|---|---|
| SEMA3A | Semaphorin 3A | Alveolar type I | RNAseq⁴ | ENSG00000075213 |
| TGFB1 | Transforming growth factor beta 1 | Alveolar type I | RNAseq⁴ | ENSG00000105329 |
| GPRCSA | G protein-coupled receptor class C group 5 member A | Alveolar type I | RNAseq⁴ | ENSG00000013588 |
| EGFL6 | EGF like domain multiple 6 | Alveolar type II | RNAseq⁴, in-situ hyb⁴ | ENSG00000198759 |
| ABCA3 | ATP binding cassette subfamily A member 3 | Alveolar type II | qPCR⁴, RNAseq⁴ | ENSG00000167972 |
| MUC1 | Mucin 1, cell surface associated | Alveolar type II | qPCR⁴, RNAseq⁴ | ENSG00000185499 |
| LYZ | Lysozyme | Alveolar type II | qPCR⁴, RNAseq⁴ | ENSG00000090382 |
| SFTPB | Surfactant protein B | Alveolar type II | qPCR⁴, RNAseq⁴ | ENSG00000168878 |
| CFTR | Cystic fibrosis transmembrane conductance regulator | Alveolar type II | qPCR⁴, RNAseq⁴ | ENSG00000001626 |
| CEBPA | CCAAT/enhancer binding protein alpha | Alveolar type II | qPCR⁴, RNAseq⁴ | ENSG00000245848 |
| SFTPD | Surfactant protein D | Alveolar type II | qPCR⁴, RNAseq⁴ | ENSG00000133661 |
| ID2 | Inhibitor of DNA binding 2, HLH protein | Alveolar type II | qPCR⁴, RNAseq⁴ | ENSG00000115738 |
| SOX9 | SRY-box 9 | Alveolar type II | RNAseq⁴ | ENSG00000125398 |
| CITED2 | Cbp/p300 interacting transactivator with Glu/Asp rich carboxy-terminal domain 2 | Alveolar type II | RNAseq⁴ | ENSG00000164442 |
| CMTM8 | CKLF like MARVEL transmembrane domain containing 8 | Alveolar type II | RNAseq⁴ | ENSG00000170293 |
| FGFR2 | Fibroblast growth factor receptor 2 | Alveolar type II | RNAseq⁴ | ENSG00000066468 |

| | | | | |
|---|---|---|---|---|
| ¹ Wuenschell 1996; 2 Zemke 2009; 3 Kim 2005; 4 Treutlein 2014; 5 Nielsen 1997 | | | | |

## Claims

1. A method of detecting whether a lung tissue sample is positive for usual interstitial pneumonia (UIP) or non-usual interstitial pneumonia (non-UIP), comprising:
(a) assaying the expression level of each of a first group of transcripts and a second group of transcripts in a test sample of a subject, wherein the first group of transcripts includes sequences corresponding to all of the genes listed in Table 5 that are overexpressed in UIP and the second group of transcripts includes sequences corresponding to all of the genes listed in Table 5 that are under-expressed in UIP; and
(b) comparing the expression level of each of the first group of transcripts and the second group of transcripts with reference expression levels of the corresponding transcripts to (1) classify said lung tissue as usual interstitial pneumonia (UIP) if there is (i) an increase in an expression level corresponding to the first group and/or (ii) a decrease in an expression level corresponding to the second group as compared to the reference expression levels, or (2) classify the lung tissue as non-usual interstitial pneumonia (non-UIP) if there is (i) an increase in the expression level corresponding to the second group and/or (ii) a decrease in the expression level corresponding to the first group as compared to the reference expression levels.

2. The method of claim 1, wherein the test sample is a pool of a plurality of samples obtained from the subject.

3. The method of claim 1 or 2, wherein the test sample is a biopsy sample or a bronchoalveolar lavage sample.

4. The method of claim 3, wherein the biopsy sample is a transbronchial biopsy sample.

5. The method of any one of the preceding claims, wherein assaying the expression level is accomplished using qRT-PCR, DNA microarray hybridization, RNAseq, or a combination thereof.

6. The method of any one of the preceding claims, comprising synthesizing cDNA from RNA expressed in the test sample prior to assaying the expression level.

7. The method claim 1, further comprising using smoking status as a covariate to the classification step of (1) or (2).

8. The method of claim 7, wherein smoking status is determined by detecting an expression profile indicative of the subject's smoker status.

9. The method of any one of the preceding claims, comprising implementing a classifier trained using one or more features selected from gene expression, variants, mutations, fusions, loss of heterozygoxity (LOH), and biological pathway effect.

10. The method of any one of the preceding claims, wherein the expression data used to classify the sample as UIP or non-UIP comprises expression data for at least two transcripts corresponding to genes selected from the genes listed in Table 5.

11. The method of claim 10, wherein the expression data used comprises each of the genes listed in Table 5.
